(19) Europäisches Patentamt
European Patent Office
Office européen des brevets

(11) EP 1 635 851 B1

(12) **EUROPEAN PATENT SPECIFICATION**

(45) Date of publication and mention of the grant of the patent:
**16.10.2013 Bulletin 2013/42**

(21) Application number: **04752902.9**

(22) Date of filing: **21.05.2004**

(51) Int Cl.:
***A61K 31/135*** (2006.01) ***A61K 31/137*** (2006.01)
***A61P 25/28*** (2006.01)

(86) International application number:
**PCT/US2004/015974**

(87) International publication number:
**WO 2005/000203 (06.01.2005 Gazette 2005/01)**

(54) **METHODS FOR TREATING COGNITIVE IMPAIRMENT AND IMPROVING COGNITION**

VERFAHREN ZUR BEHANDLUNG VON KOGNITIVEN STÖRUNGEN UND ZUR VERBESSERUNG DER KOGNITION

METHODES PERMETTANT DE TRAITER LA DEFICIENCE COGNITIVE ET D'AMELIORER LA COGNITION

(84) Designated Contracting States:
**AT BE BG CH CY CZ DE DK EE ES FI FR GB GR HU IE IT LI LU MC NL PL PT RO SE SI SK TR**

(30) Priority: **23.05.2003 US 444970**
**23.05.2003 US 473168 P**
**02.03.2004 US 791223**

(43) Date of publication of application:
**22.03.2006 Bulletin 2006/12**

(73) Proprietor: **Cognition Pharmaceuticals LLC**
**New York, NY 10022 (US)**

(72) Inventors:
- **EPSTEIN, Mel, H.**
  **Bristol, RI 02806 (US)**
- **WIIG, Kjesten, A.**
  **Providence, RI 02906 (US)**
- **CARPENTER, Randall, L.**
  **Waban, MA 02468 (US)**
- **ARNOLD, H., Moore**
  **Lower Gwynedd, PA 19002 (US)**
- **ZAREVICS, Peter**
  **Spring City; Pennsylvania (US)**

(74) Representative: **Kirkham, Nicholas Andrew et al**
**Graham Watt & Co. LLP**
**St. Botolph's House**
**7-9 St. Botolph's Road**
**Sevenoaks**
**Kent TN13 3AJ (GB)**

(56) References cited:
**EP-A- 1 743 631**
**WO-A-02/39998**
**US-A- 5 151 449**
**US-B2- 6 492 427**
**US-B2- 6 635 675**

- **GELOWITZ D L ET AL: "CHRONIC L-DEPRENYL OR L-AMPHETAMINE: EQUAL COGNITIVE ENHANCEMENT, UNEQUAL MAO INHIBITION" PHARMACOLOGY BIOCHEMISTRY AND BEHAVIOR, ELSEVIER, US, vol. 47, no. 1, 1994, pages 41-45, XP008020420 ISSN: 0091-3057**
- **YASAR ET AL: 'Are Metabolites of L-Deprenyl (Selegiline) Useful or Harmful? Indications from Preclinical Research.' J NEURAL TRANSM. vol. 48, 1996, pages 61 - 73, XP008020416**
- **NICKEL ET AL: "EFFECT OF ENANTIOMERS OF DEPRENYL (SELEGILINE) AND AMPHETAMINE ON PHYSICAL ABUSE LIABILITY AND CORTICAL ELECTRICAL ACTIVITY IN RATS", NEUROPHARMACOLOGY, vol. 11, 1990, pages 983-992,**
- **SOETENS ET AL: "Amphetamine enhances human-memory consolidation", NEUROSCIENCE LETTERS, vol. 161, 1993, pages 9-12,**
- **RICHARD MORRIS: "Developments of a water-maze procedure for studying spatial learning in the rat", JOURNAL OF NEUROSCIENCE METHODS, vol. 11, 1984, pages 47-60,**
- **ROBERT S. SAINSBURY: "Hippocampal Theta: a Sensory-inhibition Theory of Function", NEUROSCIENCE & BIOBEHAVIORAL REVIEWS, vol. 22, no. 2, 1998, pages 237-241,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- UCHIDA ET AL: "Cortical oscillations inhuman medial temporal lobe during wakefulness and all-night sleep", BRAIN RESEARCH, vol. 891, 2001, pages 7-18,
- HALGREN ET AL: "HUMAN HIPPOCAMPAL FORMATION EEG DESYNCHRONIZES DURING ATTENTIVENES AND MOVEMENT", ELECTROENCEPHALOGRAPHY AND CLINICAL NEUROPHYSIOLOGY, vol. 44, 1978, pages 778-781,
- BARTUS ET AL: "DRUGS TO TREAT AGE-RELATED NEURODEGENERATIVE PROBLEMS THE FINAL FRONTIER OF MEDICAL SCIENCE", JOURNAL OF THE AMERICAN GERIATRICS SOCIETY, vol. 38, no. 6, 1990, pages 680-695,
- NICKEL ET AL: "Evaluation of physical dependence liability of l-deprenyl (selegiline) in animals", CLINICAL PHARMACOLOGY AND THERAPEUTICS, vol. 56, 1994, pages 757-767,
- LYNCH ET AL: "The nature and causes of hippocampal long-term potentiation.", PROGRESS IN BRAIN RESEARCH, vol. 83, 1990, pages 233-250,
- RAMOS ET AL: "EEG activity during cognitive performance in women.", THE INTERNATIONAL JOURNAL OF NEUROSCIENCE, vol. 69, 1993, pages 185-195,
- CORSI-CABRERA ET AL: "EEG activity during cognitive performance in women.", THE INTERNATIONAL JOURNAL OF NEUROSCIENCE, vol. 72, 1993, pages 257-264,
- JELIC ET AL: "Quantitative electroencephalography in mild cognitive impairment: longitudinal changes and possible prediction of Alzheimer’s disease", NEUROBIOLOGY OF AGING, vol. 21, 2000, pages 553-540,
- CODY JT: "Metabolic Precursors to Amphetamine and Methamphetamine", FORENSIC SCIENCE REVIEW, vol. 5, no. 2, 1993, pages 110-127,


Remarks:

The file contains technical information submitted after the application was filed and not included in this specification

## Description

BACKGROUND OF THE INVENTION.

The term "memory" subsumes many different processes and requires the function of many different brain areas. Overall, human memory provides declarative recall, e.g., for facts and events accessible to conscious recollection, and non-declarative recall, e.g., procedural memory of skills and operations not stored regarding time and place. Research in recent years has provided information necessary to understand many of the various components of memory and has identified associated brain regions. A newly acquired experience initially is susceptible to various forms of disruption. With time, however, the new experience becomes resistant to disruption. This observation has been interpreted to indicate that a labile, working, short-term memory is consolidated into a more stable, long-term memory.

Behavioral research has found that the human mind consolidates memory at certain key time intervals. The initial phase of memory consolidation occurs in the first few minutes after an exposure to a new idea or learning experience. The next phase occurs over a longer period of time, such as during sleep. If a learning experience has on-going meaning to us, the next week or so serves as a further period of memory consolidation. In effect, in this phase, the memory moves from short-term to long-term storage.

Moreover, various mechanisms have been proposed to account for the formation of long- term memory. A wide range of observations suggest an evolutionarily conserved molecular mechanism involved with the formation of long-term memory. These include increased release of synaptic transmitter, increased number of synaptic receptors, decreased $K_D$ of receptors, synthesis of new memory factors either in the presynaptic or postsynaptic element, sprouting of new synaptic connections, increase of the active area in the presynaptic membrane and many others. Synaptic plasticity, the change in the strength of neuronal connections in the brain, is thought to underlie long- term memory storage.

Memory consolidation, the process of storing new information in long-term memory is also believed to play a crucial role in a variety of neurological and mental disorders, including mental retardation, Alzheimer's disease and depression. Indeed, loss or impairment of long-term memory is a significant feature of such diseases, and no effective therapy for that effect has emerged. Short-term memory and working memory, are generally not significantly impaired in such patients.

Accordingly, methods and compositions that enhance long- term memory function and/or performance, or prophylactically (e.g., as a neuroprotective treatment) prevent or slow degradation of long- term memory function and/or performance would be desirable. Similarly, methods and compositions for restoring long- term memory function and/or performance are needed.

Impairments in cognitive and memory processes in a human can occur in a number of conditions or diseases, such as age-related memory loss, Mild Cognitive Impairment, Alzheimer's disease, Multiple Sclerosis, brain injury, brain aneurysm, stroke, schizophrenia, epilepsy, chronic fatigue syndrome, fibromyalgia syndrome, chemotherapy (e.g., cancer chemotherapy), traumatic brain injury, and Parkinson's disease. Following exposure to a muscarinic cholinergic receptor antagonist, such as atropine or scopolamine, humans can experience impairment of cognitive and memory processes. Clinical management strategies currently provide minimal, if any, improvement in memory and cognitive function. Thus, there is a need to develop new, improved and effective methods for the treatment of a human suffering with an impairment in cognitive and memory processes.

SUMMARY OF THE INVENTION

The present invention relates to compositions useful in treating a human having an impairment in memory and/or cognitive function associated with multiple scelerosis as claimed in claims 1 to 11.

The human can have an impairment in memory consolidation (the process of storing new information in long term memory), an impairment in short term memory processes, an impairment in working memory, an impairment in long- term memory, an impairment in declarative memory or an impairment in procedural memory. The humans are treated with the amphetamine class of compounds (collectively referred to herein as "amphetamine compounds") to enhance, prevent and/or restore long- term memory function and performance, e.g., to improve the process of storing new information in long term memory in humans (memory consolidation) or to improve short term memory or to improve working memory. The human can have an impairment in memory and/or a cognition function as a consequence of exposure to a muscarinic cholinergic receptor antagonist. More particularly, the invention relates to the discovery that a particular enantiomer of amphetamine compounds (R)- (- )- amphetamine (l- amphetamine, levo- amphetamine) or (R)- (- )- methamphetamine (l- methamphetamine, levo- methaniphetamine) is effective for treating humans having an impairment in memory and an impairment in cognitive function.

In another embodiment, the invention includes a method of improving memory consolidation in a human, comprising the step of administering at least one member selected from the group consisting of l-amphetamine and 1-methamphetamine to a human having an impairment in memory consolidation, wherein the 1-amphetamine is at least

about 80 mole percent l-amphetamine relative to d-amphetamine and the l-methamphetamine is at least about 90 mole percent 1-methamphetamine relative to d-methamphetamine.

In yet another embodiment, the invention is useful in a method of improving memory consolidation in a human, comprising the step of administering at least one member selected from the group consisting of l-amphetamine and l-methamphetamine to a human having an impairment in memory consolidation, wherein the l-amphetamine is at least about 90 mole percent l-amphetamine relative to d-amphetamine and the l-methamphetamine is at least about 90 mole percent l-methamphetamine relative to d-methamphetamine.

An additional embodiment of the invention is useful in a method of improving memory consolidation in a human, comprising the steps of assessing the degree of an impairment in memory consolidation in a human; administering at least one member selected from the group consisting of l-amphetamine and l-methamphetamine to the human; and determining the improvement in memory consolidation after administering the l-amphetamine and l-methamphetamine to the human.

In still another embodiment, the invention is useful in a method of improving memory consolidation in a human, comprising the step of administering an amphetamine to a human having an impairment in memory consolidation in an amount effective to improve memory consolidation in the human, wherein the amphetamine is at least about 85 mole percent l-amphetamine.

In another embodiment, the invention is useful in a method of improving memory consolidation in a human, comprising the step of administering an amphetamine to a human having an impairment in memory consolidation in an amount effective to improve memory consolidation in the human, wherein the amphetamine is at least about 80 mole percent l-amphetamine.

Another embodiment of the invention is useful in a method of improving memory consolidation in a human, comprising the step of administering an amphetamine to a human having an impairment in memory consolidation in an amount effective to improve memory consolidation in the human, wherein the amphetamine is at least about 99 mole percent l-amphetamine and the l-amphetamine is administered to the human in a dose of at least about a 0.01 mg dose.

In yet another embodiment, the invention is useful in a method of improving memory consolidation in a human, comprising the step of administering an amphetamine to a human having an impairment in memory consolidation in an amount effective to improve memory consolidation in the human, wherein the amphetamine is at least about 90 mole percent l-amphetamine and the 1-amphetamine is administered to the human in a dose between about a 0.01 mg dose to about a 125 mg dose.

In an additional embodiment, the invention is useful in a method of improving memory consolidation in a human, comprising the step of administering an amphetamine to a human having an impairment in memory consolidation in an amount effective to improve memory consolidation in the human, wherein the amphetamine is at least about 80 mole percent l-amphetamine and the 1-amphetamine, is administered to the human in a dose at lease about a 0.01 mg dose.

In a further embodiment, the invention is useful in a method of improving memory consolidation in a human, comprising the step of administering an amphetamine to a human having an impairment in memory consolidation in an amount effective to improve memory consolidation in the human, wherein the amphetamine is between about 80 mole percent l-amphetamine to about 99 mole percent l-amphetamine.

In still another embodiment, the invention is useful in a method of improving memory consolidation in a human, comprising the step of administering an amphetamine to a human having an impairment in memory consolidation in an amount effective to improve memory consolidation in the human, wherein the amphetamine is between about 80 mole percent l-amphetamine to about 99 mole percent 1-amphetamine and the l-amphetamine is administered to the human in a dose at least about a 0.01 mg dose.

Another embodiment of the invention is useful in a method of improving memory consolidation in a human comprising assessing the degree of impairment in memory consolidation in a human having an impairment in memory consolidation and administering an amphetamine to the human in an amount effective to improve memory consolidation in the human, wherein the amphetamine is at least about 80 mole percent l-amphetamine. The improvement in memory consolidation after administering the amphetamine to the human is determined.

In an additional embodiment, the invention is useful in a method of improving memory consolidation in a human, comprising the step of administering an amphetamine to a human having an impairment in memory consolidation in an amount effective to improve memory consolidation in the human, wherein the amphetamine is at least about 90 mole percent l-amphetamine and has the structural formula:

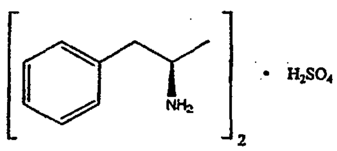

In still another embodiment, the invention is useful in a method of improving memory consolidation in a human, comprising the step of administering an amphetamine to a human having an impairment in memory consolidation in an amount effective to improve memory consolidation in the human, wherein the amphetamine is at least about 90 mole percent l-methamphetamine.

In yet another embodiment, the invention is useful in a method of improving memory consolidation in a human, comprising the step of administering an amphetamine to a human having an impairment in memory consolidation in an amount effective to improve memory consolidation in the human, wherein the amphetamine is at least about 99 mole percent l-methamphetamine and the dose of l-methamphetamine administered to the human is at least about a 0.01 mg dose.

Another embodiment of the invention is useful in a method of improving memory consolidation in a human, comprising the step of administering an amphetamine to a human having an impairment in memory consolidation in an amount effective to improve memory consolidation in the human, wherein the amphetamine is at least about 90 mole percent l-methamphetamine and the l-methamphetamine is administered to the human in a dose at least about a 0.01 mg dose.

In an additional embodiment, the invention is useful in a method of improving memory consolidation in a human, comprising the step of administering an amphetamine to a human having an impairment in memory consolidation in an amount effective to improve memory consolidation in the human, wherein the amphetamine is at least about 90 mole percent 1-methamphetamine and has the structural formula:

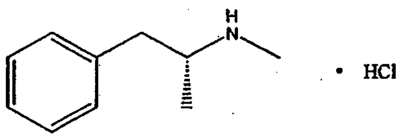

In one embodiment, the invention is a pharmaceutical kit comprising one or more amphetamine compound(s) in an amount sufficient to enhance long-term memory in a patient, a pharmaceutically acceptable carrier, and instructions (written and/or pictorial) describing the use of the formulation for enhancing memory.

In another embodiment, the invention is a pharmaceutical preparation comprising one or more amphetamine compounds provided as a single oral dosage formulation in an amount sufficient to enhance long-term memory in a patient but resulting in a concentration in the patient lower than its $EC_{50}$ as a CNS stimulant.

In still another embodiment, the invention is a pharmaceutical preparation comprising one or more amphetamine compounds provided in the form of a transdermal patch and formulated for sustained release of the amphetamine(s) in order to administer an amount sufficient to enhance long-term memory in a patient but resulting in a concentration in the patient lower than its $EC_{50}$ as a CNS stimulant.

In particular embodiments of the kits, preparations and compositions, the invention features a pharmaceutical kit or preparation comprising a mixture of at least a single species of amphetamine compounds or at least two different species of amphetamine compounds. The different species of amphetamine compounds can be present in equal or in differing amounts with respect to one another.

In another embodiment of the kits, preparation and compositions, the invention features a composition comprising at least about 60 percent (w/w or mole percent), about 75 percent (w/w or mole percent), about 80 percent (w/w or mole percent), about 85 percent (w/w or mole percent), about 95 percent (w/w or mole percent) or about 99 percent (w/w or mole percent) of l-amphetamine relative to d-amphetamine or above 80 percent (w/w or mole percent) l-methamphetamine relative to d-methamphetamine. For example, an amphetamine composition employed in the methods can be about 80 percent (w/w or mole percent) l-amphetamine or l-methamphetamine relative to d-amphetamine or d-methamphetamine, where d-amphetamine or d-methamphetamine is about 20 percent (i.e., the remainder) (w/w or mole percent) of the amphetamine.

In another embodiment, the methods of the invention employ an amphetamine that is about 100 percent (w/w or mole percent) l-amphetamine or l-methamphetamine, wherein the l-amphetamine is a composition that includes at least about 100 mole percent l-amphetamine relative to a total amphetamine content of the composition or wherein the l-methamphetamine is administered as a composition that includes, at least about 100 mole percent l-amphetamine relative to a total amphetamine content of the composition. An amphetamine that is "about 100 percent" l-amphetamine or l-methamphetamine can contain insignificant trace amounts of d-amphetamine or d-methamphetamine.

In certain preferred embodiments, particularly for those which use (R)- (- )- amphetamine (l- amphetamine) or l- methamphetamine, use compositions of (R)- (- )- amphetamine which contain less than 10 percent (w/w or mole percent) (S)- (+)- amphetamine, and even more preferably less than less than 5 percent (w/w or mole percent), 1 percent (w/w or mole percent) or even less than 0.5 percent (w/w or mole percent) (S)- (+)- amphetamine.

In another embodiment, the amphetamine employed in the invention can be a percent of the total composition

administered to the human. The amphetamine component of the composition can be about 50 percent (w/w), about 60 percent (w/w), about 75 percent (w/w), about 80 percent (w/w), about 85 percent (w/w), about 90 percent (w/w), about 95 percent (w/w) and about 100 percent (w/w) of the total composition administered to the human. For example, the human can be administered a composition which comprises about 80 weight or volume percent amphetamine and about 20 weight or volume percent, respectively, inert excipient The amphetamine component of the composition includes at least one member selected from the group consisting of l-amphetamine, l-methamphetamine, d-amphetamine and d-methamphetamine.

In still another embodiment of the invention features one or more amphetamine compound(s) provided in an amount sufficient to enhance long-term memory in a patient by a statistically significant amount when assessed by a standardized performance test.

In certain embodiments of the invention features one or more amphetamine compound(s) comprising at least 2-fold less, or at least 4-fold less of the distomer(s) as compared to an equally effective long term memory enhancing dose of the distomer(s) of the amphetamine compound(s).

In certain embodiments of the invention features amphetamine comprising at least 2- fold less, or at least 4-fold less of (R)- (- )- amphetamine as compared to an equally effective long term memory enhancing dose of (S)- (+)-amphetamine.

In certain embodiments of the invention features one or more amphetamine compound(s) provided in an amount sufficient to enhance long-term memory in a patient by a statistically significant amount when assessed by standardized performance test, such as one or more of a Rey Auditory and Verbal Learning Test (RAVLT); Cambridge Neuropsychological Test Automated Battery (CANTAB); a Children's Memory Scale (CMS); a Contextual Memory Test; a Continuous Recognition Memory Test (CMRT); a Denman Neuropsychology Memory Scale; a Fuld Object Memory Evaluation (FOME); a Graham-Kendall Memory for Designs Test; a Guild Memory Test; a Learning and Memory Battery (LAMB); a Memory Assessment Clinic Self-Rating Scale (MAC-S); a Memory Assessment Scales (MAS); a Randt Memory Test; a Recognition Memory Test (RMT); a Rivermead Behavioral Memory Test; a Russell's Version of the Wechsler Memory Scale (RWMS); a Test of Memory and Learning (TOMAL); a Vermont Memory Scale (VMS); a Wechsler Memory Scale; and a Wide Range Assessment of Memory and Learning (WRAML); First-Last Name Association (Youngjohn J.R., et al., Archives of Clinical Neuropsychology 6:287-300 (1991)); Name-Face Association; Wechsler Memory Scale-Revised; (Wechsler, D., Wechsler Memory Scale-Revised Manual, NY, NY, The Psychological Corp. (1987)); California Verbal Learning Test-Second Edition (Delis, D.C., et al., The Californian Verbal Learning Test, Second Edition, Adult Version, Manual, San Antonio, TX: The Psychological Corporation (2000)); Facial Recognition (delayed non-matching to sample); Cognitive Drug Research (CDR) Computerized Assessment Battery-Wesnes; Buschke's Selective Reminder Test (Buschke, H., et al., Neurology 24:1019-1025 (1974)); Telephone Dialing Test; and Brief Visuospatial Memory Test-Revised. In certain embodiments, the pharmaceutical composition features one or more amphetamine compounds provided in an amount sufficient to enhance long-term memory (to improve memory consolidation in a human) when assessed by a word recall test such as RAVLT.

In yet another embodiment of the invention features one or more amphetamine compound(s) provided in an amount sufficient to enhance long-term memory in a patient by a statistically significant amount when assessed by a Providence Recognition Memory Test.

In an additional embodiment, the invention is a method to improve a memory impairment in a human having multiple sclerosis by administration of the amphetamine compounds of the invention. The memory impairment and improvement in memory can be assessed using established criteria (for example, Thornton, A.E., et al. Neuropsychology 11:357-366 (1997)). These techniques include the Brown-Peterson task (Brown, J., Quarterly J. of Exp. Psychology 10: 12-21 (1958)); the Paced Auditory Serial Addition Test (PASAT) (Gronwall, D.M.A., Perceptual and Motor Skills 44: 367-373 (1977)); and tasks described, for example, by DeLuca, J., et al., J. Clinical and Exp. Neuropsychology (2004).

In another embodiment of the invention features one or more amphetamine compound(s) provided in the form of a saccharate, a sulfate or an aspartate.

In certain embodiments, the subject pharmaceutical preparations are formulated for variable dosing, and preferably to deliver a sustained and increasing dose, e.g., over at least 4 hours, and more preferably over at least 8 or even 16 hours. For instance, the amphetamine compound is contained within a nonabsorbable shell that releases the drug at a controlled rate.

In certain escalating dose formulations, the amphetamine compound(s) are formulated in a delivery system including a multiplicity of layers each including the same or different polymers, a dose of the amphetamine compound (s) in an increasing dose in the multiplicity of layers, wherein in operation the preparation delivers an increasing dose of the amphetamine compound(s) over time.

In other embodiments of escalating dose formulations, the amphetamine compound(s) are formulated in a delivery system including a bioerodible polymer, a dose of the amphetamine compound(s) present in an initial dose and a final dose, whereby the preparation delivers an initial dose then a final dose over time.

In still other embodiments of escalating dose formulations, the amphetamine compound(s) are formulated in

a delivery system including a plurality of beads, each bead including a amphetamine compound and having a dissolution profile, which plurality of beads is a variegated population with respect to dose and/or dissolution profile so as to deliver, upon administration, said sustained and increasing dose over at least 4 hours.

In certain escalating dose formulations, the amphetamine compound(s) are formulated in a delivery system wherein the amphetamine compound is (i) contained within a nonabsorbable shell that releases the drug at a controlled rate, and (ii) formulated in at least two different dissolution profiles.

Levo- amphetamine, l- amphetamine and (R)- (- )- amphetamine are used interchangeably herein. Levo- methamphetamine, l- methamphetamine and (R)- (- )- methamphetamine are used interchangeably herein.

In one embodiment, the (R)- (- )- amphetamine employed in the invention has the structural formula:

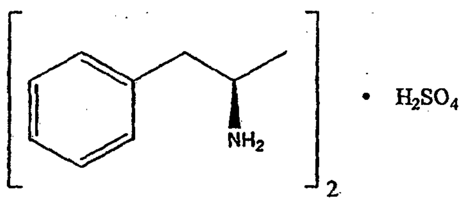

IV

Formula IV is also referred to herein as C105, levo- amphetamine sulfate or l- amphetamine sulfate. Formula IV has the molecular formula $C_{18}H_{28}N_2O_4S$ and a molecular weight of 368.50. The IUPAC chemical name of Formula IV is (- )- 1- methyl- 2- phenylethylamine sulfate (2: 1) and the CAS chemical name (- )- $\alpha$- methylphenethylamine sulfate (2: 1) .

In another embodiment, the (R)- (- )- amphetamine employed in the invention has the structural formula:

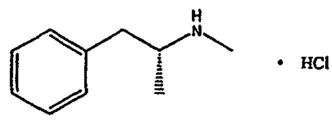

V

Formula V is also referred to herein as SN522- HCl (hydrochloride), levo- methamphetamine HCl or l- methamphetamine HCl. Formula V has the molecular formula $C10H_{16}NCl$.

In still another embodiment, the (R)- (- )- amphetamine employed in the invention has the structural formula:

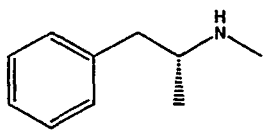

VI

Formula VI is also referred to herein as SN522, the free base of SN522, levo- methamphetamine, levo- desoxyephedrine, l- desoxyephedrine or levmetamfetamine. Formula VI has the molecular formula $C_{10}H_{15}N$ and a molecular weight of 149.24.

In still another embodiment, the amphetamine compounds employed in the invention can be a combination of the amphetamine compounds described herein, e.g., Formulas IV, V and/or VI can be employed in any combination. For example, a human having an impairment in a cognitive function (e.g., attention, executive function, reaction time, learning, information processing, conceptualization, problem solving; verbal fluency) or memory (e.g., memory consolidation, short- term memory, working memory, long- term memory, declarative memory or procedural memory) can be treated, with 1- amphetamine (e.g., C105) and 1- methamphetamine (e.g., SN522, SN522- HCl), either in combination or sequentially.

The amphetamine compounds employed in the methods of the invention (e.g., l-amphetamine and/or l-methamphetamine) can be administered as a component of a composition that includes at least about 99 mole %, at least about 95 mole %, at least about 90 mole %, at least about 85 mole %, at least about 80 mole %, at least about 75 mole %, at least about 70 mole %, at least about 65 mole %, or at least about 60 mole %, of l-amphetamine relative to the total amphetamine content in the composition; or at least about 99 mole %, at least about 95 mole %, at least about 90 mole % of l-methamphetamine relative to the total amount of amphetamine content in the composition.

In certain embodiments, the animal to be treated is a mammal. In certain preferred embodiments the animal to be treated is a human, dog, cat, cattle, horse, sheep, hog or goat

In certain embodiments, the pharmaceutical composition is for oral administration.

In certain other embodiments the pharmaceutical composition is a transdermal patch. In certain embodiments the transdermal patch includes one or more penetration enhancers.

In certain embodiments, the pharmaceutical composition features an amphetamine compound provided as at least about 60 percent (w/w or mole percent), about 75 percent (w/w or mole percent), about 80 percent (w/w or mole percent), about 85 percent (w/w or mole percent) about 95 percent (w/w or mole percent), or 99 percent (w/w or mole percent) of the eutomers relative to the distomers of the amphetamine compound l-amphetamine relative to d-amphetamine. In another embodiment, the amphetamine employed to treat a human is about 100 % 1-amphetamine (w/w or mole percent).

In certain embodiments, the pharmaceutical compositions are formulated for variable dosing, preferably to deliver a sustained dose, e.g., over at least 4 hours and more preferably over at least 8 or even 16 hours. For instance, the amphetamine compound(s) are contained within a nonabsorbable shell that releases the drug at a controlled rate.

In certain embodiments, the pharmaceutical composition features one or more amphetamine compound(s) provided in the form of a saccharate, a sulfate or an aspartate.

In other embodiments of the kits, preparations and compositions, the invention further features amphetamine compound(s) being provided as a single oral dosage formulation in an amount sufficient to enhance long-term memory in a patient but resulting in a concentration in the patient lower than its $EC_{50}$ as a CNS stimulant.

In one embodiment of the invention features a single oral dosage formulation of at least about 2.5 mg to about 25 mg, about 50 mg, about 75 mg, about 100 mg or about 125 mg of an amphetamine compound (e.g., l-amphetamine, C105, l-methamphetamine, SN522, SN522-HCl) and a pharmaceutically acceptable carrier.

In another embodiment, the single dosage formulation is at least about 0.001 mg, about 0.01 mg, about 0.1 mg, about 1 mg, about 2 mg, about 2.5 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 75 mg, about 80 mg, about 85 mg, about 90 mg, about 95 mg, about 100 mg, about 125 mg, about 150 mg, about 200 mg, about 300 mg, about 400 mg, about 500 mg, about 750 mg, or about 1000 mg of an amphetamine compound (e.g., 1- amphetamine, C105, 1- methamphetamine, SN522, SN522- HCl) . In a particular embodiment, the dose of an amphetamine compound (e.g., l- amphetamine, C105, l- methamphetamine, SN522, SN522- HCl) is between about a 5 mg dose and about a 50 mg dose; or between about a 2 mg dose and about a 60 mg dose per day; or between about 1 mg to between about a 100 mg dose; or between about a 1 mg to about a 150 mg dose.

In still another embodiment, the invention employ multiple doses of an amphetamine compound. Each dose of the multiple dose is at least about 0.001 mg, about 0.01 mg, about 0.1 mg, about 1 mg, about 2.5 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 75 mg, about 80 mg, about 85 mg, about 90 mg, about 95 mg, about 100 mg, about 125 mg, about 150 mg, about 200 mg, about 300 mg, about 400 mg, about 500 mg, about 750 mg or about 1000 mg of an amphetamine compound (e.g., l-amphetamine, C105, 1-methamphetamine, SN522, SN522-HCl. The multiple doses can be administered for a day, days, a week, weeks, a month, months or years.

The amphetamine compounds of the invention can be administered to a human acutely (briefly or short-term) or chronically (prolonged or long-term). For example, the amphetamine compounds, (e.g., l-amphetamine, C105, 1-methamphetamine, SN522, SN522-HCl) of the invention can be used in methods to treat a human by administering the amphetamine to the human once a day, multiple times (e.g., 2, 3, 4) in a day, for a day, days, a week, weeks, a month, months or years.

In yet another embodiment of the invention features a single oral dosage formulation of between about 0.001 mg to about 125 mg; between about 0.001 mg to about 250 mg; between 0.001 mg to 500 mg; or between about 0.01 mg to about 125 mg; or between about 0.1 mg to about 125 mg; or between about 1 mg to about 125 mg; or between about 1 mg to about 250 mg; or between about 1 mg to about 500 mg; or between about 1 mg to about 1000 mg; or between about 2.5 mg to about 25 mg, about 50 mg, about 75 mg, about 100 mg or about 125 mg of the eutomer (s) of amphetamine compound (s) (1- amphetamine, C105, 1- methamphetamine, SN522) and, optionally, a pharmaceutically acceptable carrier.

In a further embodiment, the invention employ multiple doses between about 0.001 mg to about 500 mg of the amphetamine compound (e.g., l- amphetamine, C105, l- methamphetamine, SN522, SN522- HCl), wherein each of the multiple doses of the amphetamine compound is between about 0.001 mg to about 125 mg; or between about 0.001 mg to about 250 mg; or between about 0.001 mg to about 500 mg; or between about 0.01 mg to about 125 mg; or between about 0.01 mg to about 500 mg; or between about 0.1 mg to about 125 mg; or between about 1 mg to about 125 mg; or between about 1 mg and about 100 mg; or between about a 1 mg to about a 150 mg dose; or between about 1 mg and about 500 mg; between about 5 mg and about 50 mg; between about 2 mg and 60 mg between about 2.5 mg to about 25 mg, about 50 mg, about 75 mg, about 100 mg, about 125 mg, about 250 mg, about 500 mg, about 750 mg, about 1000 mg of the eutomer (s) of amphetamine compound (s) (1- amphetamine, C105, 1- methamphetamine, SN522, SN522- HCl) and, optionally, a pharmaceutically acceptable carner.

In a further embodiment, the invention employ a single dose of the amphetamine compound (l- amphetamine, C105, 1- methamphetamine, SN522, SN522- HCl) between about 0.0015 mg/kg to about 2 mg/kg; between about 0.015 mg/kg to about 2 mg/kg; or about 0.07 mg to about 0.7 mg or between about 0.14 mg to about 0.7 mg; or about 0.03 mg to about 1.0 mg per day.

In yet another embodiment, the invention employ a single dose about 0.04 mg/kg, about 0.07 mg/kg, about 0.15 mg/kg, about 0.20 mg/kg, about 0.40 mg/kg, about 0.65 mg/kg, about 1 mg/kg, about 1.50 mg/kg, about 1.80 mg/kg or about 3.5 mg/kg of l-amphetamine, C105, 1-methamphetamine, SN522, SN522-HCl.

In an additional embodiment, the invention employ multiple doses of the amphetamine compound (l- amphetamine, C105, 1- methamphetamine, SN522, SN522- HCl), wherein each dose of the multiple dose is between about 0.0015 mg/kg to about 2 mg/kg; or between about 0.. 015 mg/kg to about 2 mg/kg.

In still another embodiment, the invention employ multiple doses, wherein each does of the multiple dose is about 0.04 mg/kg, about 0.07 mg/kg, about 0.15 mg/kg, about 0.20 mg/kg, about 0.40 mg/kg, about 0.65 mg/kg, about 1 mg/kg, about 1.50 mg/kg, about 1.80 mg/kg or about 3.5 mg/kg of 1-amphetamine, C105, 1-methamphetamine, SN522, SN522-HCl.

The cumulative dose of the amphetamine compound (1- amphetamine, C105, l- methamphetamine, SN522, SN522- HCl) employed in the invention, regardless of whether the amphetamine is administered in a single dose or in multiple doses is between about 0.2 mg to about 250 mg; or between about 1 mg to about 1250 mg of the amphetamine compound. In a particular embodiment, the cumulative dose is about 2 mg, about 10 mg, about 20 mg, about 30 mg, about 50 mg, about 60 mg, about 90 mg, about 100 mg, about 150 mg, about 200 mg, about 250 mg, about 450 mg, about 750 mg, about 1000 mg, about 1250 mg, about 2500 mg or about 5000 mg.

In still another embodiment, the invention is useful in a method of treating a human for an impairment in a cognitive function, comprising the step of administering an amphetamine composition selected from the group consisting of 1-amphetamine, l-methamphetamine or a combination of both to a human having impairment in a cognitive function associated with multiple sclerosis.

In still another embodiment, the invention is useful in a method of treating a human for a memory impairment, comprising the step of administering an amphetamine composition selected from the group consisting of l-amphetamine, l-methamphetamine or a combination of both to a human having an impairment in memory associated with multiple sclerosis.

In another embodiment, the invention is useful in a method of treating a human for an impairment in a cognitive function, comprising the step of administering an amphetamine composition selected from the group consisting of l-amphetamine, l-methamphetamine or a combination of both to a human having an impairment in a cognitive function associated with multiple sclerosis.

The practice of the present invention will employ, unless otherwise indicated, conventional techniques of synthetic chemistry, organic chemistry, inorganic chemistry, organometallic chemistry, pharmaceutical chemistry, and behavioral science, which are within the skill of the art. Such techniques are described in the literature. See, for example, Advanced Organic Chemistry: Reactions, Mechanisms, And Structure by J. March (John Wiley and Sons, N.Y., 1992) ; The Chemist's Companion: A Handbook Of Practical Data, Techniques, And References by A. J. Gordon and R. A. Ford (Wiley, NY, 1972) ; Synthetic Methods Of Organometallic And Inorganic Chemistry by W.A. Herrmann and Brauer (Georg Thieme Verlag, N.Y., 1996) ; Experimental Organic Chemistry by D. Todd (Prentice- Hall, N.J., 1979) ; Experimental Organic Chemistry: Standard And Microscale by L. M. Harwood (Blackwell Science, M.A., 1999) ; Experimental Analysis Of Behavior by I. H. Iversen and K. A. Lattal (Elsevier, N.Y., 1991) ; A Practical Guide To Behavioral Research: Tools And Techniques by R. Sommer and B. Sommer (Oxford University Press, N.Y., 2002) ; Advances In Drug Discovery Techniques by A. L. Harvey (Chichester, N.Y., 1998) ; Quantitative Calculations In Pharmaceutical Practice And Research by T. P. Hadjiioannou (VCH, N.Y., 1993) ; Drug Fate And Metabolism: Methods And Techniques by E. R. Garrett and J. L. Hirtz (M. Dekker, N.Y., 1977) ; Behavioral Science Techniques: An Annotated Bibliography For Health Professionals by M. K. Tichy (Praeger Publishers, N.Y., 1975) .

The invention described herein is useful in methods of treating a human having an impairments in a cognitive function (e.g., attention, executive function, reaction time, learning information processing, conceptualization, problem solving, verbal fluency) and memory (e.g., memory consolidation, short term memory, working memory, long term memory, declarative memory or procedural memory). Advantages of the claimed invention include, for example, the treatment of humans suffering an impairment in a cognitive function or memory in a cost effective manner and without significant side affects, especially in individuals who have had a condition or disease for an extended period of time and where clinical management strategies are difficult to implement. Of particular importance, are conditions which require long-term treatment where addictive and potent side effects would be considerably undesirable. The claimed methods provide an efficient way to treat and reduce the severity of an impairment in a cognitive function (also referred to herein as "cognition") and memory in humans.

Gelowitz D et al, in Pharacology Biochemistry and Behavior, Vol 47 pp 41-45, 1994 disclose "chronic L-Depeny1 or L-Amphetamine : Equal Cognitive Enhancement, Unequal MAO Inhibition".

WO- A- 01/39998 discloses methods and compositions for regulating memory consolidation.

Yasar S et al, in J Neural Transm (1996) [suppl] 48: 61-73 discloses "Are metabolites of L-Depeny1 (selegiline) useful or harmful? Indications from preclinical research".

BRIEF DESCRIPTION OF THE DRAWINGS

Figure 1 presents the effectiveness of various doses of S- (+)- amphetamine on Performance in the Inhibitory Avoidance Task.
Figure 2 demonstrates the effect of 2 mg/kg of S- (+)- amphetamine on Performance in the Inhibitory Avoidance Task.
Figure 3 shows the varying effect of S- (+)- amphetamine depending on the time between administration and inception of training.
Figure 4 illustrates the effect of S- (+)- amphetamine on memory retention one week after the initial training.
Figure 5 depicts the effects of S- (+)- amphetamine on normal and fornix- lesioned animals.
Figure 6 shows the effect of S- (+)- amphetamine (2.0 mg/kg) on Performance in Inhibitory Avoidance.
Figures 7A, 7B, 7C, 7D, 7E and 7F show the effect of S- (+)- amphetamine on Activity Levels.
Figure 8 shows the effectiveness of various doses of R- (- )- amphetamine on memory retention.
Figure 9 shows the effectiveness of R- (- )- amphetamine on memory retention.
Figure 10 shows the effect of R- (- )- amphetamine (0.5 mg/kg) on Performance in the Inhibitory Avoidance Task.
Figure 11 shows the effect of Post Training Administration of R- (- )- amphetamine (0.5 mg/kg) on Performance in the Inhibitory Avoidance Task.
Figure 12 shows the effect of R- (- )- amphetamine (1.0 mg/kg) on Inhibitory Avoidance Performance in Fornix Lesion Rats.
Figures 13A, 13B, 13C and 13D show the effect of R- (- )- amphetamine on Performance in the Object Recognition Task in Normal and Fornix Lesion Rats.
Figures 14A, 14B, 14C, 14D, 14E and 14F show the effect of R- (- )- amphetamine (0.5 mg/kg) on Activity Levels.
Figures 15A, 15B, 15C, 15D, 15E and 15F shows the effect of S- (+)- amphetamine (2 mg/kg) on Activity Levels.
Figure 16 shows the effect of R- (- )- amphetamine on Tail- Flick Analgesia.
Figure 17 shows an exemplary sustained release device.
Figure 18 depicts the pharmacokinetics of R- (- )- amphetamine and Memory Assessments and PK.
Figure 19 shows that administration of R- (- )- amphetamine to human patients can improve verbal memory.
Figure 20 depicts the Step-Through Latency (sec) for rats treated with control/vehicle (veh), d-amphetamine (d-amph), l-amphetamine (C105) or l-methamphetamine (SN522).
Figure 21 depicts the Step-Through Latency (sec) for rats treated with control (0) or varying doses of l-methamphetamine (SN522). The asterisk indicates a significant difference from the control (p<0.05).
Figure 22 depicts the Escape Latency (sec) for rats treated with saline control or l-methamphetamine (SN522).
Figure 23 depicts the Activity Measure (% increase from control) for rats treated with 1-methamphetamine (SN522).
Figure 24 depicts the Activity Measure (% increase from control) for rats treated with D-amphetamine (d-amph).
Figure 25 depicts the Memory Score, as assessed by the Rey Auditory and Verbal Learning Test, following a 30 minute (min) and a 24 hour (hr) recall time for humans treated with l-amphetamine (C105). The asterisk depicts significant differences.
Figure 26 compares individual subject's memory scores, as assessed by the Rey Auditory and Verbal Learning Test (RAVLT Score (0-15)), following placebo treatment to their best score following treating with l-amphetamine (C105).
Figure 27 illustrates the keyboard proficiency for subjects diagnosed with mild cognitive impairment treated with l-amphetamine (5 mg, 15 mg, 30 mg) and subjects diagnosed with mild cognitive impairment receiving placebo.
Figure 28 illustrates improvements in learning in subjects diagnosed with mild cognitive impairment following treatment with l-amphetamine compared to placebo controls.
Figure 29 illustrates improvements in memory in subjects diagnosed with mild cognitive impairments following treatment with l-amphetamine compared to placebo controls.
Figure 30 illustrates improvements in executive function in subjects diagnosed with mild cognitive impairment following treatment with l-amphetamine compared to placebo controls.
Figure 31 illustrates improvement in memory and learning, as depicted by Z scores, in subjects diagnosed with mild cognitive impairment following treatment with l-amphetamine (30 mg) compared to placebo controls.
Figure 32 depicts the Step-Through Latency (sec) for rats treated with saline control (Sal); and scopolamine rats treated with varying doses of l-methamphetamine (SN522) or no l-methamphetamine (0).
Figure 33 depicts the Step-Through Latency (sec) for rats treated with saline alone (sal-sal); and scopolamine rats treated with varying doses of l-amphetamine (C105) or saline (sal). The asterisk indicates a significant difference

between group means (p<0.05).

Figure 34 depicts an improvement in memory in humans following the administration of l-methamphetamine (SN522).

Figure 35 depicts an improvement in total speed score from baseline following the administration of l-methamphetamine to humans.

Figure 36 depicts improvements in Picture Recognition/Sensitivity Index following the administration of l-methamphetamine to humans.

Figure 37 depicts an improvement of l-methamphetamine in Information Processing-Targets Detected following the administration of l-methamphetamine to humans.

Figure 38 depicts an improvement in Information Processing-False Alarms following the administration of 1-methamphetamine to humans.

DETAILED DESCRIPTION OF THE INVENTION

The features and other details of the invention, either as steps of the invention or as combinations of parts of the invention, will now be more particularly described and pointed out in the claims. It will be understood that the particular embodiments of the invention are shown by way of illustration and not as limitations of the invention. The principal features of this invention can be employed in various embodiments without departing from the scope of the invention.

The present invention relates to the discovery that the amphetamine class of compounds (collectively referred to herein as "amphetamine compounds") can be used to enhance and/or restore cognitive or memory function and performance, e.g., to improve attention, executive function, reactive time, learning, short- term memory, working memory, long- term memory, declarative memory, or procedural memory in animal subjects. More particularly, the invention relates to the discovery that particular stereoisomers of amphetamine compounds are the most effective for therapeutic use. The amphetamine compounds of the invention (e.g., R- (- )- amphetamine and R- (- )- methamphetamine) improve cognitive processes and memory (e.g., memory consolidation or the process of storing new information in long- term memory) in a human.

Furthermore, the present invention relates to the discovery that the amphetamine compounds can be used to enhance and/or restore cognitive processes such as attention span, focus, executive function, reaction time or learning in animal subjects. The compounds can be useful in improving the attention span of normal individuals, as well as improving the attention span of individuals characterized by a deficit in attention span and/or focus (e.g., individuals diagnosed with an attention deficit disorder) . Lack of attentiveness may lead to a failure to process new information and accordingly commit such new information to memory. Lack of focus may also lead to difficulties in later recalling previously processed information. Thus, deficits in attentiveness and/or focus may affect learning and memory. In addition to memory and learning difficulties, lack of attentiveness has many other negative social and behavioral consequences. Accordingly, the subject amphetamine compounds may be used to enhance and/or restore at least one of memory, learning, attentiveness, or focus.

Amphetamine is a nervous system stimulant that may mildly increase blood pressure and decreases appetite. Abuse of amphetamine has been shown to cause severe side effects including dependence and possibly induced psychosis. Amphetamine is synonymous with actedron; actemin; adderall; adipan; akedron; allodene; alpha- methyl- (±)- benzeneethanamine; alpha- methylbenzeneethanamine; alpha- methylphenethylamine; amfetamine; amphate; anorexine; benzebar; benzedrine; benzyl methyl carbinamine; benzolone; beta- amino propylbenzene; beta- phenylisopropylamine; biphetamine; desoxynorephedrine; dietamine; DL- amphetamine; elastonon; fenopromin; finam; isoamyne; isomyn; mecodrin; monophos; mydrial; norephedrane; novydrine; obesin; obesine; obetrol; octedrine; oktedrin; phenamine; phenedrine; phenethylamine, alpha- methyl- ; percomon; profamina; profetamine; propisamine; racephen; raphetamine; rhinalator; sympamine; simpatedrin; simpatina; sympatedrine; and weckamine.

The present invention relates to the use of an amphetamine composition which is enriched for eutomers of amphetamine compounds. In particular, the use of pharmaceutical preparations for improving memory consolidation in humans, include (R)- (- )- amphetamine or a derivative thereof. (R)- (- )- amphetamine (l- amphetamine, levo- amphetamine, C105) is effective at a dose one- fourth (1/4) the dose of the (S)- (+) enantiomer (d- amphetamine, dexo- amphetamine) of amphetamine. In addition, unlike (S)- (+)- amphetamine, the ®)- (- ) enantiomer has not been shown to be addictive and does not produce undesirable side effects such as increased activity, increased blood pressure or increased heart rate.

In the invention the therapeutic preparation is enriched to provide predominantly one enantiomer of a subject compound, so the enantiomerically enriched mixture can comprise an amphetamine compound that is at least about 60 w/w or mole percent, about 75 w/w or mole percent, about 80 w/w or mole percent, about 85 w/w or mole percent, about 90 w/w or mole percent, about 95 w/w or mole percent or about 99 w/w or mole percent l-amphetamine relative to d-amphetamine. In another embodiment, the amphetamine compound employed in the methods is about 100 w/w or mole percent l-amphetamine. In preferred embodiments, the amphetamine compound provided in the formulation is at least about 60 percent (w/w or mole percent) of the eutomer relative to the distomer of the amphetamine compound, and

more preferably at least about 75 w/w or mole percent, about 80 w/w or mole percent, about 85 w/w or mole percent, about 90 w/w or mole percent, about 95 w/w or mole percent or about 99 w/w or mole percent. Furthermore, the present invention is based on using the subject compounds for enhancing or restoring attention span and/or focus. The effects of the subject compounds on attention span may have secondary consequences on the ability to process and/or recall information, and therefore may also enhance memory and/or learning.

The amphetamine compounds can also be provided in the form of pharmaceutical salts.

In certain embodiments, the method includes administering, conjointly with the pharmaceutical preparation, one or more of a neuronal growth factor, a neuronal survival factor, and a neuronal trophic factor. Additionally or alternatively, a subject compound may be administered in conjunction with a cholinergic, adrenergic, nonadrenergic, dopaminergic, or glutaminergic modulator. Other agents directed at modulating GABA, NMDA, cannabinoid, AMPA, kainate, phosphodiesterase (PDE), PKA, PKC, CREB or nootropic systems may be important to the improvement of cognitive function and may be administered in conjunction with a subject compound. An agent to be administered conjointly with a subject compound may be formulated together with a subject compound as a single pharmaceutical preparation, e.g., as a pill or other medicament including both agents, or may be administered as a separate pharmaceutical preparation.

In another aspect, the present invention provides pharmaceutical preparations comprising, as an active ingredient, an enantiomerically enriched preparation of R-(-) amphetamine or a derivative thereof. The amphetamine compound is formulated in an amount sufficient to improve memory consolidation in an animal. The preparations are treatments using amphetamine compounds effective for human and/or animal subjects. In addition to humans, other animal to which the invention is applicable extend to both domestic animals and livestock, raised either as pets or for commercial purposes. Examples are dogs, cats, cattle, horses, sheep, hogs, and goats.

Still another aspect of the invention relates to the use of enantiomerically enriched preparations of amphetamine compounds for lessening the severity or prophylactically preventing the occurrence of learning and/or memory defects in an animal, and thus, altering the learning ability and/or memory capacity of the animal. As a result, the compounds of the present invention may be useful for treating and/or preventing memory impairment, in Multiple Sclerosis.

The invention also relates to the conjoint use of an amphetamine compound with agents that mimic or stimulate PKC and/or PKA pathways.

In another embodiment, the invention is useful in a method of treating an impairment in cognitive processes. Cognition is also referred to herein as a cognitive process or a cognitive function. Using standard cognition testing criteria, one of skill in the art would be capable of determining whether a person has an impairment in a cognitive process, the degree of cognitive impairment and an improvement in cognition following treatments by the methods described herein.

The impairment in a cognitive process can be in a human having multiple sclerosis.

Impairment in a cognitive function treated by the methods described herein can be an impairment in attention, which is the capacity or process of selecting out of the totality of available sensory or affective stimuli, those stimuli that are most appropriate or desirable for focus at a given time (Kinchla, R.A., et al., Annu. Rev. Psychol. 43:711-742 (1992)). The impairment in a cognitive process can be an impairment in executive function, which are neuropsychological functions such as decision making, planning, initiative, assigning priority, sequencing, motor control, emotional regulation, inhibition, problem solving, planning, impulse control, establishing goals, monitoring results of action and self-correcting (Elliott, R., Br. Med. Bull. 65:49-59 (2003)). The cognitive impairment can be an impairment in alertness, wakefulness, arousal, vigilance, and reaction time information processing, conceptualization, problem solving and/or verbal fluency. One of skill in the art would be capable of identifying and evaluating the impairment in a cognitive function in the individual.

Definitions

For convenience, certain terms employed in the specification, examples, and appended claims are collected here.

As used herein, the term "amphetamine compounds" is meant to include amphetamine, analogs of amphetamine, enantiomerically or isomerically enriched amphetamine, and enantiomerically or isomerically enriched analogs of amphetamine, as well as pharmaceutically acceptable salts of such compounds and prodrugs. In particular, amphetamine compounds of the invention, or analogs thereof which are administered to the human having an impairment in memory (impairment in memory consolidation, impairment in short-term memory, an impairment in working memory), include compounds having the structure as given in Formulas I, II, III, IV, V and VI above.

The term "amphetamine," such as is used when referring to "l-amphetamine" and "d-amphetamine," means a compound having Formula VII, including its salts, acids, esters, amides, carbamates, Schiff bases, prodrugs and other structural and functional derivatives thereof. "An amphetamine" can be in the form of the free base, salt, acid, ester, amide, carbamate, Schiff base, prodrug and other structural and functional derivatives of amphetamine or any combination thereof. In a preferred embodiment, the amphetamine is the compound represented by the Formula shown below including salts, acids, esters, amides, carbamates and Schiff bases. In another preferred embodiment, the amphetamine is the compound represented by the Formula shown below, including its salts and acids. In still another preferred

embodiment, the amphetamine is the compound of following Formula .

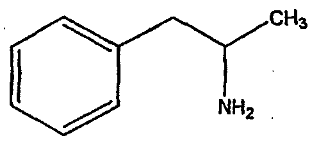

The term "methamphetamine," such as is used when referring to "l-methamphetamine" and "d-methamphetamine," means a compound having the Formula above VIII, including its salts, acids, esters, amides, carbamates, Schiff bases, prodrugs and other structural and functional derivatives thereof. "A methamphetamine" can be in the form of the free base, salt, acid, ester, amide, carbamate, Schiff base, prodrug and other structural and functional derivatives of methamphetamine or any combination thereof. In a preferred embodiment, the methamphetamine is the compound represented by the Formula above including salts, acids, esters, amides, carbamates and Schiff bases. In another preferred embodiment, the methamphetamine is the compound represented by the Formula above, including its salts and acids. In still another preferred embodiment, the methamphetamine is the compound represented by the Formula below.

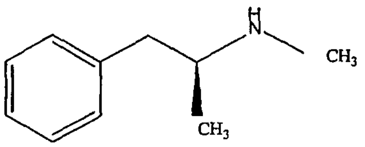

VIII

The dextro enantiomer of amphetamine is referred to in the art as the d, (+), D or S isomer and is represented by the general formula:

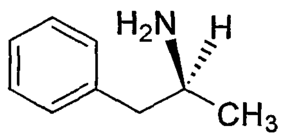

The levo enantiomer of amphetamine is referred to in the art as the l, (-), L or R isomer and is represented by the general formula:

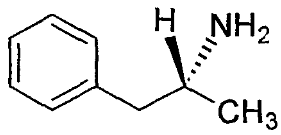

The racemic mixtures may be referred to as d,l or (+,-) or (±) or DL or (R)(S).

The term "$ED_{50}$" means the dose of a drug which produces 50% of its maximum response or effect.

An "effective amount" of, e.g., an amphetamine compound, with respect to the subject method of treatment, refers to an amount of the activator in a pharmaceutical preparation which, when applied as part of a desired dosage regimen brings about enhanced memory (memory consolidation, short term memory, working memory, declarative memory, or procedural memory) according to clinically acceptable standards.

The term "$LD_{50}$" means the dose of a drug which is lethal in 50% of test subjects.

A "patient" or "subject" to be treated by the subject method can mean either a human or non-human animal.

The term "therapeutic index" refers to the therapeutic index of a drug defined as $LD_{50}/ED_{50}$.

By "transdermal patch" is meant a system capable of delivery of a drug to a patient via the skin, or any suitable external surface, including mucosal membranes, such as those found inside the mouth. Such delivery systems generally comprise a flexible backing, an adhesive and a drug retaining matrix, the backing protecting the adhesive and matrix and the adhesive holding the whole on the skin of the patient. On contact with the skin, the drug-retaining matrix delivers drug to the skin, the drug then passing through the skin into the patient's system.

The term "adrenergic" refers to neurotransmitters or neuromodulators chemically related to adrenaline (epinephrine) or to neurons which release such adrenergic mediators. Examples are dopamine, norepinephrine, epinephrine.

Such agents are also referred to as catecholamines, which are derived from the amino acid tyrosine.

The term "biogenic amines" refers to a class of neurotransmitters which include catecholamines (e.g., dopamine, norepinephrine, and epinephrine) and serotonin.

The term "cholinergic" refers to neurotransmitters or neuromodulators chemically related to choline or to neurons which release such cholinergic mediators.

The term "dopaminergic" refers to neurotransmitters or neuromodulators chemically related to dopamine or to neurons which release such dopaminergic mediators.

The term "dopamine" refers to an adrenergic neurotransmitter, as is known in the art.

The term "metabolites" refers to active derivatives produced upon introduction of a compound into a biological milieu, such as a patient L-amphetamine and l-methamphetamine employed in the methods of the invention are not metabolites resulting from the administration of l-deprenyl. The oral administration of l-amphetamine or l-methamphetamine means ingestion of l-amphetamine and/or l-methamphetamine by the subject (e.g., human) not a metabolite of another ingested compound such as 1-deprenyl. Humans with impairments in a cognitive function or memory are treated with amphetamine and/or methamphetamine, wherein the amphetamine and/or methamphetamine is enantiomerically enriched for l-amphetamine of l-methamphetamine relative to the total content of amphetamine and/or methamphetamine in the composition, wherein the l-amphetamine and/or l-methamphetamine is not administered as l-deprenyl or a result of the metabolism of l-deprenyl in the human.

An agent to be administered conjointly with a subject compound may be formulated together with a subject compound as a single pharmaceutical preparation, e.g., as a pill or other medicament including both agents, or may be administered as a separate pharmaceutical preparation.

In another aspect, the present invention provides pharmaceutical preparations comprising, as an active ingredient amphetamine. The subject amphetamine compound is formulated in an amount sufficient to improve LTP in an animal. The subject preparations and methods can be treatments using amphetamine compounds effective for human and/or animal subjects. In addition to humans, other animal subjects to which the invention is applicable extend to both domestic animals and livestock, raised either as pets or for commercial purposes. Examples are dogs, cats, cattle, horses, sheep, hogs, and goats.

Still another aspect of the invention relates to the use of amphetamine compounds for lessening the severity or prophylactically preventing the occurrence of cognitive, learning and/or memory defects in an animal, and thus, altering the cognitive, learning ability and/or memory capacity of the animal. As a result, the compounds of the present invention may be useful for treating and/or preventing cognitive or memory impairment, e.g., due to toxicant exposure, brain injury, brain aneurysm, age-associated memory impairment, in Multiple Sclerosis.

The present invention also relates to treatment with at least one member selected from the group consisting of 1-amphetamine or, 1-methamphetamine.

The cognitive and/or memory processes and impairments in cognitive and/or memory processes can be assessed or determined by established techniques. For example, memory can be assessed before, concomitantly with or after treatment of the individual with at least one member selected from the group consisting of l- amphetamine, l-methamphetamine, l- threo- methylphenidate, d- threo- methylphenidate, methylphenidate, atomoxetine and modafinil by one or more well established tests known to one of skill in the art. Such tests include the Rey Auditory Verbal Learning Test (RAVLT) ; Cambridge Neuropsychological Test Automated Battery (CANTAB) ; a Children's Memory Scale (CMS) ; a Contextual Memory Test; a Continuous Recognition Memory Test (CMRT) ; a Denman Neuropsychology Memory Scale; a Fuld Object Memory Evaluation (FOME) ; a Graham- Kendall Memory for Designs Test; a Guild Memory Test; a Learning and Memory Battery (LAMB) ; a Memory Assessment Clinic Self- Rating Scale (MAC- S) ; a Memory Assessment Scales (MAS) ; a Randt Memory Test; a Recognition Memory Test (RMT) ; a Rivermead Behavioral Memory Test; a Russell's Version of the Wechsler Memory Scale (RWMS) ; a Test of Memory and Learning (TOMAL) ; a Vermont Memory Scale (VMS) ; a Wechsler Memory Scale; a Wide Range Assessment of Memory and Learning (WRAML) ; First- Last Name Association (Youngjohn J.R., et al., Archives of Clinical Neuropsychology 6: 287- 300 (1991) ) ; Name-Face Association; Wechsler Memory Scale- Revised (Wechsler, D., Wechsler Memory Scale- Revised Manual, NY, NY, The Psychological Corp. (1987) ) ; California Verbal Learning Test- Second Edition (Delis, D.C., et al., The Californian Verbal Learning Test, Second Edition, Adult Version, Manual, San Antonio, TX: The Psychological Corporation (2000) ) ; Facial Recognition (delayed non- matching to sample) ; Cognitive Drug Research (CDR) Computerized Assessment Battery- Wesnes; Buschke's Selective Reminder Test (Buschke, H., et al., Neurology 24: 1019- 1025 (1974) ) ; Telephone Dialing Test; Brief Visuospatial Memory Test- Revised; and Test of Everyday Attention (Perry, R.J., et al., Neuropsychologia 38: 252- 271 (2000) ) .

In one embodiment, the individual (also referred to herein as a "subject") can have an impairment in memory. The impairment in memory can be an impairment in memory consolidation, the process of storing new information in long term memory ("Neuroscience: Exploring The Brain," Bear, M.F. et al., Williams & Wilkins, Baltimore, Maryland, Ch. 19, pp. 517-545 (1996); McGaugh, J.L. Science 287:248-251 (2000)). Alternatively, or additionally, the impairment in memory can be an impairment in short-term memory or an impairment in working memory. Short-term memory and

working memory are processes whereby newly acquired information is maintained for short periods of time and the newly acquired information is made available for further information processing ("Neuroscience: Exploring The Brain," Bear, M.F. et al., Williams & Wilkins, Baltimore, Maryland, Ch. 19, pp. 517-545 (1996); McGaugh, J.L. Science 287: 248-251 (2000); Becker, J.T., et al., Brain and Cognition 41:1-8 (1999)).

The impairment in memory can also be an impairment in declarative memory, which is the memory of facts and events ("Neuroscience: Exploring The Brain," Bear, M.F. et al., William & Wilkins, Baltimore, Maryland, Ch. 19, pp. 517-545 (1996); McGaugh, J.L. Science 287: 248-251 (2000); Tulving, E., et al., Science 247: 301-306 (1990); Squire, L.R., et al., Proc. Natl. Acad. Sci. 93: 13515-13522 (1996)). The impairment in memory can also be an impairment in procedural memory (also referred to as "tacit knowledge" or "implicit knowledge"), which is the memory for skills or behavior ("Neuroscience: Exploring The Brain," Bear, M.F. et al., Williams & Wilkins, Baltimore, Maryland, Ch. 19, pp. 517-545 (1996); McGaugh, J.L. Science 287: 248-251 (2000)). The impairment can also be an impairment in attention, acquisition, retrieval or retention. One of skill in the art would be capable of identifying and evaluating the impairment in memory in the individual.

In another embodiment, the individual can have an impairment in a cognitive process (Carlson, N.R., Physiology of Behavior, Allyn and Bacon, Boston, MA (1986); Cognition on Cognition, eds., Mehler, J. et al., Bradford Books (1995)). The impairment in a cognitive process can be an impairment in attention, which is the capacity or process of selecting out of the totality of available sensory or affective stimuli, those stimuli that are most appropriate or desirable for focus at a given time (Kinchla, R.A., et al., Annu. Rev. Psychol. 43: 711-742 (1992)). The impairment in a cognitive process can be an impairment in executive function, which are neuropsychological functions such as decision making, planning, initiative, assigning priority, sequencing, motor control, emotional regulation, inhibition, problem solving, planning, impulse control, establishing goals, monitoring results of action and self-correcting (Elliott, R., Br. Med. Bull. 65:49-59 (2003)). The cognitive impairment can be an impairment in alertness, wakefulness, arousal, vigilance, reaction time, attention, information processing, conceptualization, and verbal fluency. One of skill in the art would be capable of identifying and evaluating the impairment in cognition in the individual.

The compounds employed in the methods of the invention can be the free base or can exist as salts with pharmaceutically acceptable acids. Examples of such salts include hydrochlorides, hydrobromides, sulfates, methanesulfonates, nitrates, maleates, acetates, citrates, fumarates, tartrates (e.g., (+)- tartrates, (- )- tartrates or mixtures thereof including racemic mixtures), succinates, benzoates and salts with amino acids such as glutamic acid.

In another embodiment, the single dosage formulation is at least about 0.001 mg, about 0.01 mg, about 0.1 mg, about 1 mg, about 2.5 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 75 mg, about 80 mg, about 85 mg, about 90 mg, about 95 mg, about 100 mg, about 125 mg, about 150 mg, about 200 mg, about 300 mg, about 400 mg, about 500 mg, about 750 mg, or about 1000 mg of the compound (e.g., l-amphetamine, C105, l-methamphetamine, SN522, SN522-HCl).

In still another embodiment, the invention employ multiple doses of the compound (e.g., 1-amphetamine, C105, 1-methamphetamine, SN522, SN522-HCl). Each dose of the multiple dose is at least about 0.001 mg, about 0.01 mg, about 0.1 mg, about 1 mg, about 2.5 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 75 mg, about 80 mg, about 85 mg, about 90 mg, about 95 mg, about 100 mg, about 125 mg, about 150 mg, about 200 mg, about 300 mg, about 400 mg, about 500 mg, about 750 mg or about 1000 mg of the compound (e.g., l-amphetamine, C105, l-methamphetamine, SN522, SN522-HCl). The multiple doses can be administered for a day, days, a week, weeks, a month, months or years.

The compounds employed in the invention can be administered to a human acutely (briefly or short-term) or chronically (prolonged or long-term). For example, the compounds, (e.g., l-amphetamine, C105, l-methamphetamine, SN522, SN522-HCl) of the invention can be used in methods to treat a human by administering the compound to the human once a day, multiple times (e.g., 2, 3, 4) in a day, for a day, days, a week, weeks, a month, months or years.

In yet another embodiment of the invention, the compounds are provided in a single oral dosage formulation of between about 0.001 mg to about 125 mg; between about 0.001 mg to about 250 mg; between 0.001 mg to 500 mg; between about 0.01 mg to about 125 mg; between about 0.1 mg to about 125 mg; between about 1 mg to about 125 mg; between about 1 mg to about 250 mg; between about 1 mg to about 500 mg; or between about 1 mg to about 1000 mg of the compound employed in the methods (e.g., 1-amphetamine, C105, 1-methamphetamine, SN522, SN522-HCl) and, optionally, a pharmaceutically acceptable carrier.

In a further embodiment, the invention employ multiple doses of the compound (e.g., 1- amphetamine, C105, 1- methamphetamine, SN522, SN522- HCl) wherein each of the multiple doses of the compound is between about 0.001 mg to about 125 mg; or between about 0.001 mg to about 250 mg; or between about 0.001 mg to about 500 mg; or between about 0.01 mgto about 125 mg; or between about 0.1 mg to about 125 mg; or between about 0.01 mg; to about 500 mg; or between about 1 mg to about 125 mg; or between about 1 mg to about 500 mg; or between about 2.5 mg to about 25 mg, about 50 mg, about 75 mg, about 100 mg, about 125 mg, about 250 mg, about 500 mg or about 1000

mg of the compound (s) (e.g., l- amphetamine, C105, l- methamphetamine, SN522, SN522- HCl), and, optionally, a pharmaceutically acceptable carrier.

In a further embodiment, the invention employs a single dose of the compound (e.g., l-amphetamine, C105, l-methamphetamine, SN522, SN522-HCl) between about 0.0015mg/kg to about 2 mg/kg; or between about 0.015 mg/kg to about 2 mg/kg.

In yet another embodiment, the invention employs a single dose about 0.04 mg/kg, about 0.07 mg/kg, about 0.15 mg/kg, about 0.20 mg/kg, about 0.40 mg/kg, about 0.65 mg/kg, about 1 mg/kg, about 1.50 mg/kg, about 1.80 mg/kg or about 3.5 mg/kg of the compound (e.g., l-amphetamine, C105, l-methamphetamine, SN522, SN522-HCl).

In an additional embodiment, the invention employs multiple doses of the compound (e.g., 1- amphetamine, C105, 1- methamphetamine, SN522, SN522- HCl), wherein each dose of the multiple dose is between about 0.0015 mg/kg to about 2 mg/kg; or between about 0.015 mg/kg to about 2 mg/kg.

In still another embodiment, the invention employs multiple doses, wherein each does of the multiple dose is about 0.04 mg/kg, about 0.07 mg/kg, about 0.15 mg/kg, about 0.20 mg/kg, about 0.40 mg/kg, about 0.65 mg/kg, about 1 mg/kg, about 1.50 mg/kg, about 1.80 mg/kg or about 3.5 mg/kg of the compound (e.g., 1-amphetamine, C105, 1-methamphetamine, SN522, SN522-HCl).

The cumulative dose of the compounds (e.g., 1-amphetamine, C105, 1-methamphetamine, SN522, SN522-HCl) employed in the invention, regardless of whether the compound is administered in a single dose or in multiple doses is between about 0.2 mg to about 250 mg; or between about 1 mg to about 1250 mg of the compound(s). In a particular embodiment, the cumulative dose is about 2 mg, about 10 mg, about 20 mg, about 30 mg, about 50 mg, about 60 mg, about 90 mg, about 100 mg, about 150 mg, about 200 mg, about 250 mg, about 450 mg, about 750 mg, about 1000 mg, about 1250 mg, about 2500 mg, or about 5000 mg.

The multiple doses or cumulative dose of the compound can be any combination of a compound of the invention (e.g., 1-amphetamine, C105, 1-methamphetamine, SN522, SN522-HCl) in any combination of dose or doses.

An "effective amount" or "amount effective, " when referring to the amount of the compound (e.g., 1- amphetamine, C105, 1- methamphetamine, SN522, SN522- HCl), administered to the individual, is defined as that amount, or dose, of the compound that, when administered to an individual having an impairment in memory as a consequence of exposure to a muscarinic cholinergic receptor antagonist, is sufficient for therapeutic efficacy (e.g., an amount sufficient to improve memory in an individual having an impairment in memory; an amount sufficient to improve cognition in an individual having an impairment in cognition) .

The present invention can be accomplished by the administration of the compounds (e.g., l-amphetamine, C105, l-methamphetamine, SN522, SN522-HCl) of the invention by enteral or parenteral means. Specifically, the route of administration can be by oral ingestion (e.g., tablet, capsule form) or injection (e.g., intramuscular) of the compound. Other routes of administration are also useful in the present invention including intravenous, intraarterial, intraperitoneal, subcutaneous routes or nasal administration. Suppositories or transdermal patches can also be employed.

The compounds employed in the invention can be administered alone or can be coadministered to the human. Coadminstration is meant to include simultaneous or sequential administration of one or more of the compounds (l-amphetamine, C105, 1- methamphetamine, SN522, SN522- HCl) individually or in combination. The simultaneous or sequential administration of compounds of the invention is conducted so that the mode of administration and the timing of administration results in a maximal improvement in memory (memory consolidation, short term memory, working memory) or cognition (e.g., attention, executive function, alertness, wakefulness, arousal, conceptualization, information processing, problem solving, verbal fluency) with minimal side effects (e.g., addiction, increases in heart rate, increases in blood pressure) . It is also envisioned that multiple routes of administration (e.g., oral, transdermal, suppository, intramuscular) can be used to administer l- amphetamine, C105, l- methamphetamine, SN522, SN522- HCl)

The dosage and frequency (single or multiple doses) administered to an individual can vary depending upon a variety of factors, including the duration of exposure to the muscarinic cholinergic receptor antagonist and severity of the impairment in memory (e.g., impairment in memory consolidation, impairment in short- term memory, an impairment in working memory) or cognition (e.g., attention, alertness, executive function, wakefulness, arousal, vigilance, executive function, reaction time); size, age, sex, health, body weight, body mass index and diet of the human; nature and extent of symptoms of the impairment in memory or cognition, kind of concurrent treatment (e.g., atropine, scopolamine), complications from exposure to the muscarinic cholinergic receptor antagonist, or other health-related problems of the human being treated.

Other therapeutic regimens or agents can be used in conjunction with the compounds of the invention. Adjustment and manipulation of established dosages (e.g., frequency and duration) are well within the ability of those skilled in the art.

The invention also relates to the conjoint use of a amphetamine compound with agents that mimic or stimulate PKC and/or PKA pathways.

A. Synthesis of Amphetamine compounds

As described in further detail below, it is contemplated that the subject methods can be carried out using a stereomerically enriched preparation in a eutomer of amphetamine compound (s), particularly R- (- )- amphetamine, or a variety of different derivatives thereof. The suitability of use of a particular amphetamine compound can be readily determined, for example, by such drug screening assays as described herein.

The subject amphetamine compounds, and derivatives thereof, can be prepared readily by employing known synthetic methodology. As is well known in the art, these coupling reactions are carried out under relatively mild conditions and tolerate a wide range of "spectator" functionality. Additional compounds may be synthesized and tested in a combinatorial fashion, to facilitate the identification of additional amphetamine compounds which may be employed in the subject method.

Numerous methods for synthesizing amphetamine and for resolving the enantiomers of amphetamine have been described in the art, see for example: US Patent No. 5,075,338 to Knoll et al.; US Patent No. 2,828,343 to Tindall; US Patent No. 3,458,576 to Bryan; UK Patent No. GB 2,122,617; US Patent No. 3,996,381 to Florvall et al.; Croce et al., 1996, Gazz. Chim. Ital. 126:107-109; Mastagli et. al., 1950, Bull. Soc. Chim. Fr. 1045-1047; Smith et al.., 1988, J. Med. Chem. 31:1558-1566; Bobranskii et al., 1941, J. Applied Chem. (U.S.S.R.) 14:410-414; Magidson, 1941, J. Gen. Chem. (U.S.S.R.) 11:339-343.

In one embodiment, a subject amphetamine compound can be synthesized according to the methods set forth in US Patent 5,075,338. Briefly, amphetamine compounds of the general formula:

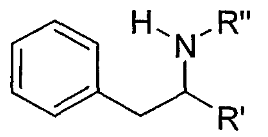

can be prepared by reacting a ketone of the formula:

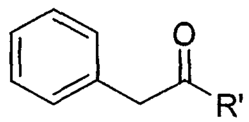

with an amine of the formula: $R''NH_2$ and reducing the ketimine intermediate formed without or after isolation. The reduction can be carried out by methods known per se, e.g., by catalytic hydrogenation (preferably in the presence of a palladium or Raney-nickel catalyst) or by using a complex metal hydride (e.g. sodium borohydride) or with the aid of a conventional reducing agent (e.g. sodium dithionite or amalgamated aluminum).

R- (- )- amphetamine and S- (+)- amphetamine may be obtained by optical resolution of racemic mixtures of R- and S- enantiomers of amphetamine. Such a resolution can be accomplished by any conventional resolution methods well known to a person skilled in the art, such as those described in J. Jacques, A. Collet and S. Wilen, "Enantiomers, Racemates and Resolutions, " Wiley, N.Y. (1981) . For example, the resolution may be carried out by preparative chromatography on a chiral column. Another example of a suitable resolution method is the formation of diastereomeric salts with a chiral acid such as tartaric, malic, mandelic acid or N- acetyl derivatives of amino acids, such as N- acetyl leucine, followed by recrystallization to isolate the diastereomeric salt of the desired R enantiomer.

In one embodiment, a subject R- (- )- amphetamine may be resolved according to the methods set forth in J. Med. Chem, 1988, 31: 1558: 1570. Briefly, racemic amphetamine is combined with a hot ethanol solution of D- (- )- tartaric acid. The solution is allowed to cool to room temperature and the white crystals are collected and recrystallized twice more from ethanol to give D- tartaric acid salt of R- (- )- amphetamine. To recover R- (- )- amphetamine, the D- tartaric acid salt of R- (- )- amphetamine is treated with sodium hydroxide in water and extracted with diethyl ether.

The compounds of the present invention, particularly libraries of amphetamine analogs having various representative classes of substituents, are amenable to combinatorial chemistry and other parallel synthesis schemes (see, for example, PCT WO 94/08051). The result is that large libraries of related compounds, e.g., a variegated library of compounds represented above, can be screened rapidly in high throughput assays in order to identify potential amphetamine analogs, as well as to refine the specificity, toxicity, and/or cytotoxic-kinetic profile of a lead compound.

Simply for illustration, a combinatorial library for the purposes of the present invention is a mixture of chemically related compounds which may be screened together for a desired property. The preparation of many related compounds in a single reaction greatly reduces and simplifies the number of screening processes which need to be carried out. Screening for the appropriate physical properties can be done by conventional methods.

Diversity in the library can be created at a variety of different levels. For instance, the substrate aryl groups used in the combinatorial reactions can be diverse in terms of the core aryl moiety, e.g., a variegation in terms of the ring structure, and/or can be varied with respect to the other substituents.

A variety of techniques are available in the art for generating combinatorial libraries of small organic molecules such as the subject amphetamine compounds. See, for example, Blondelle et al. (1995) Trends Anal. Chem. 14:83; the Affymax U.S. Patents 5,359,115 and 5,362,899: the Ellman U.S. Patent 5,288,514: the Still et al. PCT publication WO 94/08051; the ArQule U.S. Patents 5,736,412 and 5,712,171; Chen et al. (1994) JACS 116:2661: Kerr et al. (1993) JACS 115:252; PCT publications WO92/10092, WO93/09668 and WO91/0708; and the Lemer et al. PCT publication WO93/20242). Accordingly, a variety of libraries on the order of about 100 to 1,000,000 or more diversomers of the subject amphetamine compounds can be synthesized and screened for particular activity or property.

In an exemplary embodiment, a library of candidate amphetamine compound diversomers can be synthesized utilizing a scheme adapted to the techniques described in the Still et al. PCT publication WO 94/08051, e.g., being linked to a polymer bead by a hydrolyzable or photolyzable group, optionally located at one of the positions of the candidate regulators or a substituent of a synthetic intermediate. According to the Still et al. technique, the library is synthesized on a set of beads, each bead including a set of tags identifying the particular diversomer on that bead. The bead library can then be "plated" with cells for which an amphetamine compound is sought. The diversomers can be released from the bead, e.g., by hydrolysis.

Many variations on the above and related pathways permit the synthesis of widely diverse libraries of compounds which may be tested as amphetamine compounds.

B. Generation of Animal Models to Test Agents

Applicants have previously described an animal model for studying fornix-mediated memory consolidation. See, for example, Taubenfield et al., *supra.* The fornix-lesioned animals can be used for drug screening, e.g., to identify dosages of the subject compositions which enhance memory consolidation. The lesioned mammal can have a lesion of the fornix or a related brain structure that disrupts memory consolidation (e.g., perirhinal cortex, amygdala, medial septal nucleus, locus coeruleus, hippocampus, mammallary bodies). Lesions in the mammal can be produced by mechanical or chemical disruption. For example, the fornix lesion can be caused by surgical ablation, electrolytic, neurotoxic and other chemical ablation techniques, or reversible inactivation such as by injection of an anesthetic, e.g., tetrodotoxin or lidocaine, to temporarily arrest activity in the fornix.

To further illustrate, fimbrio-fornix (rodents) and fornix (primates) lesions can be created by stereotactic ablation. In particular, neurons of the fornix structure are axotomized, e.g., by transection or aspiration (suction) ablation. A complete transection of the fornix disrupts adrenergic, cholinergic and GABAergic function and electrical activity, and induces morphological reorganization in the hippocampal formation. In general, the fornix transection utilized in the subject method will not disconnect the parahippocampal region from the neocortex. In those embodiments, the fornix transection will not disrupt functions that can be carried out by the parahippocampal region independent of processing by the hippocampal formation, and hence would not be expected to produce the full-blown amnesia seen following more complete hippocampal system damage.

In one embodiment, the animal can be a rat. Briefly, the animals are anesthetized, e.g., with intraperitoneal injections of a ketamine-xylazine mixture and positioned in a Kopf® stereotaxic instrument. A sagittal incision is made in the scalp and a craniotomy is performed extending 2.0 mm posterior and 3.0 mm lateral from Bregma. An aspirative device, e.g., with a 20 gauge tip, is mounted to a stereotaxic frame (Kopf® Instruments) and fimbria-fornix is aspirated by placing the suction tip at the correct sterotaxic location in the animal's brain. Unilateral aspirative lesions are made by suction through the cingulate cortex, completely transecting the fimbria fornix unilaterally, and (optionally) removing the dorsal tip of the hippocampus as well as the overlying cingulate cortex to inflict a partial denervation on the hippocampus target. See also, Gage et al., (1983) Brain Res. 268:27 and Gage et al. (1986) Neuroscience 19:241.

In another exemplary embodiment, the animal can be a monkey. The animal can be anesthetized, e.g., with isoflurane (1.5- 2.0%) . Following pretreatment with mannitol (0.25 g/kg, iv), unilateral transections of the left fornix can be performed, such as described by Kordower et al. (1990) J. Comp. Neurol., 298: 443. Briefly, a surgical drill is used to create a parasagittal bone flap which exposes the frontal superior sagittal sinus. The dura is retracted and a self-retaining retractor is used to expose the interhemispheric fissure. The corpus callosum is longitudinally incised. At the level of the foramen of Monro, the fornix is easily visualized as a discrete 2- 3 mm wide white fiber bundle. The fornix can be initially transected using a ball dissector. The cut ends of the fornix can then be suctioned to ensure completeness of the lesion.

In still other illustrative embodiments, the fornix lesion can be created by excitotoxicity, or by other chemical means, inhibiting or ablating fornix neurons, or the cells of the hippocampus which are innervated by fornix neurons. In certain preferred embodiments, the fornix lesion is generated by selective disruption of particular neuronal types, such as fornix cholinergic and adrenergic neurons.

For instance, the afferant fornix signals to the hippocampus due to cholinergic neurons can be ablated by atropine blockade. Another means for ablation of the cholinergic neurons is the use of 192IgG- saporin (192IgG- sap), e.g., intraventricularly injection into the fornix and hippocampus. The agents such as 6- OHDA and ibotenic acid can be used to selectively destroy fornix dopamine neurons as part of the ablative regimen.

In one embodiment, the animal is a non-human mammal, such as a dog, cat, horse, cow, pig, sheep, goat, chicken, monkey, ape, rat, rabbit, etc. In another embodiment, the animal is a non-human primate. In still another embodiment, the subject is a human.

There are a variety of tests for cognitive function, especially learning and memory testing, which can be carried our using the lesioned and normal animals. Learning and/or memory tests include, for example, Inhibitory Avoidance Test (also referred to herein as "Passive Avoidance Test"), contextual fear conditioning, visual delay non-match to sample, spatial delay non-match to sample, visual discrimination, Barnes circular maze, Morris water maze, radial arm maze tests, Ray Auditory-Visual Learning Test, the Wechsler Logical Memory Test, and the Providence Recognition Memory Test.

An exemplary Inhibitory Avoidance Test utilizes an apparatus that consists of a lit chamber that can be separated from a dark chamber by a sliding door. At training, the animal is placed in the lit chamber for some period of time, and the door is opened. The animal moves to the dark chamber after a short delay - the step-through latency - which is recorded. Upon entry into the dark chamber, the door is shut closed and a foot shock is delivered. Retention of the experience is determined after various time intervals, e.g., 24 or 48 hours, by repeating the test and recording the latency. The protocol is one of many variants of the passive avoidance procedures (for review, see Rush (1988) Behav. Neural. Biol. 50:255).

An exemplary maze testing embodiment is the water maze working memory test. In general, the method utilizes an apparatus which consists of a circular water tank. The water in the tank is made cloudy by the addition of milk powder. A clear plexiglass platform, supported by a movable stand rest on the bottom of the tank, is submerged just below the water surface. Normally, a swimming rat cannot perceive the location of the platform but it may recall it from a previous experience and training, unless it suffers from some memory impairment. The time taken to locate the platform is measured and referred to as the latency. During the experiment, all orientational cues such as ceiling lights, etc., remain unchanged. Longer latencies are generally observed with rats with some impairment to their memory.

Another memory test includes the eyeblink conditioning test, which involves the administration of white noise or steady tone that precedes a mild air puff which stimulates the subject's eyeblink.

Still another memory test which can be used is fear conditioning, e.g., either "cued" and "contextual" fear conditioning. In one embodiment, a freeze monitor administers a sequence of stimuli (sounds, shock) and then records a series of latencies measuring the recovery from shock induced freezing of the animal.

Another memory test for the lesioned animals is a holeboard test, which utilizes a rotating holeboard apparatus containing (four) open holes arranged in a 4-corner configuration in the floor of the test enclosure. A mouse is trained to poke its head into a hole and retrieve a food reward from a "baited" hole which contains a reward on every trial. There is a food reward (e.g., a Fruit Loop) in every exposed hole which is made inaccessible by being placed under a screen. The screen allows the odor of the reward to emanate from the hole, but does not allow access to the reinforcer. When an individual hole is baited, a reward is placed on top of the screen, where it is accessible. The entire apparatus rests on a turntable so that it may be rotated easily to eliminate reliance on proximal (e.g., olfactory) cues. A start tube is placed in the center of the apparatus. The subject is released from the tube and allowed to explore for the baited ("correct") hole.

As set out above, one use for the fornix-lesioned animals is for testing amphetamine compounds for ability to modulate memory consolidation, as well as for side effects and toxicity. In general, the subject method utilizes an animal which has been manipulated to create at least partial disruption of fornix-mediated signalling to the hippocampus, the disruption affecting memory consolidation and learned behavior in the animal. The animal is conditioned with a learning or memory regimen which results in learned behavior in the mammal in the absence of the fornix lesion. Amphetamine compounds are administered to the animal in order to assess their effects on memory consolidation. An increase in learned behavior, relative to the absence of the test agents, indicates that the administered combination enhances memory consolidation.

Another memory test especially developed for use in pharmaceutical studies is the Providence Recognition Memory Test. This test consists of one pictorial and one verbal assessment of long-term declarative memory. In each of the two modes, the patient views stimuli on a computer screen and is later asked to recognize those stimuli in a two-alternative forced-choice format. The pictorial assessment mode consists of two parts: a study phase and a recognition phase. In the study phase, patients view a series of 120 pictures, for 3 seconds each. They are told to look at the pictures and remember them, so that they can recognize them later. In the recognition phase, patients view pictures two at a time and are asked to indicate by button press which of the two pictures they saw in a study phase. Recognition memory testing occurs at ten minutes, one hour, and 24 hours after the end of the study phase. The verbal assessment mode consists of two parts: a study phase and a recognition phase. In the study phase, patients view a series of 60 sentences

one at a time. They are asked to read the sentences aloud and remember them, so that they can recognize them later. Each sentence remains on the computer screen until the patient has finished reading it aloud. If patients read words incorrectly, the examiner supplies the correct word or words. In the recognition phase, patients view sentences two at a time and are asked to indicate by button press which of the two sentences they saw in the study phase. Recognition memory testing occurs at ten minutes, one hour, and 24 hours after the end of the study phase.

In the present invention, retention of the learned behavior can be determined, for example, after at least about 12-24 hours, 14-22 hours, 16-20 hours and or 18-19 hours after completion of the learning phase to determine whether the agents promote memory consolidation. In a particular embodiment, retention of the learned behavior can be determined 24 hours after completion of the learning phase.

In addition to models for studying memory consolidation, models to assess side effects of amphetamine compounds on behavior have been utilized including locomotor activity models. An exemplary locomotor activity test utilizes an apparatus that consists of photocell activity cages with a grid of photocell beams placed around the cage. The animals are placed in individual activity cages some period of time prior to administration of agents. Locomotor activity is measured by the number of interruptions of the photoelectric beam during a given period of time.

As used herein, a "control mammal" can be an untreated lesion mammal (i.e., a lesion animal receiving no agents or not the same combinations to be assessed), a trained control mammal (i.e., a mammal that undergoes training to demonstrate a learned behavior without any lesion) and/or an untrained control mammal (i.e., a mammal with or without a lesion, that receives no training to demonstrate a learned behavior).

C. Pharmaceutical preparations of amphetamine compounds

In another aspect, the present invention provides pharmaceutical preparations comprising the subject amphetamine compounds. The amphetamine compounds for use in the subject method may be conveniently formulated for administration with a biologically acceptable, non-pyrogenic, and/or sterile medium, such as water, buffered saline, polyol (for example, glycerol, propylene glycol, liquid polyethylene glycol and the like) or suitable mixtures thereof. The optimum concentration of the active ingredient(s) in the chosen medium can be determined empirically, according to procedures well known to behavioral scientists. As used herein, "biologically acceptable medium" includes any and all solvents, dispersion media, and the like which may be appropriate for the desired route of administration of the pharmaceutical preparation. The use of such media for pharmaceutically active substances is known in the art. Except insofar as any conventional media or agent is incompatible with the activity of the amphetamine compounds, its use in the pharmaceutical preparation of the invention is contemplated. Suitable vehicles and their formulation inclusive of other proteins are described, for example, in the book *Remington's Pharmaceutical Sciences* (Remington's Pharmaceutical Sciences. Mack Publishing Company, Easton, Pa., USA 1985). These vehicles include injectable "deposit formulations".

Pharmaceutical formulations of the present invention can also include veterinary compositions, e.g., pharmaceutical preparations of the amphetamine compounds suitable for veterinary uses, e.g., for the treatment of livestock or domestic animals, e.g., dogs.

Methods of introduction may also be provided by rechargeable or biodegradable devices. Various slow release polymeric devices have been developed and tested *in vivo* in recent years for the controlled delivery of drugs. A variety of biocompatible polymers (including hydrogels), including both biodegradable and non-degradable polymers, can be used to form an implant for the sustained release of a amphetamine compound at a particular target site. In accordance with the practice of this invention, it has been found that a dosage form and a method can be provided that administers a amphetamine compound in a program that substantially lessens or completely compensates for tolerance in a patient. Tolerance, as defined in Pharmacology in Medicine, by Brill, p. 227 (1965) McGraw-Hill, is characterized as a decrease in effect followed by administering a drug. When tolerance develops following a single dose or a few doses over a very short time, it is referred to as acute tolerance. When the drug is administered over a more protracted period of time to show a demonstrable degree of tolerance, it is referred to as chronic tolerance. The medical literature, as exemplified in, The Pharmacological Bases of Therapeutics, by Goodman and Gilman, 8th Ed., p. 72 (1990) Pergamon Press, reported tolerance may be acquired to the effects of many drugs and this literature classifies tolerance as acute or chronic based on when it is acquired. That is, acute tolerance develops during a dosing phase of one dose or on one day, and chronic tolerance is acquired due to chronic administration typically weeks, months, and years.

In certain embodiments, particularly where the selected amphetamine compound is one which may produce tolerance, e.g., acute tolerance, in the patient, it may desirable to formulate the compound for variable dosing, and preferably for use in a dose-escalation regimen. In preferred embodiments, the subject amphetamine compounds are formulated to deliver a sustained and increasing dose, e.g., over at least 4 hours, and more preferably over at least 8 or even 16 hours.

In certain embodiments, representative dosage forms include hydrogel matrix containing a plurality of tiny pills. The hydrogel matrix comprises a hydrophilic polymer, such as selected from the group consisting of a polysaccharide, agar, agarose, natural gum, alkali alginate including sodium alginate, carrageenan, fucoidan, furcellaran, laminaran,

hypnea, gum arabic, gum ghatti, gum karaya, gum tragacanth, locust bean gum, pectin, amylopectin, gelatin and a hydrophilic colloid. The hydrogel matrix comprises a plurality of tiny pills (such as 4 to 50), each tiny pill comprising an increasing dose population of from 100 ng ascending in dose such as 0.5 mg, 1 mg, 1.2 mg, 1.4 mg, 1.6 mg, 1.8 mg, etc. The tiny pills comprise a release rate controlling wall of 0.0 mm to 10 mm thickness to provide for the timed ascending release of drug. Representative of wall-forming materials include a triglyceryl ester selected from the group consisting of glyceryl tristearate, glyceryl monostearate, glyceryl dipalmitate, glyceryl laureate, glyceryl didecenoate and glyceryl tridecenoate. Other wall forming materials comprise polyvinyl acetate phthalate, methylcellulose phthalate, and microporous vinyl olefins. Procedures for manufacturing tiny pills are disclosed in U.S. Pat. Nos. 4,434,153; 4,721,613; 4,853,229; 2,996,431; 3,139,383 and 4,752,470.

In certain embodiments, the drug releasing beads are characterized by a dissolution profile wherein 0 to 20% of the beads undergo dissolution and release the drug in 0 to 2 hours, 20 to 40% undergo dissolution and release the drug in 2 to 4 hours, 40 to 60% exhibit dissolution and release in 4 to 6 hours, 60 to 80% in 6 to 8 hours, and 80 to 100% in 8 to 10 hours. The drug releasing beads can include a central composition or core comprising a drug and pharmaceutically acceptable composition forming ingredients including a lubricant, antioxidant, and buffer. The beads comprise increasing doses of drug, for example, 1 mg, 2 mg, 5 mg, and so forth to a high dose, in certain preferred embodiments, of 15 to 100 mg. The beads are coated with a release rate controlling polymer that can be selected utilizing the dissolution profile disclosed above. The manufacture of the beads can be adapted from, for example, Liu et al. (1994) Inter. J. of Pharm., 112:105-116; Liu et al. (1994) Inter. J. of Pharm., 112:117-124; Pharm. Sci., by Remington, 14th Ed. pp. 1626-1628 (1970); Fincher et al. (1968) J. Pharm. Sci., 57:1825-1835; and U.S. Pat. No. 4,083,949.

Another exemplary dosage form provided by the invention comprises a concentration gradient of amphetamine compound from 1 mg to 15-600 mg coated from the former low dose to the latter high dose on a polymer substrate. The polymer can be erodible or a nonerodible polymer. The coated substrate is rolled about itself from the latter high dose at the center of the dosage form, to the former low dose at the exposed outer end of the substrate. The coated substrate is rolled from the high dose to the low dose to provide for the release of from low to high dose as the substrate unrolls or erodes. For example, 1 mg to 600 mg of amphetamine is coated onto an erodible polymer such as an polypeptide, collagen, gelatin, or polyvinyl alcohol, and the substrate rolled concentrically from the high dose rolled over and inward to adapt a center position, and then outward towards the low dose to form an outer position. In operation, the dosage form erodes dispensing an ascending dose of amphetamine that is released over time.

Another dosage form provided by the invention comprises a multiplicity of layers, wherein each layer is characterized by an increasing dose of drug. The phrase "multiplicity of layers" denotes 2 to 6 layers in contacting lamination. The multiplicity of layers are positioned consecutively, that is, one layer after another in order, with a first exposed layer, the sixth layer in contact with the fifth layer and its exposed surface coated with a drug impermeable polymer. The sixth layer is coated with a drug impermeable polymer to insure release of the amphetamine compound from the first layer to the sixth layer. The first layer comprises, for example, 1 to 50 mg of drug and each successive layer comprises an additional 1 to 50 mg of drug. The biodegradable polymers undergo chemical decomposition to form soluble monomers or soluble polymer units. The biodegradation of polymers usually involves chemically or enzymatically catalyzed hydrolysis. Representative of biodegradable polymers acceptable for an increase drug loading in each layer of from 5 to 50 wt % over the first and successive layers wherein the first layer comprises 100 ng. Representative biodegradable polymers comprise a member selected from the group consisting of biodegradable poly (amides), poly (amino acids), poly (esters), poly (lactic acid), poly (glycolic acid), poly (orthoesters), poly (anhydrides), biodegradable poly (dehydropyrans), and poly (dioxinones) . The polymers are known to the art in Controlled Release of Drugs, by Rosoff, Ch. 2, pp. 53- 95 (1989) ; and in U.S. Pat. Nos. 3, 811, 444; 3, 962, 414; 4, 066, 747; 4, 070, 347; 4, 079, 038; and 4, 093, 709.

In still other embodiments, the invention employs a dosage form comprising a polymer that releases a drug by diffusion, flux through pores, or by rupture of a polymer matrix. The drug delivery polymeric system comprises a concentration gradient, wherein the gradient is an ascent in concentration from a beginning or initial concentration to a final, or higher concentration. The dosage form comprises an exposed surface at the beginning dose and a distant nonexposed surface at the final dose. The nonexposed surface is coated with a pharmaceutically acceptable material impermeable to the passage of drug. The dosage form structure provides for a flux increase delivery of drug ascending from the beginning to the final delivered dose.

Figure 17 illustrates such an embodiment, where the amphetamine compound is contained within a nonabsorbable shell that releases the drug at a controlled rate.

The dosage form matrix can be made by procedures known to the polymer art. In one manufacture, 3 to 5 or more casting compositions are independently prepared wherein each casting composition comprises an increasing dose of drug with each composition overlayered from a low to the high dose. This provides a series of layers that come together to provide a unit polymer matrix with a concentration gradient. In another manufacture, the higher does is cast first followed by laminating with layers of decreasing dose to provide a polymer matrix with a drug concentration gradient. An example of providing a dosage form comprises blending a pharmaceutically acceptable carrier, like polyethylene glycol, with a known dose of a amphetamine compound and adding it to a silastic medical grade elastomer with a cross-linking agent, like stannous octanoate, followed by casting in a mold. The step is repeated for each successive layer. The system is allowed to set, for 1 hour, to provide the dosage form. Representative polymers for manufacturing the dosage form comprise a member selected from the group consisting of olefin and vinyl polymers, condensation polymers, carbohydrate polymers, and silicon polymers as represented by poly (ethylene), poly (propylene), poly (vinyl acetate), poly (methyl acrylate), poly (isobutyl methacrylate), poly (alginate), poly (amide), and poly (silicone) . The polymers and manufacturing procedures are known in Polymers, by Coleman et al., Vol. 31, pp. 1187- 1230 (1990) ; Drug Carrier Systems, by Roerdink et al., Vol. 9, pp. 57- 109 (1989) ; Adv. Drug Delivery Rev., by Leong et al., Vol. 1, pp. 199- 233 (1987) ; Handbook of Common Polymers, Compiled by Roff et al., (1971) published by CRC Press; and U.S. Pat. No. 3, 992, 518.

In still other embodiments, the subject formulations can be a mixture of different prodrug forms of one or more different amphetamine compounds, each prodrug form having a different hydrolysis rate, and therefore activation rate, to provide an increasing serum concentrationof the active amphetamine compounds.

In other embodiments, the subject formulations can be a mixture different amphetamine compounds, each compound having a different rate of adsorption (such as across the gut or epithelia) and/or serum half-life.

The dose-escalation regimen useful with the present invention can be used to compensate for the loss of a therapeutic effect of a amphetamine compound, if any, by providing a method of delivery that continually compensates for the development of acute tolerance, by considering the clinical effect (E) of a drug at time (t) as a function of the drug concentration (C) according to Equation 1:

$$\text{Effect} = f(t, C)$$

In addition, the rate of drug delivered (A), in mg per hour is inversely proportional to the concentration times the clearance of the drug. As the effect varies with time and the functionality is expressed, then according to this invention (A) can be governed to ensure the therapeutic effect is maintained at a clinical value. If the effect from a drug is found clinically to decrease with time, this decline could be linear as expressed by Equation 2:

$$\text{Effect}_{(t)} = \text{Effect}_{(ini)} - k_{effect} * t$$

wherein, $\text{Effect}_{(ini)}$ is the clinical effect observed initially at the start of drug administration and Effect (t) is the effect observed at time (t) hours, keffect is a proportionality constant ascertained by measuring the clinical effect (E1) at time (t1) hours and (E2) at time (t2) hours while maintaining a constant plasma concentration followed by dividing (E1) minus (E2) by (t1) minus (t2) . In order to maintain a constant effect, (A) must be adjusted with the same functionality according to Equation 3:

$$A_{(t)} = A_{(ini)} + k_{effect} * t$$

wherein $A_{(iii)}$ is the initial drug input in mg per hour at the start of the therapy and $A_{(t)}$ is the drug input at time (t) hours, and keffect is the proportionality constant presented above. If the therapeutic effect is found to decline exponentially with time, this relationship is expressed by Equation 4:

$$\text{Effect}_{(t)} = \text{Effect}_{(ini)} * \exp^{(-keffect*t)}$$

wherein $\text{Effect}_{(ini)}$ and $\text{Effect}_{(t)}$ are as defined before, k*effect* (or k*effect*) is a rate constant (h- 1), a unit of reciprocal hours, ascertained by measuring the clinical effect (E1) at time (t1) hours and (E2) at time (t2) hours while maintaining a constant plasma concentration followed by dividing natural log of (E1) minus natural log of (E2) by (t1) minus (t2) . To maintain a constant effect, (A) must be adjusted according to Equation 5:

$$A_{(t)} = A_{(ini)} * \exp^{(keffect*t)}$$

wherein $A_{(ii)}$ and $A_{(t)}$ are as defined before, keffect is the rate constant (h- 1) presented above. The equations are presented in Holford et al. (1982) Pharmac. Ther., 16: 143- 166.

The preparations of the present invention may be given orally, parenterally, topically, or rectally. They are of course given by forms suitable for each administration route. For example, they are administered in tablets or capsule form, by injection, infusion, inhalation, rectal suppository, or controlled release patch. Oral and controlled release patch administrations are preferred. In a particular embodiment, 1-amphetamine and/or 1-methamphetamine are orally administered to a human. The oral administration of the 1-amphetamine and/or 1-methamphetamine means that the 1-amphetamine and/or 1-methamphetamines is ingested by the human and is not a metabolite of another ingested compound such as 1-deprenyl.

In certain preferred embodiments, the subject therapeutic is delivered by way of a transdermal patch. A patch is generally a flat hollow device with a permeable membrane on one side and also some form of adhesive to maintain the patch in place on the patient's skin, with the membrane in contact with the skin so that the medication can permeate out of the patch reservoir and into and through the skin. The outer side the patch is formed of an impermeable layer of material, and the membrane side and the outer side are joined around the perimeter of the patch, forming a reservoir for the medication and carrier between the two layers.

Patch technology is based on the ability to hold an active ingredient in constant contact with the epidermis. Over substantial periods of time, drug molecules, held in such a state, will eventually find their way into the bloodstream. Thus, patch technology relies on the ability of the human body to pick up drug molecules through the skin. Transdermal drug delivery using patch technology has recently been applied for delivery of nicotine, in an effort to assist smokers in quitting, the delivery of nitroglycerine to angina sufferers, the delivery of replacement hormones in post menopausal women, etc. These conventional drug delivery systems comprise a patch with an active ingredient such as a drug incorporated therein, the patch also including an adhesive for attachment to the skin so as to place the active ingredient in close proximity to the skin. Exemplary patch technologies are available from Ciba-Geigy Corporation and Alza Corporation. Such transdermal delivery devices can be readily adapted for use with the subject amphetamine compounds.

The flux of the subject amphetamines across the skin can be modulated by changing either (a) the resistance (the diffusion coefficient), or (b) the driving force (the solubility of the drug in the stratum corneum and consequently the gradient for diffusion). Various methods can be used to increase skin permeation by the subject amphetamines, including penetration enhancers, use of pro-drug versions, superfluous vehicles, iontophoresis, phonophoresis and thermophoresis. Many enhancer compositions have been developed to change one or both of these factors. See, for example, U.S. Pat. Nos. 4,006,218; 3,551,154; and 3,472,931, for example, respectively describe the use of dimethylsulfoxide (DMSO), dimethyl formamide (DMF), and N,N-dimethylacetamide (DMA) for enhancing the absorption of topically applied drugs through the stratum corneum. Combinations of enhancers consisting of diethylene glycol monoethyl or monomethyl ether with propylene glycol monolaurate and methyl laurate are disclosed in U.S. Pat. No. 4,973,468. A dual enhancer consisting of glycerol monolaurate and ethanol for the transdermal delivery of drugs is shown in U.S. Pat. No. 4,820,720. U.S. Pat. No. 5,006,342 lists numerous enhancers for transdermal drug administration consisting of fatty acid esters or fatty alcohol ethers of C2 to C4 alkanediols, where each fatty acid/alcohol portion of the ester/ether is of about 8 to 22 carbon atoms. U.S. Pat. No. 4,863,970 shows penetration-enhancing compositions for topical application comprising an active permeant contained in a penetration-enhancing vehicle containing specified amounts of one or more cell-envelope disordering compounds such as oleic acid, oleyl alcohol, and glycerol esters of oleic acid; a C2 or C3 alkanol; and an inert diluent such as water. Other examples are included in the teachings of U.S. Pat. No. 4,933,184 which discloses the use of menthol as a penetration enhancer; U.S. Pat. No. ,229,130 discloses the use of vegetable oil (soybean and/or coconut oil) as a penetration enhancer; and U.S. Pat. No. 4,440,777 discloses the use of eucalyptol as a penetration enhancer.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms such as described below or by other conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular amphetamine compounds employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, intravenous, intracerebroventricular and subcutaneous doses of the compounds of this invention for a patient will range from about 0.0001 mg to about 100 mg per kilogram (kg) of body weight per day; about 0.0001 mg/kg to about 500 mg/kg; or 0.0001 mg/kg to about 1000 mg/kg.

If desired, the effective daily dose of the active compound may be administered as two, three, four, five, six or more sub-doses administered separately at appropriate intervals throughout the day, optionally, in unit dosage forms.

The term "treatment" is intended to encompass also prophylaxis, therapy and cure.

The patient receiving this treatment is any animal in need, including primates, in particular humans, and other mammals such as equines, cattle, swine and sheep; and poultry and pets in general.

The compound of the invention can be administered as such or in admixtures with pharmaceutically acceptable carriers and can also be administered in conjunction with other psychoactive drugs such as stimulants, antidepressants, modulators of neurotransmittors and anticonvulsants. Conjunctive therapy thus includes sequential, simultaneous and separate administration of the active compound in a way that the therapeutic effects of the first administered one is not entirely disappeared when the subsequent is administered.

While it is possible for a compound of the present invention to be administered alone, it is preferable to administer the compound as a pharmaceutical formulation (composition). The amphetamine compounds according to the invention may be formulated for administration in any convenient way for use in human or veterinary medicine.

Thus, another aspect of the present invention provides pharmaceutically acceptable compositions comprising a therapeutically effective amount of one or more of the compounds described above, formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non- aqueous solutions or suspensions), tablets, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. However, in certain embodiments the subject compounds may be simply dissolved or suspended in sterile water.

The phrase "pharmaceutically acceptable carrier" as used herein means a pharmaceutically acceptable material, composition or vehicle, such as a liquid or solid filter, diluent, excipient, solvent or encapsulating material, involved in carrying or transporting the subject regulators from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

As set out above, certain embodiments of the present amphetamine compounds may contain a basic functional group, such as amino or alkylamino, and are, thus, capable of forming pharmaceutically acceptable salts with pharmaceutically acceptable acids. The term "pharmaceutically acceptable salts" in this respect, refers to the relatively non-toxic, inorganic and organic acid addition salts of compounds of the present invention. These salts can be prepared *in*

*situ* during the final isolation and purification of the compounds of the invention, or by separately reacting a purified compound of the invention in its free base form with a suitable organic or inorganic acid, and isolating the salt thus formed. Representative salts include but are not limited to following: 2- hydroxyethanesulfonate, 2- naphthalenesulfonate, 3- hydroxy- 2- naphthoate, 3- phenylpropionate, acetate, adipate, alginate, amsonate, aspartate, benzenesulfonate, benzoate, besylate, bicarbonate, bisulfate, bitartrate, borate, butyrate, calcium edetate, camphorate, camphorsulfonate, camsylate, carbonate, citrate, clavulariate, cyclopentanepropionate, digluconate, dodecylsulfate, edetate, edisylate, estolate, esylate, ethanesulfonate, fumarate, gluceptate, glucoheptanoate, gluconate, glutamate, glycerophosphate, glycollylarsanilate, hemisulfate, heptanoate, hexafluorophosphate, hexanoate, hexylresorcinate, hydrabamine, hydrobromide, hydrochloride, hydroiodide, hydroxynaphthoate, iodide, isothionate, lactate, lactobionate, laurate, laurylsulphonate, malate, maleate, mandelate, mesylate, methanesulfonate, methylbromide, methylnitrate, methylsulfate, mucate, naphthylate, napsylate, nicotinate, nitrate, N- methylglucamine ammonium salt, oleate, oxalate, palmitate, pamoate, pantothenate, pectinate, persulfate, phosphate, phosphate/ diphosphate, picrate, pivalate, polygalacturonate, propionate, p- toluenesulfonate, salicylate, stearate, subacetate, succinate, sulfate, sulfosaliculate, suramate, tannate, tartrate, teoclate, thiocyanate, tosylate, triethiodide, undecanoate, and valerate salts, and the like. (See, for example, Berge et al. (1977) "Pharmaceutical Salts", J. Pharm. Sci. 66: 1- 19)

In certain embodiments, the pharmaceutically acceptable salts of the subject compounds include the conventional non-toxic salts of the compounds, e.g., from non-toxic organic or inorganic acids. Particularly suitable are salts of weak acids. For example, such conventional non-toxic salts include those derived from inorganic acids such as hydrochloric, hydrobromic, hydriodic, cinnamic, gluconic, sulfuric, sulfamic, phosphoric, nitric, and the like; and the salts prepared from organic acids such as acetic, propionic, succinic, glycolic, stearic, lactic, maleic, tartaric, citric, ascorbic; palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*)

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association a compound of the present invention with the carrier and, optionally, one or more accessory ingredients. In general, the formulations are prepared by uniformly and intiniately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active

ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar- agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active amphetamine compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

In certain embodiments, the subject compound(s) are formulated as part of a transdermal patch. Transdermal patches have the added advantage of providing controlled delivery of a compound of the present invention to the body. Such dosage forms can be made by dissolving or dispersing the amphetamine compounds in the proper medium. Absorption enhancers can also be used to increase the flux of the amphetamine compounds across the skin. The rate of such flux can be controlled by either providing a rate-controlling membrane or dispersing the compound in a polymer matrix or gel.

The "free base form" of amphetamine relates to a form in which amphetamine is not complexed with an acid, e.g., is not an ammonium salt. Such forms may be incorporated into a patch. It will be appreciated that the amphetamine compounds may be complexed, for example, with elements of the drug-retaining matrix of the patch and, as such, the amphetamine compounds may not necessarily be in the form of the free base, when actually retained by the patch.

The patch preferably comprises a drug-impermeable backing layer. Suitable examples of drug-impermeable backing layers which may be used for transdermal or medicated patches include films or sheets of polyolefins, polyesters, polyurethanes, polyvinyl alcohols, polyvinyl chlorides, polyvinylidene chloride, polyamides, ethylene-vinyl acetate copolymer (EVA), ethylene-ethylacrylate copolymer (EEA), vinyl acetate-vinyl chloride copolymer, cellulose acetate, ethyl cellulose, metal vapour deposited films or sheets thereof, rubber sheets or films, expanded synthetic resin sheets or films, non-woven fabrics, fabrics, knitted fabrics, paper and foils. Preferred drug-impermeable, elastic backing materials are selected from polyethylene tereplithalate (PET), polyurethane, ethylene-vinyl acetate copolymer (EVA), plasticized polyvinylchloride, woven and non-woven fabric. Especially preferred is non-woven polyethylenetereplithalate (PET). Other backings will be readily apparent to those skilled in the art.

The term 'block copolymer', in the preferred adhesives of the invention, refers to a macromolecule comprised of two or more chemically dissimilar polymer structures, tenninally connected together (Block Copolymers: Overview and Critical Survey, Noshay and McGrath, 1977) . These dissimilar polymer structures, sections or segments, represent the 'blocks' of the block copolymer. The blocks may generally be arranged in an A- B structure, an A- B- A structure, or a multi- block- $(A\text{- }B)_n$- system, wherein A and B are the chemically distinct polymer segments of the block copolymer.

It is generally preferred that the block copolymer is of an A-B-A structure, especially wherein one of A and B is an acrylic-type polymeric unit. It will be appreciated that the present invention is also applicable using block copolymers which possess three or more different blocks, such as an A-B-C block copolymer. However, for convenience, reference hereinafter to block copolymers will assume that there are only A and B sub-units, but it will be appreciated that such reference also encompasses block copolymers having more than two different sub-units, unless otherwise specified.

It will be appreciated that the properties of block copolymers are very largely determined by the nature of the A and B blocks. Block copolymers commonly possess both 'hard' and 'soft' segments. A 'hard' segment is a polymer that has a glass transition temperature (Tg) and/or a melting temperature (Tm) that is above room temperature, while a 'soft' segment is a polymer that has a Tg (and possibly a Tm) below room temperature. The different segments are thought to impart different properties to the block copolymer. Without being constrained by theory, it is thought that association of the hard segments of separate block copolymer units result in physical cross-links within the block copolymer, thereby promoting cohesive properties of the block copolymer. It is particularly preferred that the hard segments of the block copolymers form such physical close associations.

The block copolymers useful in the present invention preferably are acrylic block copolymers. In acrylic block copolymers, at least one of the blocks of the block copolymer is an acrylic acid polymer, or a polymer of an acrylic acid derivative. The polymer may be composed of just one repeated monomer species. However, it will be appreciated that a mixture of monomeric species may be used to form each of the blocks, so that a block may, in itself, be a copolymer. The use of a combination of different monomers can affect various properties of the resulting block copolymer. In particular, variation in the ratio or nature of the monomers used allows properties such as adhesion, tack and cohesion to be modulated, so that it is generally advantageous for the soft segments of the block copolymer to be composed of more than one monomer species.

It is preferred that alkyl acrylates and alkyl methacrylates are polymerized to form the soft portion of the block copolymer. Alkyl acrylates and alkyl methacrylates are thought to provide properties of tack and adhesion. Suitable alkyl acrylates and alkyl methacrylates include n-butyl acrylate, n-butyl methacrylate, hexyl acrylate, 2-ethylbutyl acrylate, isooctyl acrylate, 2-ethylhexyl acrylate, 2-ethylhexyl methacrylate, decyl acrylate, decyl methacrylate, dodecyl acrylate, dodecyl methacrylate, tridecylacrylate and tridecyl methacrylate, although other suitable acrylates and methacrylates will be readily apparent to those skilled in the art. It is preferred that the acrylic block copolymer comprises at least 50% by weight of alkyl acrylate or alkyl methacrylate(co)polymer.

Variation in the components of the soft segment affects the overall properties of the block copolymer, although the essential feature remains the cross-linking of the soft segments. For example, soft segments essentially consisting

of diacetone acrylamide with either butyl acrylate and/or 2-ethylhexyl acrylate, in approximately equal proportions, work well, and a ratio by weight of about 3 : 4 : 4 provides good results. It is preferred that diacetone acrylamide, or other polar monomer, such as hydroxyethylmethacrylate or vinyl acetate, be present in no more than 50% w/w of the monomeric mix of the soft segment, as this can lead to reduced adhesion, for example. The acrylate component may generally be varied more freely, with good results observed with both2-ethylhexyl acrylate and butyl acrylate together or individually.

As noted above, ratios of the various monomers are generally preferred to be approximately equal. For adhesives, this is preferred to be with a polar component of 50% or less of the soft segment, with the apolar portion forming up to about 85% w/w, but preferably between about 50 and 70% w/w. In the example above, this is about 72% (4+4) a polar to about 18% (3) polar.

In general, it is particularly preferred that any apolar monomer used does not confer acidity on the adhesive. Adhesives of the invention are preferably essentially neutral, and this avoids any unnecessary degeneration of the amphetamine compounds.

Limiting active functionalities, especially those with active hydrogen, is generally preferred, in order to permit wide use of any given formulation of adhesive without having to take into account how it is likely to interact, chemically, with its environment. Thus, a generally chemically inert adhesive is preferred, in the absence of requirements to the contrary.

As discussed above, polymers suitable for use as the hard portion of the block copolymer possess glass transition temperatures above room temperature. Suitable monomers for use in forming the hard segment polymer include styrene, (x- methylstyrene, methyl methacrylate and vinyl pyrrolidone, although other suitable monomers will be readily apparent to those skilled in the art. Styrene and polymethylmethacrylate have been found to be suitable for use in the formation of the hard segment of the block copolymers. It is preferred that the hard portion of the block copolymer forms from 3- 30% w/w of the total block copolymer, particularly preferably from 5- 15% w/w.

The block copolymer is further characterized in that the soft portions contain a degree of chemical cross-linking. Such cross-linking may be effected by any suitable cross-linking agent. It is particularly preferable that the cross-linking agent be in the form of a monomer suitable for incorporation into the soft segment during polymerization. Preferably the cross-linking agent has two, or more, radically polymerizable groups, such as a vinyl group, per molecule of the monomer, at least one tending to remain unchanged during the initial polymerization, thereby to permit cross-linking of the resulting block copolymer.

Suitable cross- linking agents for use in the present invention include divinylbenzene, methylene bis- acrylamide, ethylene glycol di (meth) acrylate, ethyleneglycol tetra (meth) acrylate, propylene glycol di (meth) acrylate, butylene glycoldi (meth) acrylate, or trimethylolpropane tri (meth) acrylate, although other suitable cross- linking agents will be readily apparent to those skilled in the art. A preferred cross- linking agent is tetraethylene glycol dimethacrylate. It is preferred that the cross- linking agent comprises between about 0.01 to about 0.6% by weight of the block copolymer, with between about 0.1 to about 0.4% by weight being particularly preferred.

Methods for the production of block copolymers from their monomeric constituents are well known. The block copolymer portions of the present invention may be produced by any suitable method, such as step growth, anionic, cationic and free radical methods (Block Copolymers, supra). Free radical methods are generally preferred over other methods, such as anionic polymerization, as the solvent and the monomer do not have to be purified.

Suitable initiators for polymerization include polymeric peroxides with more than one peroxide moiety per molecule. An appropriate choice of reaction conditions is well within the skill of one in the art, once a suitable initiator has been chosen.

The initiator is preferably used in an amount of about 0.005 to about 0.1% by weight of the block copolymer, with about 0.01 to about 0.05% by weight being particularly preferred, although it will be appreciated that the amount chosen is, again, well within the skill of one in the art. In particular, it is preferred that the amount should not be so much as to cause instant gelling of the mix, nor so low as to slow down polymerization and to leave excess residual monomers. A preferred level of residual monomers is below about 2000 ppm.

It will also be appreciated that the amount of initiator will vary substantially, depending on such considerations as the initiator itself and the nature of the monomers.

The block copolymers are adhesives, and preferably are pressure sensitive adhesives. Pressure sensitive adhesives can be applied to a surface by hand pressure and require no activation by heat, water or solvent. As such, they are particularly suitable for use in accordance with the present invention.

The block copolymers may be used without tackifiers and, as such, are particularly advantageous. However, it will be appreciated that the block copolymers may also be used in combination with a tackifier, to provide improved tack, should one be required or desired. Suitable tackifiers are well known and will be readily apparent to those skilled in the art.

Without being constrained by theory, it is thought that the combination of chemical cross-links between the soft segments of the copolymer combined with the, generally, hydrophobic interaction, or physical cross-linking, between the hard portions results in a "matrix-like" structure. Copolymers having only physical cross-linking of the hard segments

are less able to form such a matrix. It is believed that the combination of both forms of cross-linking of the block copolymers provides good internal strength (cohesion) and also high drug storage capacity.

More particularly, it is believed that the hard segments associate to form "islands", or nodes, with the soft segments radiating from and between these nodes.

There is a defined physical structure in the "sea" between the islands, where the soft segments are cross-linked, so that there is no necessity for extensive intermingling of the soft segments. This results in a greater cohesion of the whole block copolymer while, at the same time, allowing shortened soft segment length and still having as great, or greater, distances between the islands, thereby permitting good drug storage capacity.

The block copolymer preferably cross-links as the solvent is removed, so that cross-linking can be timed to occur after coating, this being the preferred method.

Accordingly, not only can the block copolymer easily be coated onto a surface, but the complete solution can also be stored for a period before coating. Accordingly, in the manufacturing process of the patches, the process preferably comprises polymerizing the monomeric constituents of each soft segment in solution, then adding the constituents of the hard segment to each resulting solution and polymerizing the resulting mix, followed by cross- linking by removal of any solvent or solvent system, such as by evaporation. If the solution is to be stored for any length of time, it may be necessary to keep the polymer from precipitating out, and this may be achieved by known means, such as by suspending agents or shaking. It may also be necessary to select the type of polymers that will be subject to substantially no cross-linking until the solvent is evaporated.

In general, it is preferred that the adhesive possesses a minimum number of functionalities having active hydrogen, in order to avoid undesirable reactions/interactions, such as with any drug that it is desired to incorporate into the adhesive material. It will be appreciated that this is only a preferred restriction, and that any adhesive may be tailored by one skilled in the art to suit individual requirements.

Suitable monomers for use in forming the hard segment include styrene, a-methylstyrene, methyl methacrylate and vinyl pyrrolidone, with the preferred proportion of the hard segment being between about 5 to about 15 percent (w/w). In particular, it is advantageous to use the compounds of WO 99/02141, as it is possible to load over about 30 percent of drug into such a system.

Thus, in the patches of the present invention, it is generally possible to calculate the amount of drug required and determine the appropriate patch size with a given drug loading in accordance with a patient's body weight, and this can be readily calculated by those skilled in the art.

In certain embodiments, small amounts of plasticizer, such as isopropyl myristate (IPM), are incorporated. This has the advantage of helping to solubilize the amphetamine as well as rendering the adhesive less rough on the skin. Levels of between about 2 to about 25%, by weight, are generally useful, with levels of between about 3 to about 20% being more preferred and levels of about 5 to about 15%, especially about 10%, being most preferred. Other plasticizers may also be used, and suitable plasticizers will be readily apparent to those skilled in the art.

Plasticizers generally take the form of oily substances introduced into the adhesive polymer. The effect of the introduction of such oily substances is to soften the physical structure of the adhesive whilst, at the same time, acting at the interface between the adhesive and the skin, thereby helping to somewhat weaken the adhesive, and to reduce exfoliation.

The free base oil may be obtained by basifying amphetamine salts, or any other suitable salt, with a suitable base, in the presence of a hydrophilic solvent, especially water, and an organic solvent. For instance, water and ethyl acetate, in approximately equal proportions, work well, with ammonia serving as the basifying agent. The water may then be removed and the preparation washed with further water, or other aqueous preparation, after which the preparation may be suitably extracted with ether, for example, after having removed the ethyl acetate. It is preferred to keep the preparation under an inert atmosphere, especially after completion.

Whilst it will be appreciated that patches of the present invention may be removed from the patient at any time, once it is desired to terminate a given dose, this can have the disadvantage of providing an opportunity for potential drug abuse of the partially discharged patch. Abuse of amphetamines is highly undesirable.

In certain embodiments, it may be advantage to use a patch tailored to have delivered the majority of the amphetamine that it is capable of delivering, in a 24 hour period, by about 8 hours after application, so that a patch can be left in place, and levels of drug still diminish appreciably. It is advantageous that the drug delivery profile has first order kinetics, so that the majority of the drug is delivered during the main part of the day and, even if the patient omits to remove the patch, the drug is moving towards exhaustion by the end of the day, and the amount of drug is dropping rapidly.

It will be appreciated that patches of the invention may be constructed in any suitable manner known in the art for the manufacture of transdermal patches. The patches may simply comprise adhesive, drug and backing, or may be more complex, such as having edging to prevent seepage of drug out of the sides of the patch. Patches may also be multi-layered.

Ophthalmic formulations, eye ointments, powders, solutions and the like, are also contemplated as being within

the scope of this invention.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly(anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, about 0.1 to about 99.5% (more preferably, about 0.5 to about 90%) of active ingredient in combination with a pharmaceutically acceptable carrier.

Exemplary Uses of the Compounds of the Invention.

In various embodiments, the present invention contemplates modes of treatment and prophylaxis which utilize one or more of the amphetamine compounds. These agents may be useful for increasing the occurrence of memory consolidation or decreasing or preventing the effects of defects in an animal which mitigate memory consolidation.

In various other embodiments, the present invention contemplates modes of treatment and prophylaxis which utilize one or more of the subject amphetamine compounds to alter defects in attention span and/or focus in an organism. The enhancement and/or restoration of attention span in an organism has positive behavioral, social, and psychological consequences. Additionally, enhancement of attention span can improve memory and learning.

Memory disorders which can be treated according to the present invention are associated with multiple sclerosis.

The present invention is further illustrated by the following examples.

EXEMPLIFICATION

The invention now being generally described, it will be more readily understood by reference to the following examples which are included merely for purposes of illustration of certain aspects and embodiments of the present invention, and are not intended to limit the invention.

The Inhibitory Avoidance (IA) task (also referred to herein as "Passive Avoidance") (Bammer, G., Neurosci & Biobev. Rev. 6:247-296 (1982)) and the Spontaneous Object Recognition (SOR) task (Ennaceur, A., et al., Psychopharmacol. 109:321-330 (1992); Ennaceur, A., et al., Behav. Brain Res. 33:197-207 (1989)) are well-studied behavioral paradigms which can provide the researcher with a consistent and long lasting measure of memory. The paradigms consists of one training trial and one retention trial. Test substances may be administered to the rats either before or after training. Improved memory, as a result of test substance administration, is evident on the retention trial. The objective of the following experiments was to investigate the effects of amphetamine on IA and SOR memory in the rat.

General Experimental Procedures

Inhibitory Avoidance

The Inhibitory Avoidance apparatus (Coulbourn Instruments) consisted of a light chamber and a dark chamber, which were joined by means of a sliding guillotine door. The floor of the dark compartment consisted of 2.4 mm diameter steel rods, through which a foot-shock could be administered to the animal by a constant current 18-pole shock scrambler. The test apparatus was enclosed in a ventilated, sound-attenuating cabinet, and was controlled by Graphic State™ Notation Software (Version 1.013) and a Hewlett Packard Pavilion Computer. Training involved the rat being placed in the light chamber for a ten second period, after which time the sliding door was opened, allowing the rat access to the dark chamber. Two seconds after entering the dark chamber, a continuous 0.46 mA foot-shock was delivered through the floor grid for two seconds. The animal was then removed from the apparatus and returned to the home cage. The animals received a retention test 24 hours following training. The retention test was identical to training except that no foot-shock was delivered. Latency to enter the dark chamber was recorded, and the animals were then returned to their home cages. Data was collected by the Graphic State™ Notation software, and was recorded onto an appropriate data sheet.

Spontaneous Object Recognition

Apparatus for Object Recognition testing consisted of a plexiglass open field activity chamber, measuring 30 by 30 cm. A video camera was mounted on the wall above the chamber. Three plastic objects served as stimuli for the experiment. Two of the objects were identical to one another, and the third was different. Rats were individually habituated to the open-field box for three consecutive days. Habituation sessions were six minutes in duration. Twenty-four hours after the last day of habituation, a training session was conducted, in which two identical objects were placed in the open-field box, 10 cm from the back wall. The animal was placed into the box and was allowed to explore freely for a period of four minutes. Twenty-four hours after the training session, retention testing was conducted. During retention testing, the rat was placed back into the same activity box with one of the familiar objects used during the training session and a novel object that the rat had not seen before. The rat was allowed to explore the box and objects for a period of four minutes. Testing was conducted at the same time each day, and was videotaped for off-line analysis. Two discrimination indices, D1 and D2 were calculated in order to measure the strength of recognition memory. D1 reflects the amount of time spent exploring the novel object minus the amount of time spent exploring the familiar object, and D2 reflects D1 divided by total exploration time.

Activity Monitoring

Activity monitoring was conducted in a Plexiblas open-field box. Activity levels were measured by a grid of infrared light beams that traversed the cage from left to right and back to front. The location of the animal was detected by breaks in the infrared light beams. General behavior and activity levels were recorded by a computerized monitoring system for a period of ten minutes. The analyzed behaviors included but were not limited to; horizontal activity, total distance moved, movement time, number of movements, number of rears, number of stereotyped movements, and time spent resting. Data was collected on-line using Versa Max (Version 1.83) computer software and a Hewlett Packard Pavilion computer.

Tail Flick

For Tail-Flick Analgesia Testing, the animal was placed on top of the Tail-Flick monitor and gently held in place with a cotton towel. The tail of the animal was placed in a shallow groove lying between two sensors and over the top of a radiant heat wire. The Tail Flick monitor was activated, and the latency for the animal to flick its tail out of the groove and away from the heat source was recorded. The animal was returned to its home cage immediately following testing.

Fornix Lesions

Rats were anesthetized with Nembutal (55 mg/kg) and prepared for surgery. The rat was placed in the stereotaxic apparatus, a midline incision made, and the scalp retracted to expose the skull. The skull was cleaned and dried using sterile saline and cotton swabs, and four stereotaxically determined holes were drilled in the skull at the following coordinates: 0.3 and 0.8 mm posterior to Bregma, and 0.5 and 0.7 mm lateral to the midline. An electrode (Teflon-coated wire, 125 μm in diameter) was lowered into the brain to a depth of 4.6 mm, and DC current at 1.0 mA was passed through the electrodes for a duration of 10 seconds. The electrodes were then removed, and the wound was sutured. Animals

were removed from the stereotaxic apparatus and received postoperative care and monitoring until fully conscious. The rats were left to recover for a period of seven days prior to behavioral testing. The health status of the animals was checked on a daily basis during the recovery period.

EXAMPLE 1: DOSE RESPONSE TESTING

Effects of (S)-(+)-amphetamine on Inhibitory Avoidance

In this experiment, rats were injected with three different doses of (S)- (+) amphetamine thirty minutes prior to being trained on the IA task. As can be seen from Figure 1, a dose of about 2 mg/kg of amphetamine improved retention of the task, while doses of about 0.25, about 0.50 and about 1.0 mg/kg had no effect. In order to verify this result, a second experiment was conducted. Rats were injected with about 2.0 mg/kg of amphetamine and trained on the IA task. As can be seen from Figure 2, this dose of (S)- (+)- amphetamine significantly improved retention of the task. An unpaired t- test demonstrated that this enhancement was statistically significant ($p < 0.01$) .

Effects of (R)-(-)-amphetamine (C105) on Inhibitory Avoidance

The first experiment to be conducted using C105 was a dose response experiment, in which different doses of C105 (about 0.4, about 0.5, about 0.75, 1.0 and about 2.0 mg/kg) were administered to the rats one hour prior to training on the Inhibitory Avoidance task. Retention for the task was tested 24-hours later. A one way ANOVA was conducted on the data, and the results revealed a statistically significant difference between the dose level groups ($F(5,59) = 3.368$, $p < 0.01$). Subsequent post hoc analysis (Student Newman Keuls) demonstrated that the 1.0 mg/kg group performed significantly better than saline injected controls ($p < 0.05$). The 0.5 mg/kg dose also appeared to be effective in enhancing the animals performance, however, this trend did not reach statistical significance. This experiment was subsequently replicated using 0.5 mg/kg as the target dose in order to verify this result (see section 9.1.4). Dose Response data is presented individually in Table 3.

Effects of (R)-(-)-amphetamine (C105) on Inhibitory Avoidance

In this experiment, four groups of 10 rats were injected with different doses (0.5 mg/kg, 1.0 mg/kg, 2.0 mg/kg or 4.0 mg/kg) of the R- (- ) enantiomer of amphetamine one hour prior to being trained on the IA task. The experiments were conducted with a 24 hour retention interval and a 0.46 mA shock intensity. As can be seen in Figure 8, a much lower dose of (R)- (- )- amphetamine is required for the same improved retention effect as obtained with (S)- (+)-amphetamine (compare to Figure 1) . Increasing the dose above 0.5 kg/mg did not further improve the retention results obtained with this dose possibly indicating a saturation effect.

Effects of (R)-(-)-amphetamine (C105) on Inhibitory Avoidance

In order to investigate whether doses of C105 lower than 0.5 mg/kg enhanced performance, rats were injected with 0.1, 0.25 or 0.5 mg/kg of C105 one hour prior to training. Retention was tested 24-hours later. This experiment revealed that doses of C105 lower than 0.5 mg/kg were not effective in improving the mnemonic performance of the rats. In contrast, the 0.5 mg/kg dose significantly enhanced performance on the task ($F(3,39) = 67450$, $p < 0.0477$). These data are presented individually in Table 4.

EXAMPLE 2: TIME COURSE OF EFFECTIVENESS

In this experiment, the time of drug administration was varied in order to determine the optimal pre-training drug administration time. Figure 3 shows that (S)-(+) amphetamine (2.0 mg/kg) is effective when administered to the rats between 0 and 2 hours prior to training.

EXAMPLE 3: LONG TERM RETENTION

This experiment was conducted in order to determine whether the enhanced retention observed in Experiment 2 was long- lasting. Rats received a second retention test one week after the first retention test. No additional training or drug was administered to the animals in the interim period. Figure 4 illustrates that rats that had received (S)- (+)-amphetamine the previous week performed significantly better than rats that had received control injections of vehicle solution ($F(4, 47) = 3.688$, $p < 0.01$) .

EXAMPLE 4: EFFECTS ON LESIONED ANIMALS

Effects of (-)(+)-amphetamine on Lesioned Animals

The findings of the above experiments are important, as they identify the most effective dose and time of administration for this compound. Moreover, the results demonstrate that (S)- (+)- amphetamine improves memory in normal rats, and that this improvement is long- lasting. In the next experiment, we investigated whether the performance of amnesic rats could be improved by administration of d- amphetamine. In this experiment, control rats and rats with lesions of the fornix received injections of either saline or d- amphetamine (2.0 mg/kg), and one hour later, were tested on the IA task.

As Figure 5 illustrates, (S)- (+)- amphetamine dramatically enhanced the performance of normal rats and in addition, appeared to improve the performance of the fornix lesion rats. A one way ANOVA demonstrated that there was a significant difference between the performance of the four groups ($F(3, 36) = 8.687$, $p < 0.002$) . Student- Newman-Keuls post hoc tests revealed firstly that the performance of normal rats that received (S)- (+)- amphetamine was significantly enhanced relative to all other experimental groups ($p < 0.05$) . In addition, the performance of fornix animals that received (S)- (+)- amphetamine was not significantly different from normal, saline injected animals. These results demonstrate that amphetamine is capable of enhancing memory in normal rats and has beneficial effects in brain damaged, amnesic rats.

Effects of (R)-(-)-amphetamine on Lesioned Animals

Rats with bilateral lesions of the fornix were tested on the Inhibitory Avoidance task. All rats were injected with test (0.5, 1.0, 2.0 and 4.0 mg/kg) or control article one hour prior to testing. A one-way ANOVA demonstrated that there was a significant main effect of dose ($F(4,45) = 15580$, $p < 0.0316$). A dose of 1.0 mg/kg of C105 appeared to be most effective in improving the performance of the fornix lesion animals. Data from this experiment are illustrated in Figure 12 and presented individually in Table 7.

Rats with lesions of the fornix were also tested on the Object Recognition task. Rats received I.P injections of C105 (1.0 mg/kg) or saline immediately after the training session, and were tested for retention 24-hours later. As can be seen from Figure 13, when compared with controls, lesions of the fornix had a detrimental effect on performance of this task. Administration of C105 produced a trend towards improving discrimination performance in D1 ($p = 0.0685$), and slightly improved performance in D2.

EXAMPLE 5: EFFECTS OF (R)-(-) VS. (S)-(+) AMPHETAMINE ENANTIOMERS ON STIMULATION OF MEMORY CONSOLIDATION

The effects of the (R)- (- )- amphetamine and the (S)- (+) amphetamine enantiomers on stimulation of memory consolidation and motor stimulation were compared. The (R)- (- ) enantiomer of amphetamine is referred to as C105 in the figures.

Effects of (S)-(+)-amphetamine on Inhibitory Avoidance

An experiment was conducted in which different doses of (S)- (+)- amphetamine were administered to rats one hour before training on the Inhibitory Avoidance task and were compared to a control group of rats injected with saline. Retention for the task was tested 24 hours later with a 0.46 mA shock intensity. Results for this experiment are presented individually in Tables 1 and 2, and demonstrated that (S)- (+)- amphetamine appeared to enhance performance when administered at a dose of 2.0 mg/kg. The experiment was subsequently replicated several times using a test- article dose of 2.0 mg/kg. Results from these experiments are represented in Figure 6, and demonstrate that (S)- (+)- amphetamine significantly enhanced memory for the Inhibitory Avoidance task ($t(76) = 3.416$, $p < 0.001$) . These results are in agreement with previous research and help to demonstrate the effectiveness of (S)- (+)- amphetamine as a memory-enhancing drug.

Effects of (R)-(-)-amphetamine (C 105) on Inhibitory Avoidance

In order to verify the results from the dose response test, a second experiment with (R)- (- )- amphetamine was conducted. Eighteen rats were injected with a dose of 0.5 mg/kg of (R)- (- )- amphetamine one hour prior to being trained on the IA task. The (R)- (- ) amphetamine treated rats were compared to control rats injected with saline. The experiments were conducted with a 24 hour retention interval and a 0.46 mA shock intensity. As can be seen in Figure 9, this dose of (R)- (- )- amphetamine significantly improved retention of the task. An unpaired t- test demonstrated that this enhancement was statistically significant (p<0.002) .

Based on the results obtained from the experiments described above, several more experiments were conducted investigating the effects of a 0.5 mg/kg dose of C105 on Inhibitory Avoidance. The data presented in Figure 10, and individually in Table 5, represent a summary of all such experiments. The results of these experiments clearly demonstrate a memory enhancing effect as measured by the Inhibitory Avoidance task. Rats that had been injected with C105 (0.5 mg/kg) one hour prior to training performed significantly better than control animals on the 24-hour retention test ($t$ (132) = 3.438, $p$ < 0.0008).

Effects of (R)-(-)-amphetamine (C105) on Object Recognition

In order to investigate the effects of C105 on recognition memory, rats were trained on the Spontaneous Object Recognition task. Normal rats were injected with 0.5 mg/kg C105 immediately following the training session, and were tested for retention 24-hours later. The results of the experiment indicate that C105 significantly improved recognition memory. Rats that had received injections of test article immediately after the training session, performed significantly better than their saline injected counterparts, as they spent more time exploring the novel object during retention testing. Both discrimination indices, D 1 and D2, which reflect discrimination between the familiar and novel object, were significantly higher in C105 treated animals [($t$(51) = 2.526, $p$ < 0.0147) and ($t$(51) = 3.197, $p$< 0.0024) respectively]. These results are particularly interesting, as recognition memory is the process by which a subject is aware that a stimulus has previously been experienced. This process requires that incoming stimuli be identified and compared with representations of previously encountered stimuli stored in memory. Recognition memory is used during everyday life and failures of recognition memory undoubtedly contribute to the problems encountered by amnesic patients. Results from this experiment are presented in Figure 13, and individual data are presented in Table 8.

It is interesting at this point to compare the results obtained with (R)- (- )- amphetamine (C105) to those obtained with (S)- (+)- amphetamine. (S)- (+)- Amphetamine had a memory enhancing effect at a dose of 2.0 mg/kg, while (R)- (- )- amphetamine had a memory enhancing effect on the same task at a dose of 0.5 mg/kg. Although definitive dose-response relationship experiments between these two compounds have not been conducted, it seems likely that C105 is a more potent memory enhancer for this particular task in rats. It should be noted however, that the maximal efficacy of the two compounds are the same.

EXAMPLE 6: EFFECTS OF (R)-(-) VS. (S)-(+)-AMPHETAMINE ON MOTOR STIMULATION

Effects of (S)-(+)-amphetamine on Activity Levels

In order to provide a comparison point for the results described above, a second experiment was conducted in which rats were injected with 2 mg/kg of (S)- (+)- amphetamine prior to activity testing. Results for this experiment are presented in Figure 7. The results demonstrated that (S)- (+)- amphetamine produced a clear and significant enhancement in locomotor activity for the entire 10 minute session. Significant main effects for the variables of total distance (F (9, 70) = 1514000, $p$ < 0.0001) ; number of movements (F (9, 70) = 45.89, $p$ < 0.0001) ; movement time (F (9, 70) = 53.07, $p$< 0.0001) ; rears (F (9, 70) = 49.47, $p$< 0.0001), stereotyped movements (F (9, 70) = 24.65, $p$< 0.0001) and rest time (F (9, 70) = 44.34, $p$< 0.0001) were observed. No significant effects of time or time- drug interactions were observed.

Effects of (R)-(-)-amphetamine (C105) on Activity Levels

In this experiment, rats were injected with 0.5 mg/kg of (R)- (- )- amphetamine (C105) and compared to a control group of rats injected with saline. Rat activity was monitored for a 10 minute period one hour after (R)- (- )- amphetamine injection. As can be seen in Figure 14, treatment with (R)- (- )- amphetamine had no significant effects on the activity levels of the rats as compared to the control group.

This data indicates that (R)- (- )- amphetamine can provide improved memory consolidation without producing the motor stimulatory effects observed in the (S)- (+)- amphetamine treated rats (compare to Figure 7) . A comparison of the results obtained for (S)- (+)- versus (R)- (- )- amphetamine indicates that (S)- (+)- amphetamine produced a larger locomotor effect than (R)- (- )- amphetamine, at doses that are equally effective in enhancing memory. This observation is consistent with previous research, which has repeatedly demonstrated that (S)- (+)- amphetamine is between 4 and 10 times more potent than (R)- (- )- amphetamine in producing elevated locomotor responses.

EXAMPLE 7: TAIL FLICK

Tail Flick Analgesia data is presented in Figure 16, and individual data in Table 12. Administration of 1.0, 2.0, 4.0 or 8.0 mg/kg of C105 one hour prior to testing resulted in varying degrees of analgesia. 1.0 and 2.0 mg/kg had no

analgesic properties, while 4.0 and 8.0 did. Statistical significance was observed at the 4.0 mg/kg dose ($F (4, 39) = 43.18$, $p < 0.0117$) . This experiment indicates that the therapeutic dose of (R)- (- )- amphetamine had no effect on analgesia, as measured by the tail- flick analgesiometer.

EXAMPLE 8: POST TRAINING ADMINISTRATION

While the results described above provide evidence to suggest that C105 enhances memory, it is possible that these results are due to non-mnemonic factors. Because the drug was administered prior to training, it is possible that learning or acquisitional processes were affected by drug administration. For this reason, a post training experiment was conducted in which C105 was administered to the rats immediately after the training session. Injecting the drug after the training session affects memory consolidation rather than acquisition, primarily because the drug is not on board at the time of training. The results of this experiment are represented in Figure 11 and presented individually in Table 6. As can be seen from Figure 11, post training administration of 0.5 mg/kg of C 105 significantly enhanced performance on the Inhibitory Avoidance task ($t(26) = 2.160$, $p< 0.0402$). This experiment therefore, provides strong evidence that C105 works by selectively enhancing memory consolidation.

*Table 1: Effects of Different Doses of S-(+)-Amphetamine on Inhibitory Avoidance*

| **Saline** | **0.25 mg/kg** | **0.5 mg/kg** | **1.0 mg/kg** | **2.0 mg/kg** |
|---|---|---|---|---|
| 22.0 | 6.0 | 2.0 | 7.0 | 33.0 |
| 25.0 | 19.0 | 26.0 | 17.0 | 63.0 |
| 26.0 | 29.0 | 38.0 | 19.0 | 82.0 |
| 33.0 | 29.0 | 39.0 | 25.0 | 84.0 |
| 41.0 | 44.0 | 63.0 | 31.0 | 101.0 |
| 71.0 | 59.0 | 65.0 | 34.0 | 190.0 |
| 121.0 | 94.0 | 110.0 | 35.0 | 230.0 |
| 216.0 | 124.0 | 153.0 | 47.0 | 245.0 |
| 234.0 | 310.0 | 207.0 | 157.0 | 457.0 |
| 358.0 | 452.0 | 207.0 | 263.0 | 517.0 |

Data are expressed as latency to enter the dark side of the apparatus in seconds for each animal (10 animals per treatment group). Data is rank-ordered.

Table 2: Summary of Effects of S-(+)-Amphetamine (2.0 mg/kg) on Inhibitory Avoidance

| **Saline** | ***S-(+)-Amphetamine*** |
|---|---|
| 3.0 | 5.0 |
| 6.0 | 15.0 |
| 11.0 | 26.0 |
| 17.0 | 32.0 |
| 19.0 | 33.0 |
| 22.0 | 60.0 |
| 25.0 | 63.0 |
| 26.0 | 82.0 |
| 30.0 | 84.0 |
| 33.0 | 100.0 |
| 33.0 | 101.0 |
| 33.0 | 127.0 |

(continued)

| **Saline** | ***S-(+)-Amphetamine*** |
|---|---|
| 36.0 | 148.0 |
| 40.0 | 167.0 |
| 41.0 | 169.0 |
| 42.0 | 188.0 |
| 44.0 | 190.0 |
| 53.0 | 201.0 |
| 53.0 | 204.0 |
| 57.0 | 222.0 |
| 63.0 | 230.0 |
| 71.0 | 237.0 |
| 80.0 | 245.0 |
| 105.0 | 248.0 |
| 110.0 | 289.0 |
| 121.0 | 296.0 |
| 148.0 | 300.0 |
| 204.0 | 300.0 |
| 214.0 | 364.0 |
| 216.0 | 365.0 |
| 234.0 | 371.0 |
| 242.0 | 457.0 |
| 262.0 | 461.0 |
| 266.0 | 517.0 |
| 286.0 | 557.0 |
| 297.0 | 636.0 |
| 349.0 | 736.0 |
| 358.0 | 820.0 |
| 673.0 | 900.0 |

Data are expressed as latency to enter the dark side of the apparatus in seconds for each animal (39 animals per treatment group). Data is rank-ordered.

Table 3: Effects of Different Doses of C105 on Inhibitory Avoidance

| **Saline** | **0.4 mg/kg** | **0.5 mg/kg** | **0.75 mg/kg** | **1.0 mg/kg** | **2.0 mg/kg** |
|---|---|---|---|---|---|
| 17.0 | 45.0 | 16.0 | 9.0 | 101.0 | 30.0 |
| 55.0 | 62.0 | 38.0 | 15.0 | 115.0 | 59.0 |
| 60.0 | 80.0 | 137.0 | 16.0 | 121.0 | 59.0 |
| 77.0 | 87.0 | 203.0 | 21.0 | 202.0 | 127.0 |
| 103.0 | 170.0 | 267.0 | 150.0 | 265.0 | 137.0 |
| 107.0 | 231.0 | 332.0 | 157.0 | 343.0 | 230.0 |

(continued)

| Saline | 0.4 mg/kg | 0.5 mg/kg | 0.75 mg/kg | 1.0 mg/kg | 2.0 mg/kg |
|---|---|---|---|---|---|
| 116.0 | 236.0 | 556.0 | 229.0 | 729.0 | 231.0 |
| 129.0 | 250.0 | 698.0 | 237.0 | 813.0 | 253.0 |
| 240.0 | 265.0 | 741.0 | 288.0 | 824.0 | 366.0 |
| 280.0 | 629.0 | 900.0 | 650.0 | 900.0 | 384.0 |

Data are expressed as latency to enter the dark side of the apparatus in seconds for each animal (10 animals per treatment group). Data is rank-ordered.

*Table 4: Effects of Low Doses of C105 on Inhibitory Avoidance*

| Saline | 0.1 mg/kg | 0.25 mg/kg | 0.5 mg/kg |
|---|---|---|---|
| 33.0 | 37.0 | 24.0 | 127.0 |
| 38.0 | 37.0 | 28.0 | 137.0 |
| 55.0 | 39.0 | 29.0 | 144.0 |
| 62.0 | 39.0 | 71.0 | 164.0 |
| 80.0 | 55.0 | 71.0 | 167.0 |
| 100.0 | 55.0 | 100.0 | 182.0 |
| 216.0 | 110.0 | 113.0 | 219.0 |
| 235.0 | 113.0 | 117.0 | 265.0 |
| 370.0 | 124.0 | 120.0 | 362.0 |
| 518.0 | 366.0 | 205.0 | 886.0 |

Data are expressed as latency to enter the dark side of the apparatus in seconds for each animal (10 animals per treatment group). Data is rank-ordered.

*Table 5: Summary of the Effects of C105 (0.5 mg/kg) or Saline on Inhibitory Avoidance*

| Saline | C105 |
|---|---|
| 21.0 | 21.0 |
| 21.0 | 33.0 |
| 24.0 | 40.0 |
| 26.0 | 41.0 |
| 27.0 | 43.0 |
| 27.0 | 63.0 |
| 33.0 | 65.0 |
| 38.0 | 65.0 |
| 39.0 | 66.0 |
| 39.0 | 79.0 |
| 55.0 | 126.0 |
| 59.0 | 127.0 |
| 59.0 | 137.0 |
| 62.0 | 154.0 |

(continued)

| Saline | C105 |
|---|---|
| 75.0 | 164.0 |
| 79.0 | 167.0 |
| 80.0 | 181.0 |
| 96.0 | 182.0 |
| 100.0 | 188.0 |
| 109.0 | 188.0 |
| 109.0 | 225.0 |
| 113.0 | 225.0 |
| 113.0 | 261.0 |
| 121.0 | 265.0 |
| 121.0 | 357.0 |
| 168.0 | 362.0 |
| 179.0 | 418.0 |
| 179.0 | 444.0 |
| 193.0 | 521.0 |
| 216.0 | 540.0 |
| 235.0 | 556.0 |
| 235.0 | 595.0 |
| 248.0 | 660.0 |
| 370.0 | 880.0 |
| 431.0 | 886.0 |
| 431.0 | 900.0 |
| 518.0 | 900.0 |
| 17.0 | 16.0 |
| 23.0 | 37.0 |
| 27.0 | 38.0 |
| 33.0 | 52.0 |
| 36.0 | 137.0 |
| 40.0 | 170.0 |
| 41.0 | 184.0 |
| 46.0 | 203.0 |
| 47.0 | 209.0 |
| 48.0 | 231.0 |
| 55.0 | 267.0 |
| 55.0 | 273.0 |
| 56.0 | 293.0 |
| 60.0 | 332.0 |
| 74.0 | 426.0 |

(continued)

| Saline | C105 |
|---|---|
| 77.0 | 556.0 |
| 82.0 | 582.0 |
| 92.0 | 698.0 |
| 103.0 | 741.0 |
| 105.0 | 900.0 |
| 107.0 | |
| 108.0 | |
| 114.0 | |
| 116.0 | |
| 120.0 | |
| 120.0 | |
| 129.0 | |
| 154.0 | |
| 176.0 | |
| 204.0 | |
| 218.0 | |
| 225.0 | |
| 240.0 | |
| 281.0 | |
| 334.0 | |
| 518.0 | |
| 680.0 | |
| 900.0 | |
| 900.0 | |
| 900.0 | |

Data are expressed as latency to enter the dark side of the apparatus in seconds for each animal. This table reflects data gathered from all experiments conducted using C105. The numbers of animals in the saline (n = 77) and drug conditions (n = 57) differ because in several experiments, extra control animals were run. Data is rank-ordered.

*Table 6: Effects of Post-Training Administration of C105 on Inhibitory Avoidance*

| Saline | C105 |
|---|---|
| 17.0 | 38.0 |
| 25.0 | 65.0 |
| 26.0 | 77.0 |
| 34.0 | 112.0 |
| 36.0 | 123.0 |
| 37.0 | 133.0 |
| 41.0 | 170.0 |

(continued)

| Saline | C105 |
|---|---|
| 64.0 | 185.0 |
| 64.0 | 194.0 |
| 120.0 | 223.0 |
| 137.0 | 276.0 |
| 175.0 | 338.0 |
| 271.0 | 603.0 |
| 349.0 | 824.0 |

Data are expressed as latency to enter the dark side of the apparatus in seconds for each animal (14 animals per treatment group). Data is rank-ordered.

*Table 7: Effects of C105 on Inhibitory Avoidance in Control and Fornix Lesion Rats*

| Control Saline | Fornix Saline | Control 0.5 mg/kg | Fornix 0.5 mg/kg | Control 1.0 mg/kg | Fornix 1.0 mg/kg | Control 2.0 mg/kg | Fornix 2.0 mg/kg | Control 4.0 mg/kg | Fornix 4.0 mg/kg |
|---|---|---|---|---|---|---|---|---|---|
| 50.0 | * | 28.0 | 4.0 | 26.0 | ** | 21.0 | 4.0 | 21.0 | 2.0 |
| 72.0 | ** | 28.0 | 7.0 | 26.0 | 4.0 | 25.0 | 17.0 | 36.0 | 8.0 |
| 92.0 | 2.0 | 93.0 | 13.0 | 48.0 | 7.0 | 41.0 | 18.0 | 64.0 | 13.0 |
| 123.0 | 19.0 | 162.0 | 21.0 | 56.0 | 19.0 | 84.0 | 26.0 | 64.0 | 21.0 |
| 126.0 | 22.0 | 214.0 | 21.0 | 106.0 | 19.0 | 86.0 | 28.0 | 83.0 | 25.0 |
| 164.0 | 23.0 | 240.0 | 32.0 | 195.0 | 166.0 | 92.0 | 30.0 | 83.0 | 28.0 |
| 180.0 | 35.0 | 252.0 | 55.0 | 197.0 | 221.0 | 106.0 | 40.0 | 86.0 | 42.0 |
| 217.0 | 40.0 | 271.0 | 65.0 | 213.0 | 246.0 | 160.0 | 70.0 | 98.0 | 96.0 |
| 222.0 | 72.0 | 284.0 | 72.0 | 238.0 | 274.0 | 192.0 | 84.0 | 193.0 | 140.0 |
| 228.0 | 141.0 | 577.0 | 209.0 | 317.0 | 314.0 | 581.0 | 153.0 | 208.0 | 159.0 |

Data are expressed as latency to enter the dark side of the apparatus in seconds for each animal (10 animals per treatment group). Data is rank-ordered.
* animal died during surgery - no data available
** data from these subjects excluded from analysis as they were outliers: more than two SD's away from the mean

Table 8: *Effects of C105 on Spontaneous Object Recognition: Control Data, Discrimination Index D1*

| Saline | C105 |
|---|---|
| -1.00 | * |
| -1.00 | 1.00 |
| 0.87 | 5.00 |
| 1.59 | 5.00 |
| 2.00 | 6.00 |
| 2.00 | 6.47 |
| 3.00 | 7.00 |
| 3.00 | 8.00 |

(continued)

| Saline | C105 |
|---|---|
| 3.82 | 8.00 |
| 3.92 | 9.00 |
| 4.00 | 9.00 |
| 4.00 | 9.76 |
| 4.84 | 12.00 |
| 4.97 | 12.06 |
| 5.00 | 12.31 |
| 6.00 | 13.00 |
| 7.00 | 13.00 |
| 7.00 | 13.29 |
| 7.60 | 14.03 |
| 8.00 | 16.00 |
| 9.00 | 16.00 |
| 10.00 | 18.59 |
| 11.00 | 20.00 |
| 18.00 | 20.00 |
| 23.00 | 20.00 |
| 23.00 | 22.00 |
| 32.00 | 26.00 |
| * data for one subject in the C105 excluded as it was an outlier - more than two SD's away from the mean. Data is rank-ordered. | |

Table 9: *Effects of C105 on Spontaneous Object Recognition: Fornix Data, Discrimination Index D1*

| Fornix + Saline | Fornix + C105 |
|---|---|
| * | -4.79 |
| ** | -0.12 |
| ** | 0.00 |
| -2.60 | 1.92 |
| -0.60 | 3.00 |
| -0.30 | 3.26 |
| 0.00 | 5.00 |
| 0.00 | 6.00 |
| 1.00 | 6.00 |
| 1.00 | 6.00 |
| 2.90 | 8.00 |
| 3.00 | 9.00 |
| 3.00 | 12.30 |
| 4.00 | 14.80 |

(continued)

| Fornix + Saline | Fornix + C105 |
|---|---|
| 7.00 | 16.46 |
| 10.00 | 18.00 |
| 17.00 | 19.29 |
| * animal died during surgery: no data collected<br>** data for these two animals not videotaped | |

Data is rank-ordered.

Table 10: *Effects of C105 on Spontaneous Object Recognition: Control Data, Discrimination Index D2*

| Saline | C105 |
|---|---|
| -5.88 | * |
| -3.03 | 4.00 |
| 6.48 | 10.20 |
| 8.69 | 24.32 |
| 8.77 | 25.00 |
| 10.00 | 25.30 |
| 14.29 | 27.14 |
| 17.95 | 30.36 |
| 20.67 | 31.33 |
| 22.09 | 33.52 |
| 22.48 | 36.36 |
| 22.58 | 37.14 |
| 23.08 | 39.37 |
| 23.18 | 41.18 |
| 25.00 | 42.11 |
| 27.27 | 47.06 |
| 30.77 | 47.83 |
| 31.43 | 48.15 |
| 33.33 | 52.94 |
| 35.58 | 55.56 |
| 45.45 | 55.56 |
| 50.00 | 55.61 |
| 51.11 | 57.50 |
| 52.84 | 63.29 |
| 53.49 | 76.47 |
| 56.25 | 83.33 |
| 66.67 | 100.00 |
| * data excluded because it was more than 2 SD's away from the mean | |

Data is rank-ordered.

Table 11: *Effects of C105 on Spontaneous Object Recognition: Fornix Data, Discrimination Index D2*

| Fornix + Saline | Fornix + C105 |
|---|---|
| * | -26.45 |
| ** | -0.75 |
| ** | 0.00 |
| -14.29 | 9.50 |
| -8.78 | 15.55 |
| -5.23 | 17.64 |
| 0.00 | 22.22 |
| 0.00 | 27.09 |
| 2.84 | 29.41 |
| 7.69 | 30.00 |
| 9.09 | 31.25 |
| 10.59 | 36.00 |
| 20.00 | 37.50 |
| 30.28 | 40.91 |
| 33.33 | 42.86 |
| 48.57 | 55.45 |
| 50.00 | 62.34 |
| * animal died during surgery: no data collected<br>** data for these two animals not videotaped | |

Data is rank-ordered.

Table 12: Effects of C105 on Tail-Flick Analgesia

| Saline | 1.0 mg/kg | 2.0 mg/kg | 4.0 mg/kg | 8.0 mg/kg |
|---|---|---|---|---|
| 1.55 | 1.13 | 2.46 | 2.22 | * |
| 2.19 | 2.28 | 2.68 | 3.13 | 3.13 |
| 2.37 | 3.26 | 2.79 | 5.43 | 4.67 |
| 2.71 | 3.30 | 3.05 | 6.26 | 4.72 |
| 3.44 | 4.59 | 3.56 | 6.42 | 5.22 |
| 3.58 | 4.60 | 3.89 | 6.66 | 6.54 |
| 3.64 | 5.09 | 3.96 | 16.44 | 7.39 |
| 5.34 | 6.01 | 4.45 | 20.00 | 14.43 |
| * no data for this subject was collected | | | | |

Data is rank-ordered.

EXAMPLE 9: COMPARISON OF D-AMPHETAMINE, L-AMPHETAMINE AND L-METHAMPHETAMINE

Materials and Methods

Animals

Male, Long- Evans rats (3- 5 months of age) obtained from Charles River Laboratories weighing between 250 and 350 grams at the time of arrival served as subjects in these experiments. The rats were housed two to a cage in plastic cages with corncob bedding. The rats were maintained on a 12/12 light dark cycle with *ad libitum* access to food and water.

Drugs

L- methamphetamine (SN522), 1- amphetamine (C105) and d- amphetamine were dissolved in saline and administered to the rats via intraperitoneal (i.p.) injections, in a volume of 1 ml/kg body weight.

Experiment 1: Passive Avoidance Test

The Passive Avoidance apparatus (Coulbourn Instruments) consisted of a light chamber and a dark chamber, which were joined by means of a sliding guillotine door. The floor of the dark compartment consisted of 2.4 mm diameter steel rods, through which a foot-shock could be administered to the animal by a constant current 18-pole shock scrambler. The test apparatus was enclosed in a ventilated, sound-attenuating cabinet, and was controlled by Graphic State ™ Software (Version 1.013) and a Hewlett Packard Pavilion Computer.

Training involved placing the rat inside the light chamber with its head facing away from the door. Ten seconds later, the sliding door was opened, and the latency to enter the dark chamber was recorded (100 second maximum). When the rat entered the dark chamber, it received a continuous foot-shock (0.4 mA) through the metal grid floor until it returned in the light chamber for a period of 100 consecutive seconds or until a maximum of 5 foot-shocks had been received.

Retention testing was conducted 24 hours later. The rat was placed into the light chamber with its head facing away from the door. Ten seconds later, the door was opened, alloweing the rat access to the dark chamber. No foot-shock was administered during retention testing. Latency to enter the dark chamber was recorded (900 seconds maximum) and used as a measure of memory.

In this experiment, rats were injected (ip) immediately after training with saline (control/ vehicle), SN522 (0.25 and 0.5 mg/kg), C105 (0, 5 and 1.0 mg/kg) or *d*- amphetamine (1.0 and 2.0 mg/kg) . Retention was tested 24 hours after training.

The results from this experiment are illustrated in Figure 20 and demonstrate that the different doses of the three drugs differ in terms of their potency. Figure 20 depicts a comparison of d-amphetamine, C105 and SN522 administered immediately after training in inhibitory avoidance. Data show the mean (±SEM) step-through latency (seconds) on a test 24 hours following training. Separate groups of animals (n = 10 for each treatment group) were injected (ip) with vehicle (0.9% saline), d-amphetamine (1.0, or 2.0 mg/kg), C105 (0.5 or 1.0 mg/kg) or SN522 (0.25 or 0.5 mg/kg) immediately after training. Data were analyzed using Cox regression within a Kaplan-Meier survival analysis ($p<0.05$).

A Kaplan Meier Survival Analysis demonstrated that doses of 0.5 mg/kg of C105,2.0 mg/kg *d*-amphetamine and 0.5 mg/kg SN522 significantly improved performance on this task (*p* values = 0.007, 0.0004, and 0.03 respectively). Thus, 1-methamphetamine and 1-amphetamine significantly improve memory consolidation.

EXAMPLE 10: L-METHAMPHETAMINE AND MEMORY

Materials and Methods

Animals

Male, Long- Evans rats (3- 5 months of age) obtained from Charles River Laboratories weighing between 250 and 350 grams at the time of arrival served as subjects in these experiments. The rats were housed two to a cage in plastic cages with corncob bedding. The rats were maintained on a 12/12 light dark cycle with *ad libitum* access to food and water.

Drugs

L- methamphetamine (SN522) was dissolved in saline and administered to the rats via intraperitoneal (i.p.) injections, in a volume of 1 ml/kg body weight.

Results and Discussion

Experiment 1: Passive Avoidance

The Passive Avoidance apparatus (Coulbourn Instruments) consisted of a light chamber and a dark chamber, which were joined by means of a sliding guillotine door. The floor of the dark compartment consisted of 2.4 mm diameter steel rods, through which a foot-shock could be administered to the animal by a constant current 18-pole shock scrambler. The test apparatus was enclosed in a ventilated, sound-attenuating cabinet, and was controlled by Graphic State™ Software (Version 1.013) and a Hewlett Packard Pavilion Computer.

Training involved placing the rat inside the light chamber with its head facing away from the door. Ten seconds later, the sliding door was opened, and the latency to enter the dark chamber was recorded (100 second maximum). When the rat entered the dark chamber, it received a continuous foot-shock (0.4 mA) though the metal grid floor until it returned to the light chamber. This sequence of events was continued until the rat remained in the light chamber for a period of 100 consecutive seconds or untilo a maximum of 5 foot-shocks had been received.

Retention testing was conducted 24 hours later. The rat was placed into the light chamber with its head facing away from the door. Ten seconds later, the door was opened, allowing the rat access to the dark chamber. No foot-shock was administered during retention testing. Latency to enter the dark chamber was recorded (900 seconds maximum) and used as a measure of memory.

In this experiment, the effects of SN522 on consolidation of the passive avoidance task were investigated. Rats were injected with saline (contol, no or zero drug) or six different doses (0, 0.10, 0.25, 5.0, 0.75 or 1.0 mg/kg, i.p.) of SN522 immediately after the training session. Retention for the task was tested 24 hours later.

The results from this experiment are illustrated in Figure 21. Figure 21 depicts the effects of SN522 administered immediately after training in inhibitory avoidance. Data show the mean (±SEM) step-through latency (seconds) on a test 24 hours following training. Separate groups of animals (number of animals in each treatment group indicated inside bars) were injected with vehicle (0.9% saline) or one of five doses of SN522 (0.1, 0.25, 0.5, 0.75, or 1.0 mg/kg). Data were analyzed usine Cox regression within a Kapaln-Meier survival analysis ($p<0.05$).

The step-through latency in response to SN522 is an inverted U-shaped dose response curve. A Kaplan Meier survival analysis with cox regression confirmed significant improvement in memory performance relative to the saline group at doses of 0.25, 0.5, and 0.75 mg/kg ($p <0.05$).

Experiment 2: Water Maze

Water Maze testing (Morris, R., J. Neurosci Methods 11:47-60 (1984)) was conducted in a galvanized steel pool, painted white, measuring 180 cm in diameter and 60 cm in height. The pool was equipped with a removable circular platform (10 cm in diameter) made of clear Plexiglas. The pool was filled with water (26°C) to a level of 1 cm above the surface of the platform. Nontoxic white paint was added to the water to obscure the platform's appearance. The pool was divided conceptually into four quadrants and the platform was located in the NW quadrant 30 cm from the pool wall. Extramaze cues were provided by large geometric shapes adhered to curtains that surrounded two sides of the pool, and by shelving units, a sink, and posters on the visible walls.

The training procedure involved placing the rat into the water, with the rat's head facing the wall of the pool, at one of four different starting points. The rat was allowed 60 seconds to locate the hidden platform. If the rat did not find the platform within 60 seconds, it was gently guided to the platform. After 15 seconds spent on the platform at the end of each trial, the rat was removed from the pool and injected with saline or SN522 (0.25 and 0.5 mg/kg, i.p.). The rat was dried, and returned to its home cage. One trail was conducted each day for 10 days. The latency to reach the platform (escape latencey) was recorded on each days training trial.

An ANOVA show a significant enhancement in acquisition rate in animals administered 0.25 mg/kg SN522 relative to controls (See Figure 22; $F_{1,17} = 10.245$, $p<0.005$).

Figure 22 depicts the effect of SN522 on acquisition of the water maze task. Data are the mean (±SEM) latency to locate a hidden platform by three separate groups of rats (n = 10 for each treatment group). Animals were given a single learning trial each day. Immediately following the learning trial, vehicle (saline) or SN522 (0.25 or 0.5 mg/kg) were administered IP. The data show that while all groups learned to find the platform during the 10 training days indicated by the decrease in escape latency, animals that were treated with 0.25 mg/kg SN522 after each trial learned to find the platform more quickly than rats in the saline treated group.

The comparison between the control (saline) group and the group administered the higher dose of SN522 did not reveal any statistical differences ($p>0.05$). These data confirm that SN522 has potent effects on mnemonic processing.

Figure 23 depicts the effect of SN522 on activity levels measured by an automated motion detector. Data are the mean activity (±SEM) of four separate groups of rats (n = 7 or 8 per group) treated with SN522 (0.25, 0.5, 2.5, and 5.0 mg/kg, ip) as measured by an activity monitor system, tracking beam breaks around an open field. Data are shown as a percent change from a control group treated with vehicle (0.9% saline). The data show that doses of 1-methamphetamine (SN522) have profound effects on memory processes produce no or modest changes in motor behavior (0.25 and 0.5 mg/kg). Doses of SN522 ten times over the therapeutic doses yielded only mild increases in activity. Thus, 1-methamphetamine has no or minimal side effects.

Experiment 3: Locomotor Activity

Activity monitoring was conducted in a Plexiglas open-field box measuring 30 by 30 cm. Activity levels were measured via a grid of infrared light beams that traversed the cage from left to right and back to front. The location of the animal within the cage was detected by breaks in the infrared light beams. Light beams status information was collected and rapidly analyzed by a computerized activity monitoring system (VersaMax System, Accuscan Instruments.)

In order to determine whether SN522 had any adverse effects on locomotor or exploratory activity, rats were injected with saline or 0.25, 0.5, 2.5 and 5.0 mg/kg of SN522 and immediately afterwards placed into the activity monitoring chambers for a period of three hours. Data was collected on-line using Versa Max (Version 1.83) computer software and a Hewlett Packard Pavilion computer. Analyzed behaviors included; horizontal activity, total distance moved, movement time, number of movements, number of rears, number of stereotyped movements, and time spent resting.

Figure 24 depicts the effect of d-amphetamine on activity levels measured by an automated motion detector. Data are the mean activity (±SEM) of four separate groups of rats treated with SN522 Mean activity (±SEM) of four separate groups of rats (n = 7 or 8 per group) treated with d-amphetamine sulfate (0.25, 0.5, 2.5, and 5.0 mg/kg, ip). Data are shown as a percent change from a control group treated with vehicle (0.9% saline). The data show that even very low doses of d-amphetamine have effects on activity. These effects are significant even at the very lowest dose tested (0.25 mg/kg), with profound increases in activity at the higher doses all p's,0.05.

As can be seen in Figure 24 (data are shown as a percent increase relative to the saline control), low doses SN522 resulted in no increase in activity at doses that were efficacious in the memory assays (0.25 mg/kg, $p>0.05$), and produced only modest increases in activity at a slightly higher dose (0.5 mg/kg; $F_{8, 112}= 2.303$, $p=0.028$) . Relatively high doses of SN522 (2.5 and 5.0 mg/kg) resulted in small, but significant increases in activity relative to the saline control (0.25 mg/kg; $F_{8, 112} =10.936$, $p<0.001$; 5.0 mg/kg; $F_{8, 112}=8.749$, $p<0.001$) . However, when compared with activity produced by similar doses of *d*- amphetamine, the increases in activity levels after administration of SN522 are minimal indicating minimal side effects from SN522.

Experiment 4: Tailflick Test of Analgesia

The tail-flick response was assessed using a radiant heat tail flick monitor (Accuscan Instruments model TFS) equipped with a radiant heat element and two light beam sensors to detect tail movement.

In order to determine whether SN522 has any analgesic properties, rats (n=10 per treatment group) were injected with saline or SN522 (0.25, 0.5, 2.5 and 5.0 mg/kg, i.p.) and tested at four time points: immediately prior to injection, and again 15, 30, and 60 minutes following drug administration for a tail flick response (D'Amour, F.E., et al., J. Pharmacol. Exp. Ther. 72:174-179 (1941)). To test the tailflick, the animal was placed on top of the Tail-Flick monitor and gently held in place with a cotton towel. The tail of the animal was placed in a shallow groove lying between the two sensors and over the top of the radiant heat wire. The heat element was activated, and the latency for the animal to flick its tail out of the groove and away from the heat source was automatically recorded via activation of the sensors. The intensity of the heat source was adjusted so that the animal flicked its tail within 3-4 seconds. A 10 second cutoff was imposed to avoid tissue damage. The animal was returned to its home cage immediately following testing.

The results of an ANOVA over the five drug treatments and four test intervals did not reveal any differences in tail flick latency. Thus, SN522 does not alter pain sensitivity in this test.

EXAMPLE 11: L-METHAMPHETAMINE IMPROVES MEMORY IN SUBJECTS WITH A MEMORY IMPAIRMENT

Materials and Methods

Animals

Male, Long- Evans rats (3- 5 months of age), obtained from Charles River Laboratories and weighing between

250 and 350 grams, served as subjects in these experiments. The rats were housed two to a cage in plastic cages with corncob bedding and were maintained on a 12/12 light dark cycle with ad libitum access to food and water.

Drugs

L- methamphetamine (SN522) was dissolved in saline and administered to the rats via intraperitoneal (i.p.) injections, in a volume of 1 ml/kg body weight.

Passive Avoidance Testing

The Passive Avoidance apparatus (Coulbourn Instruments) consisted of a light chamber and a dark chamber, which were joined by means of a sliding guillotine door. The floor of the dark compartment consisted of 2.4 mm diameter steel rods, through which a foot-shock could be administered to the animal by a constant current 18-pole shock scrambler. The test apparatus was enclosed in a ventilated, sound-attenuating cabinet, and was controlled by Graphic State™ Software (Version 1.013) and a Hewlett Packard Pavilion Computer.

Training involved placing the rat inside the light chamber with its head facing away from the door. Ten seconds later, the sliding door was opened, and the latency to enter the dark chamber was recorded (100 second maximum). When the rat entered the dark chamber, it received a continuous foot-shock (0.4 mA) through the metal grid floor until it returned to the light chamber. This sequence of events was continued until the rat remained in the light chamber for a period of 100 consecutive seconds or until a maximum of 5 foot-shocks had been received.

Retention testing was conducted 24 hours later. The rat was placed into the light chamber with its head facing away from the door. Ten seconds later, the door was opened, allowing the rat access to the dark chamber. No foot-shock was administered during retention testing. Latency to enter the dark chamber was recorded (900 seconds maximum) and used as a measure of memory.

In order to determine whether 1-methamphetamine (SN522) reverses memory deficiencies (also referred to herein as a "memory impairment"), separate groups of rats were injected with scopolamine hydrochloride (0.75 mg/kg, i.p.) 30 minutes before training. Rats were then trained on the passive avoidance task and immediately afterwards, injected with either saline or SN522 (0.12, 0.25, 0.5 and 1.0 mg/kg, i.p.). Retention was tested 24 hours after training.

Results and Discussion

Figure 32 depicts the effects of SN522 on memory deficiencies, in particular, as a consequence of exposure to scopolamine (the number of animals in each group are indicated inside bars). The data shown represent the mean (±SEM) step-through latency (secs) 24 hours following training. A Kaplan Meier survival analysis demonstrated that SN522 was effective at alleviating the impairment in the scopolamine-injected animals (log rank statistic c2(6) = 14.73 p = 0.02).

As shown in Figure 32, scopolamine treated rats receiving 0.25, 0.5 and 1.0 mg/kg SN522 performed significantly better than animals treated with scopolamine and saline (p values = 0.01, 0.04 and 0.007 respectively). There was no significant difference between control rats who received injections of saline, and scopolamine treated rats who received injections of SN522 at the 0.25, 0.5 and 1.0 mg/kg doses ($p > 0.05$).

These results show that 1-methamphetamine improves memory in subjects with a memory impairment.

EXAMPLE 12: L-AMPHETAMINE IMPROVES MEMORY IN SUBJECTS WITH A MEMORY IMPAIRMENT

Materials and Methods

Animals

Male, Long- Evans rats (3- 5 months of age), obtained from Charles River Laboratories and weighing between 250 and 350 grams, served as subjects in these experiments. The rats were housed two to a cage in plastic cages with corncob bedding and were maintained on a 12/12 light dark cycle with ad libitum access to food and water.

Drugs

L- amphetamine (C105) was dissolved in saline and administered to the rats via intraperitoneal (i.p.) injections, in a volume of 1 ml/kg body weight.

Passive Avoidance Testing

In order to determine whether 1-amphetamine (C105) reverses memory deficiencies (also referred to herein as a "memory impairment"), separate groups of animals (the number of animals in each treatment group are indicated inside bars) were injected with either vehicle (0.9% saline; SAL) or scopolamine (0.75 mg/kg) 30 min prior to training. Rats were then trained on the passive avoidance task and immediately afterwards were injected with saline (SAL) or C105 (0.12, 0.25, 0.5 and 1.0 mg/kg, i.p.) and an additional group was treated with physostigmine (0.075 mg/kg, i.p.) as a positive control. Retention was tested 24 hours after training. Data were analyzed using Cox regression within a Kaplan-Meier survival analysis.

Results and Discussion

Figure 33 depicts the effects of 1-amphetamine (C105) memory deficiencies, in particular, as a consequence of exposure to scopolamine. Data shown represent the mean ($\pm$SEM) step-through latency (secs) 24 hours following training. A Kaplan Meier survival analysis demonstrated that C105 was effective at alleviating the impairment in the scopolamine-injected animals (log rank statistic c2(6) = 14.73 p = 0.02). As shown in Figure 33, scopolamine treated rats receiving 1.0 mg/kg of C105 performed significantly better than animals treated with scopolamine and saline ($p > 0.05$). There was no significant difference between saline only treated rats, and scopolamine treated rats who received 1.0 mg/kg of C105 ($p > 0.05$).

These results show that 1-amphetamine improves memory in subjects with a memory impairment.

EXAMPLE 13: STUDY OF (R)-(-)-AMPHETAMINE IN HUMANS

Sixteen (n=16) healthy adult male/female subjects, ages 20-72, took part in the study. Subjects were selected from a volunteer database. Study-related procedures were carried out after informed consent had been given.

Figure 18 shows a pharmacokinetic measure, in the form of serum levels, for (R)- (- )- amphetamine up to 6 hours after administration. In the ascending dose portion of the dose curve, the Brief Visuospatial Memory Test (BVMT) and Rey Auditory and Verbal learning Test (RAVLT) tests were performed and observed to be stastistically significantly higher when compared to controls, having a $p < 0.01$. Higher BVMT and RAVLT scores indicate an improvement in memory, in particular memory consolidation. On the other hand, patients were assessed using the Providence Recognition Memory Test (Pictoral) after 3 hours, e.g., after the ascending arm of the dose curve, and the PRMT scores (both for words and pictures) were both not observed to be statistically significant from controls. These experiments demonstrate that (R)- (- )- amphetamine can enhance memory in patients, and is more effective during the ascending portion of the plasma curve.

Figure 19 shows a dose response curve for acute dosing with (R)- (- )- amphetamine. Statistically signifcant differences, e.g., as illustrated by the p values, were observed between pacebo and dosages of about 30 mg and about 45 mg per day. In particular, at 30 min: p = 0.004 for placebo vs about 30 mg, and p = 0.03 for placebo vs. about 45 mg. At 24 hour, p = 0.002 for placebo vs about 30 mg, and p = 0.05 for placebo vs about 45 mg.

EXAMPLE 14: IMPROVEMENT IN MEMORY IN HUMANS WITH L-AMPHETAMINE

Two (2) Phase 1 randomized, double-blind, placebo-controlled clinical studies of 1-amphetamine (C105) were conducted in normal healthy adult male and female subjects. The first trial was conducted in eight (8) Caucasian subjects (3 male and 5 female) with a mean age of 35.1 years (range 21-49 years), and the second trial was conducted in eight (8) Caucasian subjects (1 male and 7 female) with a mean age of 65.4 years (range 60-72 years).

The studies were intended to identify the maximum tolerated dose (MTD) and dose-limiting side effects of C105, to assess the effects of C105 on quantitative memory scores, to assess the perceived central nervous system (CNS) effects following C105 administration, to assess the effects of C105 on the cardiovascular system, to explore the relationship between dose, tolerability, safety and pharmacological effects of C105, and to define the pharmacokinetics (PK) of C105.

There were five (5) treatment periods in each Phase 1 trial. Each treatment period was one (1) week in duration and consisted of two (2) consecutive days of treatment ("treatment period") with C105 (5 mg, 15 mg, 30 mg, 45 mg) or placebo, followed by five (5) consecutive days without C105 or placebo ("washout period"). The design is a ascending dose safety design with a placebo dose randomly inserted iinto the sequence. Each subject was randomly administered a single dose of one of the C105 doses (5 mg, 15 mg, 30 mg, 45 mg) or a randomly assigned dose of placebo during each treatment period on the two consecutive treatment days. Each subsequent treatment group would included whatever dose of C105 (or placebo) had not previously been administered, until the patient had received each of the four C105 doses (5 mg, 15 mg, 30 mg, 45 mg) or single placebo treatment to conclude the five week treatment period. Safety data

were reviewed after each dose prior to advancement to the next dose level.

The Rey Auditory Visual Learning Test (RAVLT, Rey, A. (1941). L'examen psychologique dans les cas d'encéphalopathie traumatique. Archives de Psychologie, 28,21, Lezak, M.D. (1995). Neuropsychological Assessment (3rd ed.). New York: Oxford University Press) was conducted during each of the two consecutive days when the patent received C105 or placebo. RAVLT was not conducted during the washout period.

The RAVLT assessment for word recall was made at two (2) different times following C 105 or placebo treatment during each of the five treatment periods. The first RAVLT assessment was made on the first day of treatment in each treatment period and consisted of two parts. Fifteen (15) nouns were read aloud to the patient by an Examiner, followed by an interference or distraction trial, which is then followed by a free-recall test of the 15 nouns. After a additional 30-minute delay period, the subject was again required to recall the first set of 15 nouns and also complete a 50-word recognition test. The second RAVLT assessment was made on the second day of treatment in each treatment period and consisted of a repeat of the Recall test and the 50-word Recognition test given on the previous day. The second RAVLT assessment evaluated word recall 24 hours ($\pm$ 2 hours) after the first RAVLT assessment and did not consist of another exposure to nouns by an Examiner, but rather a recall of the nouns given to the subject 24 hours earlier.

Safety and tolerability assessments were changes in vital signs, ECGs, Holter monitoring, laboratory tests, physical examination and adverse events. Serial blood and urine samples were collected up to 24 hours after dosing for subsequent determination of C105 plasma concentrations and calculation of pharmacokinetic parameters.

C105 was well tolerated when administered in single oral doses ranging from 5 to 45 mg. Reported adverse events were minor. The most frequently reported adverse events were dizziness, headache, insomnia, sinus tachycardia, supraventricular tachycardia and aptyalism. Reported adverse events were generally mild in severity and resolved without requiring treatment. There were no reported serious adverse events and no subject was discontinued from the study due to an adverse event. There were no clinically meaningful findings with respect to C105 administration on physical examinations, laboratory tests, vital signs, 12-lead ECGs or Holter recordings.

For each patient, RAVLT data showed that all dose groups have mean values higher than the placebo at 30 minutes. The two highest doses (30 mg and 45 mg) exhibited the greatest improvement (highest values) compared to placebo testing. The benefit observed at 30 minutes was maintained at 24 hours, with the recalled number of words slightly lower at 24 hours for each dose compared to the corresponding results at 30 minutes. The recall scores were approximately 10 words following administration of the 45 mg dose, compared to approximately 7-8 words recalled when the placebo dose had been administered.

Figure 25 shows a pooled statistical analysis of the 30- minute and 24- hour RAVLT memory scores for all subjects. The scores showed an overall statistically significant ($p<0.05$) improvement in RAVLT score with respect to C105 dose at both 30 minutes and 24 hours post- treatment. In addition, improvements in RAVLT scores observed with the 30 mg and 45 mg doses of C105 were statistically significant ($p<0.05$), based on the Wilcoxon signed rank test, when compared to placebo at both 30 minutes and 24 hours post- dose. The difference is also significant ($p=0.0559$) at the 5 mg dose for the 24- hour recall scores. A comparison of each individual subject's placebo score to their best score on any dose of C105 showed that RAVLT memory performance for all subject (except for one subject who had a perfect RAVLT score under both conditions) improved following C105 treatment (see Figure 26) .

These data demonstrate that 1-amphetamine (C105) enhance memory, in particular memory consolidation.

EXAMPLE 15: IMPROVEMENT IN COGNITIVE PROCESSES FOLLOWING L-AMPHETAMINE ADMINISTRATION

A computerized cognitive screening tool (HeadMinder, Inc. (New York, NY)), was employed for the assessment and longitudinal tracking of cognitive functioning (Erlanger, D.M., et al., J. Head Trauma Rehabil. 17:458-476 (2002); US2003/0073885A1 (2003); WO 01/54650 (2001); WO 01/72217 (2001); WO 01/54559 (2001), the entire teachings of which are hereby incorporated by reference in their entirety).

Mild cognitive impairment in subjects was diagnosed based on conventional neuropsychiatric testing parameters - scoring below the age- and educational-adjusted cutoff on the Logical Memory II subscale from the Wechsler Memory Scale. A single test score was used to define subjects with mild cognitive impairment. In addition, the subjects used in this study did not score higher than 0.5 on the Clinical Dementia Rating scale. Some subjects with a Clinical Dementia Rating of 0.5 can have early Alzheimer's disease. Some subjects enrolled in this study scored in the Alzheimer's disease range of some cognitive assessments. Thus, some subjects classified as having mild cognitive impairment may actually have early Alzheimer's disease. In populations of humans with mild cognitive impairment and early Alzheimer's disease, neuropsychiatric tests typically measure a continuum (bell-shaped curve) and the definitions are relatively arbitrary cut points for the abnormal population - typically 1 or 1.5 standard deviations below the norm adjusted for age and education.

The battery of nine (9) tests completed by the subjects included two (2) "warm-up" tasks. The remaining seven (7) subtests were included to evaluate cognitive abilities, such as learning and memory, attention, reaction time, and executive function.

TEST FACTORS AND SUBTEST DESCRIPTIONS

Keyboard Proficiency - Warm-up Tasks

- *Keyboard Proficiency 1*
  A green ball appears on the screen. The subject presses the space bar as quickly as possible until the ball turns red.
- *Keyboard Proficiency 2*
  Numbers from 0-9 appear. The subject presses the number that appears on the screen on the keyboard as quickly as possible.

Learning and Memory

- *Mentory Cabinet 1*
  Subjects learn the placement of nine (9) household objects placed in a cabinet over three (3) learning trials.
- *Memory Cabinet 2* - Delayed Memory
  Following intervening tasks, one (1) recall trial of the Memory Cabinet is administered to the subject.

The following depicts the memory cabinet employed in the study:

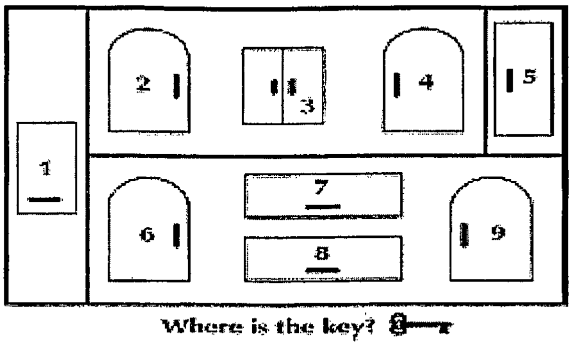


Attention and Executive Function

- *Response Direction 1* - Simple Attention (a low demand task) Numbers are presented. The subject presses the "1" key when a 1 is presented and presses the "0" key when a 0 is presented. The following depicts a schematic of this task:

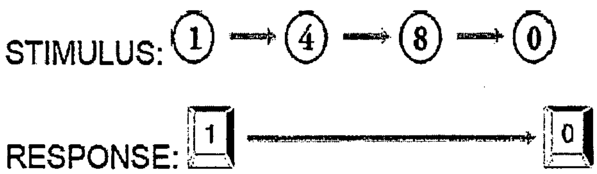


- *Response Direction* 2 - Response Reversal (a high demand task) Numbers are presented one at a time. The subject presses the "1" key when a 0 is presented and presses the "0" key when a 1 is presented. The following depicts a schematic of this task:

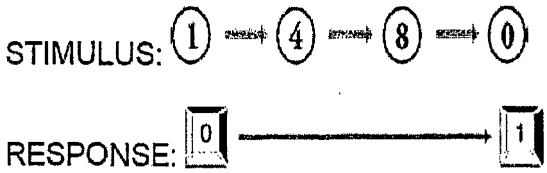


Supplemental Tests

The subject places his or her fingers on 4 keys, each representing a box on the screen.

- *Visuo-Motor Speed 1*
  Boxes light up one at a time. The subject presses the corresponding key as quickly as possible.

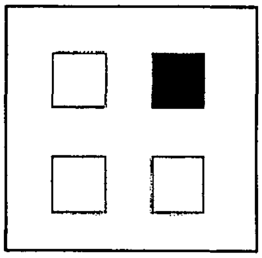

- *Visuo-Motor Speed 2*
  Instructions appear in the center of the screen. The subject presses the corresponding key as quickly as possible (e.g., "UPPER RIGHT" appears in the middle).

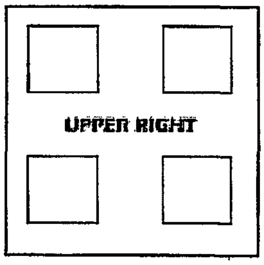


- *Visuo-Motor Speed 3*
  Instructions are presented on the screen in the wrong location. The subject presses keys according to each instruction while ignoring the location of the instruction (e.g., "UPPER RIGHT" appears in the lower right).

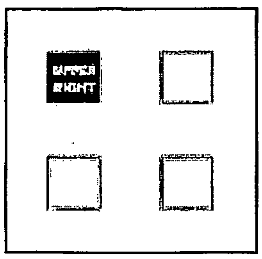

SUBJECTS

Thirteen (13) patients (age 64-88 years old, mean age 77 years old) completed the study. One subject was not included in the analyses because he was unable to conform to the protocol. In the 1-amphetamine treated group, there were 5 females and 3 males. The placebo group included 1 female and 4 males. Gender did not have an effect on cognitive performance at intake, optimal titrated dose, and washout. All participants were Caucasian. The groups differed

in age (F = 4.44, df = 1, 11, p = 0.05) and marginally in level of education (F = 4.2, df = 1, 11, p = 0.06) at time of testing. Groups did not differ significantly on any cognitive test variable at screening for inclusion in the study or washout assessment.

Age and Education of the two groups

| | 1-amphetamine (n=8) | | | Placebo (n=5) | | |
|---|---|---|---|---|---|---|
| | *Mean* | *St. Dev.* | *Range* | *Mean* | *St. Dev.* | Range |
| Age | 80.3 | 5.5 | 72.1-88.7 | 73.3 | 6.3 | 64.9-79.6 |
| Education | 12.12 | 2.4 | 9-16 | 15.4 | 3.4 | 12-20 |

Subjects were randomly assigned to receive either l-amphetamine or a placebo. Subjects treated with l-amphetamine (n=8) received 5 mg of 1-amphetamine per day for the first seven (7) days of the study, following by 15 mg per day for the next seven (7) days of the study, followed by 30 mg per day for the next fourteen (14) days of the study. Subjects receiving placebo received identical dosages of placebo pills for the duration of the study. Cognitive functions were assessed at baseline (day 0) and on days 1, 8, 15 and 28 of treatment or placebo.

<u>RESULTS</u>

To demonstrate equivalent keyboard and general cognitive skills, an ANCOVA (Analysis of Co-Variance) exploring the treatment condition (treatment = 1-amphetamine administration at 5 mg, 15 mg, 30 mg) was performed with Keyboard Proficiency 2 as the dependent variable and age as the covariate. As expected,1-amphetamine and placebo subjects did not differ in performance on this task at any test instance, indicating they were roughly equivalent (Figure 27). Higher scores in the keyboard proficiency indicate slower performance.

Keyboard Proficiency 2: Age Scaled Scores and Contrast Statistics

| | Screen | 5 mg | 15 mg | 30 mg | 30 mg | Washout |
|---|---|---|---|---|---|---|
| 1-amphetamine (means/st error) | 34.3 (c.7) | 32.2 (2.2) | 35.1 (7.3) | 29.7 (1.7) | 29.3 (1.6) | 30.6 (2.6) |
| Placebo (mean/st error) | 35.8 (4.9) | 32.3 (2.9) | 33.9 (9.7) | 30.2 (2.2) | 27.9 (2.1) | 28.8 (3.4) |
| F <u>(p)</u> (active vs. control) | .048 (.83) | .001 (.97) | .009 (.92) | .029 (.86) | .22 (.64) | .14 (.71) |
| Adjusted $R^2$ (total model) | -.16 | -.20 | -.10 | -.11 | -.13 | -.13 |

To determine whether the subjects receiving 1-amphetamine had improved memory function, two ANCOVAS exploring the treatment condition were performed with learning and memory as the dependent variables and age as the covariate. Learning (Figures 28 and 31) and memory (Figures 29 and 31) were improved in subjects receiving 1-amphetamine. Significant improvements ($p \leq .05$) in the learning and memory at 30 mg doses (days 15 and 29) were observed in subjects receiving 1-amphetamine compared to subjects receiving placebo. Placebo treated subjects showed no improvement in learning and memory.

Learning: Age Scaled Scores and Group Contrast Statistics

| | Screen | 5 mg | 15 mg | 30 mg | 30 mg | Washout |
|---|---|---|---|---|---|---|
| 1-amphetamine (means/st error) | 4.5 (.66) | 4.2 (.59) | 3.9 (.62) | 5.87 (.54) | 4.9 (.54) | 4.2 (.55) |
| Placebo (mean/st error) | 2.8 (.87) | 1.9 (.78) | 3.0 (.81) | 2.7 (.72) | 2.6(.71) | 2.7 (.72) |

(continued)

| | Screen | 5 mg | 15 mg | 30 mg | 30 mg | Washout |
|---|---|---|---|---|---|---|
| F (p) (active vs. control) | 2.21 (.16) | 4.49 (.06) | .69 (.42) | 10.01 (.01) | 5.85 (.03) | 2.34 (.15) |
| Adjusted $R^2$ (total model) | .44 | .32 | .14 | .61 | .55 | .48 |

Memory: Age Scaled Scores and Group Contrast Statistics

| | Screen | 5 mg | 15 mg | 30 mg | 30 mg | Washout |
|---|---|---|---|---|---|---|
| 1-amphetamine (means/st error) | 4.3 (.68) | 4.0 (.63) | 3.9 (.82) | 6.1 (.66) | 5.0 (.78) | 4.1 (.62) |
| Placebo (mean/st error) | 3.0 (.90) | 1.8 (.83) | 2.8 (1.0) | 2.7 (.86) | 1.9 (1.0) | 2.7 (.81) |
| F (p) (active vs. control) | 1.00 (.33) | 3.74 (.08) | .66 (.43) | 8.15 (.01) | 4.97 (.05) | 1.78 (.21) |
| Adjusted $R^2$ (total model) | .43 | .27 | .05 | -.50 | .41 | .37 |

Improvements in executive function following treatment with 1-amphetamine or placebo were assessed by determining the difference between performance on Response Direction 1 (Low Demand Task) and Response Direction 2 (High Demand Task). Maintained learning efficiency is associated with a stable difference score across repeated assessments. Decreased learning efficiency, which is expected in a subject with mild cognitive impairment, is associated with increased differences across repeated assessments since inefficient subjects make greater improvements due to practice effects on the low demand task than on the high demand task, while efficient subjects improve on both tasks at a similar rate.

An ANCOVA showed significant differences between subjects treated with 1-amphetamine (30 mg, day 15) and placebo. A linear contrast of difference scores between the low and high demand tasks across repeated assessments was not significant for participants in the 1-amphetamine group (F = .20, p = .65) and was significant for participants in the placebo group (F = 2.98, p = .05, one-tailed test). This indicated that those in the l-amphetamine group maintained learning efficiency and that those in the placebo group did not. The placebo group's performance improved on the low demand task, but not on the high demand task, causing the differences to increase in a linear manner. In contrast, the 1-amphetamine group improved equally on both the low and high demand tasks, so that the differences remain roughly the same. These differences are shown in Figure 30 and the following Table. A higher score in Figure 30 indicates a greater inefficiency. In the 1-amphetamine group, a large decrease in learning efficiency at the final assessment - when the medication is no long active - was observed.

Executive Function Scores: Raw Scores and Group Contrast Statistics

| | Screen | 5 mg | 15 mg | 30 mg | 30 mg | Washout |
|---|---|---|---|---|---|---|
| l-amphetamine (means/st error) | .12 (.34) | .17 (.16) | .11 (.16) | .12 (.19) | .11 (.23) | .21 (.08) |
| Placebo (mean/st error) | .06 (.39) | .07 (.46) | .13 (.15) | .35 (.16) | .27 (.24) | .27 (.07) |
| F (p) (active vs. control) | .09 (.76) | .33 (.57) | .07 (.78) | 4.60 (.05) | 1.25 (.28) | 1.32 (.27) |
| Adjusted $R^2$ (total model) | .08 | .06 | .08 | .223 | .02 | .02 |

CONCLUSIONS

Prior to treatment with l-amphetamine, mild cognitive impairment and early Alzheimer's disease subjects had cognitive impairments. Treatment of subjects with placebo did not improve cognitive function. In contrast, subjects treated with 1-amphetamine had improved performance on tests for learning, memory and executive function to the extent (one standard deviation or more) such that subjects treated with 1-amphetamine were scoring within the normal range on tests assessing learning, memory and executive function. The magnitude of improvement in learning, memory and

executive function following 1-amphetamine treatment is considered to be clinically significant and efficaciously statistically significant ($p < 0.05$) for each of the learning, memory and executive function assessments.

Figure 31 illustrates the performance of subjects treated with 1-amphetamine at a peak dose of 30 mg per day compared to placebos controls. A Z-score of 0 represents average performance for healthy individuals and a standard deviation equals 1.

At baseline (prior to treatment) both groups were about equivalent and scored in ranges clearly consistent with mild cognitive impairment and early Alzheimer's disease. At a peak dose of 30 mg per day of 1-amphetamine, subject's scores improved by approximately 1 standard deviation to within normal limits. No change was observed in the control, placebo group.

Improvements in memory, learning and executive function by treatment with 1-amphetamine can have profound implications for improvement in the clinical symptoms of mild cognitive impairment, early Alzheimer's disease and in performances of everyday tasks ranging from managing medications to grocery shopping and operating a vehicle.

EXAMPLE 16

IMPROVEMENT IN MEMORY IN HUMANS TREATED WITH L-METHAMPHETAMINE

Two (2) Phase I randomized, double-blind, placebo-controlled clinical studies were conducted in healthy adult male and female subjects who were administered 1-methamphetamine (SN522). The first clinical trial was conducted with sixteen (16) healthy subjects, who did not have memory or cognitive impairments (also referred to herein as "normal subjects"), ranging in age from 20-60 years (n=8) and 61-80 years (n=8) who were administered placebo or 1 mg, 4 mg, 16 mg or 32 mg of 1-methamphetamine. A second Phase I clinical trial was conducted with eight (8) normal subjects with an age range of 50-64 years. In the second Phase I clinical trial, the eight (8) subjects received 25 mg, 50 mg, 100 mg of 1-methamphetamine; of these eight (8) subjects, five (5) subjects also received placebo (0 mg of 1-methamphetamine) and three (3) subjects received 150 mg of 1-methamphetamine.

The Phase I studies were designed to identify a maximum tolerated dose and dose-limiting side effects of 1-methamphetamine; to assess the effects of 1-methamphetamine on quantitative memory scores for example, by the California Verbal Learning Test (CVLTII) or RAVLT; to assess the perceived CNS effects following the administration of 1-methamphetamine on the cardiovascular system; to explore the relationship between dose, tolerability, safety and pharmacological effects of 1-methamphetamine; and to define the pharmacokinetics of 1-methamphetamine.

There were six (6) treatment periods in the first Phase I clinical trial. There were four (4) treatment periods in the second Phase I clinical trial. Each treatment period was one (1) week in duration and consisted of two (2) consecutive days of treatment with 1-methamphetamine (1, 4, 16 or 32 mg for the first Phase I study and 25, 50 or 100 mg for the second Phase I study) or a placebo, followed by five (5) consecutive days without 1-methamphetamine or a placebo. Each subject was randomly administered a single dose of one of the 1-methamphetamine doses or randomly assigned a dose of placebo during each treatment period. Each subsequent treatment group would include whatever dose of 1-methamphetamine had not previously been administered, until the patient had received each of the 1-methamphetamine doses or a single placebo treatment to conclude the treatment period. Safety data were reviewed after each dose prior to advancement to the next dose level. The RAVLT assessment for word recall was performed as described above and made at two different times following the administration of 1-methamphetamine or a placebo during each of the treatment periods. The initial RAVLT training was conducted approximately two and a half hours after administration of the 1-methamphetamine or a placebo. After a 30-minute delay period, the subject was required to recall a first set of 15 nouns. The second RAVLT assessment was made approximately 24-hours following treatment with 1-methamphetamine.

Safety and tolerability assessments were as described for treatment with Phase I clinical study with 1-amphetamine and consisted of assessments in vital signs, ECGs, physical examination and the noting of any adverse events.

L- methamphetamine was generally well tolerated in the first and second Phase I trials. In a few subjects in the second Phase I trial, a 150 mg dose was not well tolerated and this dose was not continued.

As shown in Figure 34, RAVLT data from the second Phase I study show that all dose groups have mean values greater than placebo at 30 minutes and 24 hours. Thus, the benefit in improving memory following the administration of 1-methamphetamine observed at 30 minutes was maintained at 24 hours. No difference in memory scores was observed in subjects in the first Phase I study.

These data show that administration of l-methamphetamine can enhance memory in subjects who do not have any known impairment in memory or cognition.

EXAMPLE 17

IMPROVEMENT IN COGNITIVE PROCESSES FOLLOWING L-METHAMPHETAMINE ADMINISTRATION

A randomized, double-blind, placebo-controlled, dose escalation study in human subjects, who were not suffering from an impairment in a memory or cognitive process ("normal subjects"), was conducted to assess the safety, tolerability and pharmacokinetics and improvement in cognitive processes, including memory, following the administration of l-methamphetamine (25 mg, 50 mg, 100 mg, 150 mg).

Eight (8) subjects received 25 mg, 50 mg, 100 mg of 1-methamphetamine; of these eight (8) subjects, five (5) subjects also received placebo and three (3) subjects received 150 mg of 1-methamphetamine. The studies were conducted employing a battery of cognitive tests developed by Cognitive Drug Research (CDR) in the United States.

A selection of tasks from the CDR computerized cognitive assessment system was administered and parallel forms of the tests were presented on each testing session. The CDR tasks are well-established assessments of cognition and known to one of skill in the art. All tasks were computer-controlled, the information was presented on high resolution screens, and the responses recorded via a response module containing two buttons, one marked 'NO' and the other 'YES'. The tracking task additionally involved the use of a joystick. The test battery takes about 20-25 minutes to perform. The tests were administered in the following order:

- **Picture Presentation:** A series of 20 pictures was presented on the screen at the rate of 1 every 3 seconds for the subject to remember. No data were recorded from this task.
- **Simple Reaction Time:** The subject was instructed to press the 'YES' response button as quickly as possible every time the word 'YES' is presented on the screen. Fifty stimuli were presented with a varying inter-stimulus interval.
- **Digit Vigilance:** A target digit was randomly selected and constantly displayed to the right of the screen. A series of digits was then presented in the center of the screen at the rate of 150 per minute and the subject was required to press the 'YES' button as quickly as possible every time the digit in the series matches the target digit. There were 45 targets in the series. The task lasted for 3 minutes.
- **Choice Reaction Time:** Either the word 'NO' or the word 'YES' was presented on the screen and the subject was instructed to press the corresponding button as quickly as possible. There were 50 trials for which each stimulus word was chosen randomly with equal probability and there was a varying inter-stimulus interval.
- **Rapid Visual Information Processing:** A series of digits was presented on the screen at the rate of 100 per minute. The subject had to detect targets consisting of consecutive sequences of either three odd digits or three even digits, and to report them by pressing the 'YES' button as quickly as possible. There were 32 targets. The task lasted for 4 minutes.
- **Tracking:** The subject used a joystick to track a randomly moving target on the screen for one minute. The distance off-target per second was recorded.
- **Spatial Working Memory:** A picture of a house was presented on the screen with four of its nine windows lit. The subject had to memorize the position of the lit windows. For each of the 36 subsequent presentations of the house, the subject was required to decide whether or not the one window that was lit was also lit in the original presentation. The subject responded by pressing the 'YES' or 'NO' buttons as appropriate, as quickly as possible.
- **Numeric Working Memory:** A series of five digits was presented for the subject to hold in memory. This was followed by a series of 30 probe digits for each of which the subject had to decide whether or not it was in the original series and press the 'YES' or 'NO' response button as appropriate, as quickly as possible. This procedure was repeated twice more, using two different series and probes.
- **Picture Recognition:** The original pictures plus 20 distracter pictures were presented one at a time in a randomized order. For each picture the subject had to indicate whether or not the subject recognized it as being from the original series by pressing the 'YES' or 'NO' button as appropriate, as quickly as possible.

Summary statistics were calculated for the unadjusted scores, and the difference from baseline (pre-dose) data collected. Repeated measures analysis of variance (ANOVA) was conducted on the difference from baseline data fitting terms for dose, time, period and the dose-time interaction. A random effect of subjects was fitted to the model.

As shown in Figure 35, l-methamphetamine (SN522) had a dose-dependent effect on speed of response. This dose dependent pattern indicates a post-dose decline with placebo treatment, and increasing post-dose improvements in response speed with active dosing as shown using a combined Total Speed score, which combines the reaction time measures from all the CDR tasks, except the Tracking task. The data shown in Figure 35 compares the score obtained after l-methamphetamine treatment with a pre-dose score. Subjects receiving placebo take a longer time to respond with a dose-response reduction in the total response time. A dose dependent pattern was evident in the LSmean difference from baseline, as shown in Figure 35.

A pattern for dose dependent benefit was evident on several of the reaction time measures, as shown in Figure

35. Total speed tasks assess cognitive functions.

As shown in Figure 36, administration of 1-methamphetamine (25 mg, 150 mg) improved the Picture Recognition - Sensitivity Index, a task that assesses memory. A significant effect of treatment was seen from the ANOVA (p=0.004). The LSmean comparisons showed significant benefits for 25 mg (p=0.0177) and 150 mg (p=0.0254) over placebo. Three (3) subjects received 150 mg dose of 1-methamphetamine. Figure 36 shows a pattern for dose dependent improvement for 50 mg, 100 mg and 150 mg of l-methamphetamine. A possible explanation for the lack of fit of the 25 mg dose was that the pre-dose baseline for 25 mg was poor, leading to a post-dose improvement. Thus, the relative pre-dose performance of the dose groups should be considered.

As shown in Figures 37 and 38, a significant effect of dose was observed for Information Processing Targets Detected (p=0.0005), an assessment of cognition, following administration of l- methamphetamine. The LSmean comparisons showed significant benefits for 100 mg (p=0.0002) and 150 mg (p=0.0001) over placebo (Figure 37) . This pattern was improved with a 100 and 150 mg dose and was in contrast to a pattern for a dose dependent increase in False Alarms, which also showed a significant main effect of dose (p=0.0001) . The LSmean comparisons showed significant decrements against placebo for 50 mg (p=0.0002), 100 mg (p=0.0001) and 150 mg (p=0.0001) doses (Figure 38) .

In summary, these data show a dose dependent effect of 1- methamphetamine on reaction time (speed of response) on the CDR task measures. A dose of 25 mg and 150 mg of 1- methamphetamine improved Picture Recognition-Sensitivity Index (accuracy), an assessment of memory. A dose dependent benefit of l- methamphetamine on Information Processing Targets Detected (accuracy) was also observed, which is an assessment of cognition. This benefit was also associated with increased False Alarms on the task, which may indicate a change in response strategy (increased responding), rather than a direct benefit to accuracy. However, when the False Alarms measures was used as a covariate in the analysis of the Targets Detected (accuracy) measure, the benefits following 1- methamphetamine administration remained, indicating a benefit to accuracy following 1- methamphetamine administration. Therefore, 1- methamphetamine has dose dependent effects benefiting response speed, and beneficial effects, on memory and information processing accuracy. These beneficial effects observed in normal healthy subjects support the beneficial effects for use of the amphetamine compounds of the invention in subjects having cognitive impairments.

## Claims

1. An amphetamine composition for use in treating an impairment in memory or an impairment in a cognitive function, wherein the amphetamine composition is selected from the group consisting of 1-amphetamine, 1-methamphetamine, or combinations of both, and further wherein: the impairment in memory or the impairment in cognitive function is associated with multiple sclerosis and the l-amphetamine is administered as a component of a composition that includes at least about 60 mole percent l-amphetamine relative to a total amphetamine content of the composition and the l-methamphetamine is administered as a component of a composition that includes at least about 90 mole percent l-methamphetamine relative to a total methamphetamine content of the composition.

2. The amphetamine composition of Claim 1 for use of claim 1, wherein the memory impairment and an improvement in memory in the human is determined by a Rey Auditory Verbal Learning Test.

3. The amphetamine composition of Claim 1 for use of claim 1, wherein at least one member selected from the group consisting of short-term memory, working memory, long-term memory, memory consolidation, procedural memory and declarative memory is improved in the treatment of the impairment in memory.

4. The amphetamine composition of Claim 1 for use of claim 1, wherein either the l-amphetamine is administered at a dose of between about a 1 mg dose and about a 150 mg does per day; or the l-methamphetamine is administered at a dose of between about a 1 mg dose and about a 150 mg dose per day.

5. The amphetamine composition of Claim 1 for use of claim 1, wherein at least one member selected from the group consisting of attention, executive function, reaction time, learning, information processing, conceptualization, problem solving and verbal fluency is improved in the treatment of the impairment in cognitive function.

6. The amphetamine composition of Claim 1 for use of claim 1, wherein 1-amphetamine is administered, and wherein the l-amphetamine is administered as a component of a composition that includes at least 75 mole percent l-amphetamine relative to a total amphetamine content of the composition.

7. The amphetamine composition of Claim 1 for use of claim 1, wherein l-amphetamine is administered, and wherein

the l-amphetamine is administered as a component of a composition that includes at least 99 mole percent l-amphetamine relative to a total amphetamine content of the composition.

8. The amphetamine composition of Claim 1 for use of claim 1, wherein l-methamphetamine is administered, and wherein the l-methamphetamine is administered as a component of a composition that includes at least 95 mole percent l-methamphetamine relative to a total methamphetamine content of the composition.

9. The amphetamine composition of Claim 1 for use of claim 1, wherein l-methamphetamine is administered, and wherein the l-methamphetamine is administered as a component of a composition that includes at least 99 mole percent l-methamphetamine relative to a total methamphetamine content of the composition.

10. The amphetamine composition of Claim 1 for use of claim 1, wherein the amphetamine composition is administered in a single daily dose.

11. The amphetamine composition of Claim 1 for use of claim 1, wherein the amphetamine composition is administered in a multiple daily sub-doses.

**Patentansprüche**

1. Eine Amphetamin-Zusammensetzung zur Anwendung bei der Behandlung einer Gedächtnisstörung oder der Störung einer kognitiven Funktion, wobei die Amphetamin-Zusammensetzung aus einer Gruppe gewählt wird, die l-Amphetamin, l-Methamphetamin oder eine Kombination dieser beiden Substanzen umfasst, und wobei darüber hinaus die Gedächtnisstörung oder die Störung einer kognitiven Funktion mit multipler Sklerose assoziiert ist, und das l-Amphetamin als Inhaltsstoff einer Zusammensetzung verabreicht wird, die mindestens ca. 60 Molprozent l-Amphetamin im Verhältnis zum Gesamt-Amphetamingehalt der Zusammensetzung enthält, und das l-Methamphetamin als Inhaltsstoff einer Zusammensetzung verabreicht wird, die mindestens ca. 90 Molprozent l-Methamphetamin im Verhältnis zum Gesamt-Methamphetamingehalt der Zusammensetzung enthält.

2. Die Amphetamin-Zusammensetzung von Anspruch 1 zur Anwendung nach Anspruch 1, wobei die Gedächtnisstörung und eine Besserung der Gedächtnisleistung beim Menschen anhand des verbalen Merkfähigkeitstests RAVLT (Auditory Verbal Learning Test nach Rey) ermittelt wird.

3. Die Amphetamin-Zusammensetzung von Anspruch 1 zur Anwendung nach Anspruch 1, wobei es bei mindestens einem Element, das aus der Gruppe für Kurzzeitgedächtnis, Arbeitsgedächtnis, Langzeitgedächtnis, Gedächtniskonsolidierung, prozedurales Gedächtnis und deklaratives Gedächtnis gewählt wird, bei Behandlung der Gedächtnisstörung zu einer Besserung kommt.

4. Die Amphetamin-Zusammensetzung von Anspruch 1 zur Anwendung nach Anspruch 1, wobei entweder das l-Amphetamin in einer Dosierung von ca. 1 mg bis ca. 150 mg täglich verabreicht wird, oder das l-Methamphetamin in einer Dosierung von ca. 1 mg bis ca. 150 mg täglich verabreicht wird.

5. Die Amphetamin-Zusammensetzung von Anspruch 1 zur Anwendung nach Anspruch 1, wobei es bei mindestens einem Element, das aus der Gruppe für Aufmerksamkeit, exekutive Funktion, Reaktionszeit, Lernen, Informationsverarbeitung, Konzeptualisierung, Problemlösung und Sprachkompetenz gewählt wird, bei Behandlung der Störung der kognitiven Funktion zu einer Besserung kommt.

6. Die Amphetamin-Zusammensetzung von Anspruch 1 zur Anwendung nach Anspruch 1, wobei l-Amphetamin verabreicht wird, und wobei das l-Amphetamin als Inhaltsstoff einer Zusammensetzung verabreicht wird, in der im Verhältnis zum Gesamt-Amphetamingehalt der Zusammensetzung mindestens 75 Molprozent l-Amphetamin enthalten sind.

7. Die Amphetamin-Zusammensetzung von Anspruch 1 zur Anwendung nach Anspruch 1, wobei l-Amphetamin verabreicht wird, und wobei das l-Amphetamin als Inhaltsstoff einer Zusammensetzung verabreicht wird, in der im Verhältnis zum Gesamt-Amphetamingehalt der Zusammensetzung mindestens 99 Molprozent l-Amphetamin enthalten sind.

8. Die Amphetamin-Zusammensetzung von Anspruch 1 zur Anwendung nach Anspruch 1, wobei l-Methamphetamin

verabreicht wird, und wobei das I-Methamphetamin als Inhaltsstoff einer Zusammensetzung verabreicht wird, in der im Verhältnis zum Gesamt-Methamphetamingehalt der Zusammensetzung mindestens 95 Molprozent I-Methamphetamin enthalten sind.

9. Die Amphetamin-Zusammensetzung von Anspruch 1 zur Anwendung nach Anspruch 1, wobei I-Methamphetamin verabreicht wird, und wobei das I-ethamphetamin als Inhaltsstoff einer Zusammensetzung verabreicht wird, in der im Verhältnis zum Gesamt-Methamphetamingehalt der Zusammensetzung mindestens 99 Molprozent 1-Methamphetamin enthalten sind.

10. Die Amphetamin-Zusammensetzung von Anspruch 1 zur Anwendung nach Anspruch 1, wobei die Amphetamin-Zusammensetzung in einer einzigen Dosis pro Tag verabreicht wird.

11. Die Amphetamin-Zusammensetzung von Anspruch 1 zur Anwendung nach Anspruch 1, wobei die Amphetamin-Zusammensetzung in mehreren Teildosen pro Tag verabreicht wird.

**Revendications**

1. Composition d'amphétamine pour utilisation dans un traitement de déficience de la mémoire ou de déficience d'une fonction cognitive, dans laquelle la composition d'amphétamine est sélectionnée parmi le groupe constitué de L-amphétamine, L-métamphétamine ou de combinaisons des deux, et de plus dans laquelle : la déficience de mémoire ou la déficience de fonction cognitive est associée à de multiples scléroses et la L-amphétamine est administrée sous la forme d'un composant d'une composition qui comprend au moins environ 60 % en moles de L-amphétamine par rapport à la teneur totale en amphétamine de la composition et la L-métamphétamine est administrée sous la forme d'un composant d'une composition qui comprend au moins environ 90 % en moles de L-métamphétamine par rapport à la teneur totale en amphétamine de la composition.

2. Composition d'amphétamine selon la revendication 1 pour utilisation de la revendication 1, dans laquelle la déficience de mémoire et une amélioration de la mémoire chez l'humain sont déterminées par un test d'apprentissage auditivo-verbal de Rey

3. Composition d'amphétamine selon la revendication 1 pour utilisation de la revendication 1, dans laquelle au moins un élément, sélectionné parmi le groupe constitué d'une mémoire à court terme, d'une mémoire de travail, d'une mémoire à long terme, d'une consolidation de mémoire, d'une mémoire procédurale et d'une mémoire déclarative, est amélioré dans le traitement de la déficience de mémoire.

4. Composition d'amphétamine selon la revendication 1 pour utilisation de la revendication 1, dans laquelle la L-amphétamine est administrée à une dose comprise entre environ une dose de 1 mg et environ une dose de 150 mg par jour ; ou la L-métamphétamine est administrée à une dose comprise entre environ une dose de 1 mg et environ une dose de 150 mg par jour.

5. Composition d'amphétamine selon la revendication 1 pour utilisation de la revendication 1, dans laquelle au moins un élément, sélectionné parmi le groupe constitué de l'attention, d'une fonction exécutive, d'un temps de réaction, d'un apprentissage, d'un traitement d'informations, d'une conceptualisation, d'une résolution de problème et d'une facilité de parole, est amélioré dans le traitement de la déficience.

6. Composition d'amphétamine selon la revendication 1 pour utilisation de la revendication 1, dans laquelle la L-amphétamine est administrée, et dans laquelle la L-amphétamine est administrée sous la forme d'un composant d'une composition qui comprend au moins 75 % en môles de L-amphétamine par rapport à la teneur totale en amphétamine de la composition.

7. Composition d'amphétamine selon la revendication 1 pour utilisation de la revendication 1, dans laquelle la L-amphétamine est administrée, et dans laquelle la L-amphétamine est administrée sous la forme d'un composant d'une composition qui comprend au moins 99 % en moles de L-amphétamine par rapport à la teneur totale en amphétamine de la composition.

8. Composition d'amphétamine selon la revendication 1 pour utilisation de la revendication 1, dans laquelle la L-métamphétamine est administrée, et dans laquelle la L-métamphétamine est administrée sous la forme d'un composant d'une composition qui comprend au moins 95 % en moles de L-métamphétamine par rapport à la teneur totale en métamphétamine de la composition.

**9.** Composition d'amphétamine selon la revendication 1 pour utilisation de la revendication 1, dans laquelle la L-métamphétamine est administrée, et dans laquelle la L-métamphétamine est administrée sous la forme d'un composant d'une composition qui comprend au moins 99 % en moles de L-métamphétamine par rapport à la teneur totale en métamphétamine de la composition

**10.** Composition d'amphétamine selon la revendication 1 pour utilisation de la revendication 1, dans laquelle la composition d'amphétamine est administrée en une dose quotidienne unique.

**11.** Composition d'amphétamine selon la revendication 1 pour utilisation de la revendication 1, dans laquelle la composition d'amphétamine est administrée en multiples sous-doses quotidiennes.

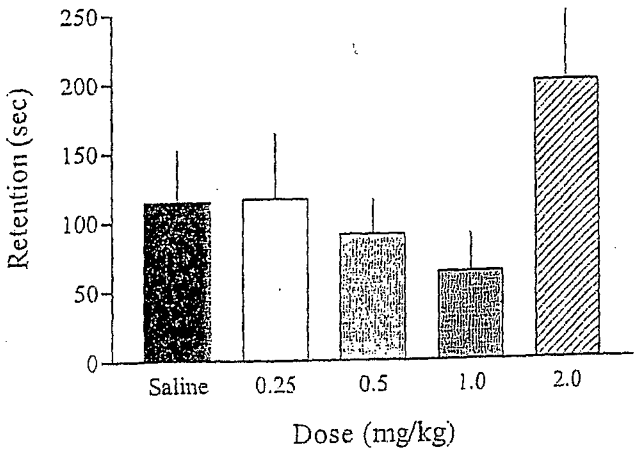
Retention (sec)
250
200
150
100
50
0
Saline
0.25
0.5
1.0
2.0
Dose (mg/kg)

FIG. 1

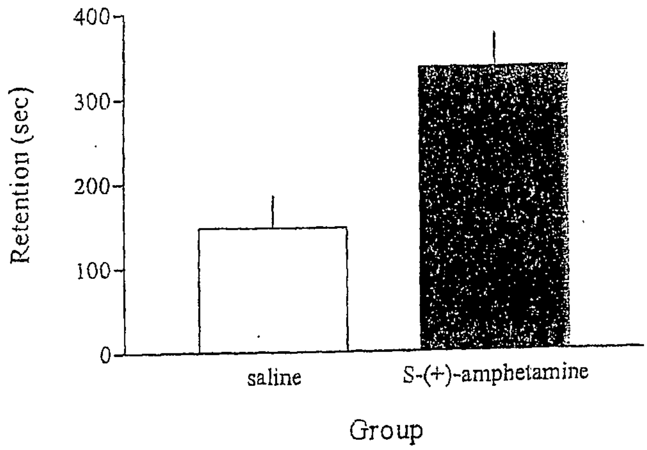
Retention (sec)
400
300
200
100
0
saline
S-(+)-amphetamine
Group

FIG. 2

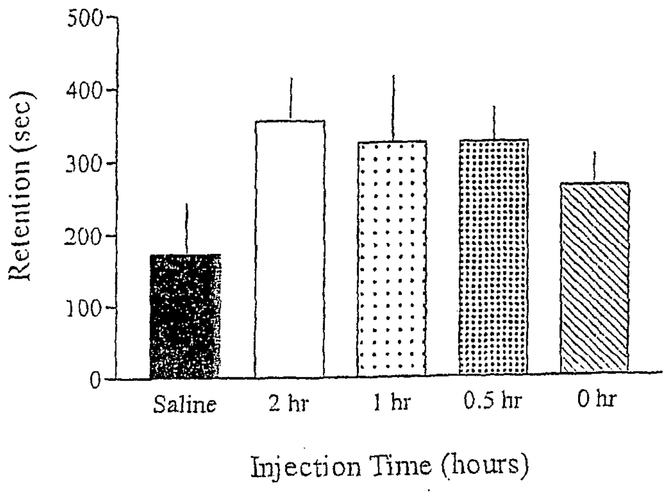
500
400
300
200
100
0
Retention (sec)
Saline
2 hr
1 hr
0.5 hr
0 hr
Injection Time (hours)

FIG. 3

| One-way analysis of variance | |
|---|---|
| P value | 0.0114 |
| P value summary | * |
| Are means signif. different? (P < 0.05) | Yes |
| Number of groups | 5 |
| F | 3.688 |
| R squared | 0.2554 |

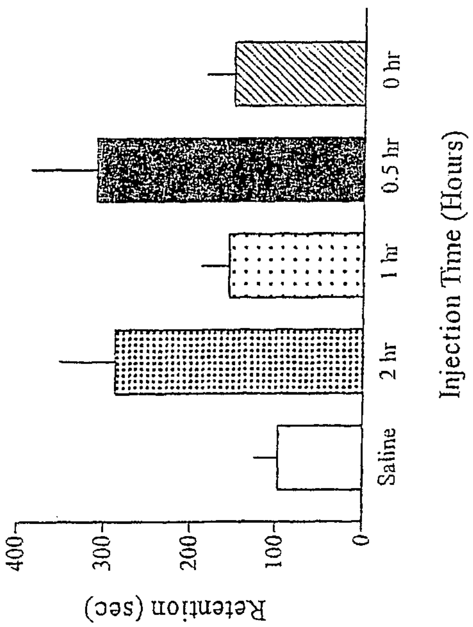


FIG. 4

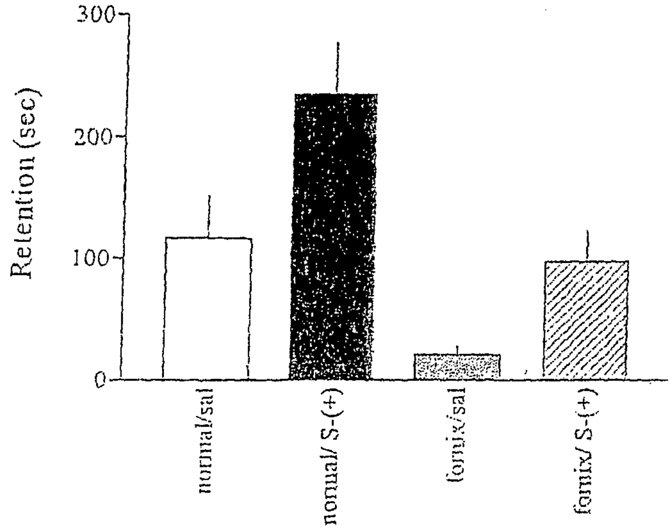
300
200
100
0
Retention (sec)
normal/sal
normal/ S-(+)
fornix/sal
fornix/ S-(+)

FIG. 5

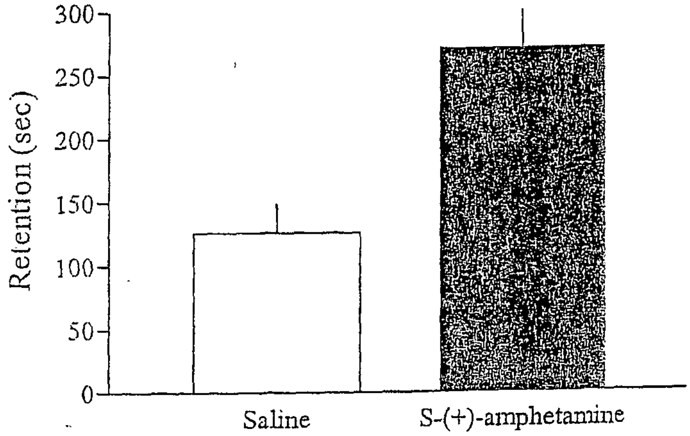


FIG. 6

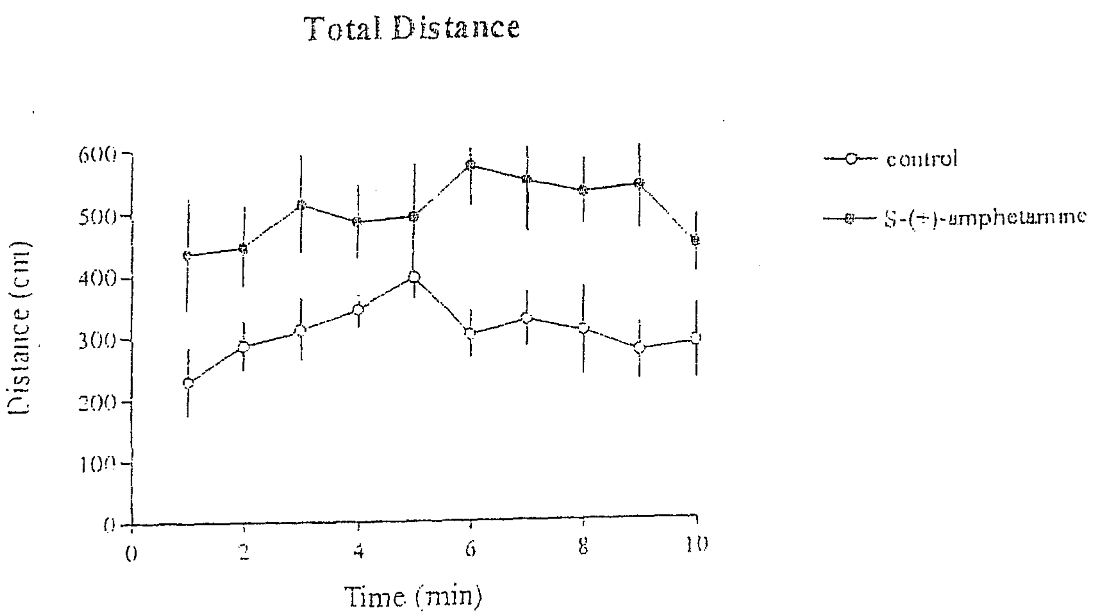
Total Distance
Distance (cm)
600
500
400
300
200
100
0
0
2
4
6
8
10
Time (min)
control
S-(+)-amphetamine

FIG. 7A

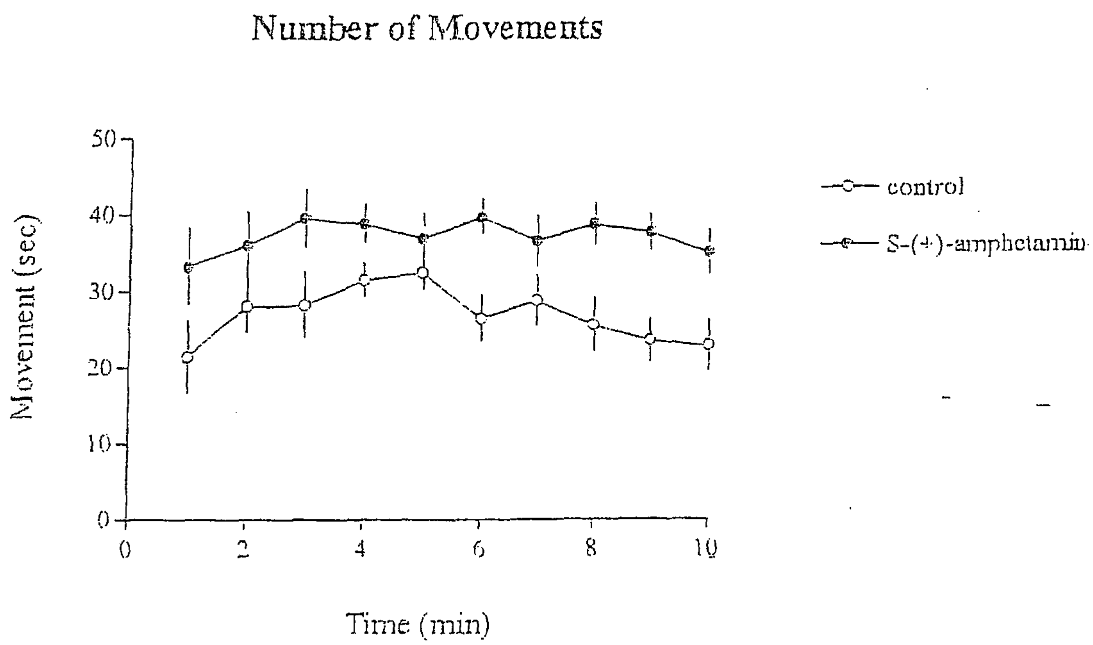
Number of Movements
Movement (sec)
50
40
30
20
10
0
0
2
4
6
8
10
Time (min)
control
S-(+)-amphetamin

FIG. 7B

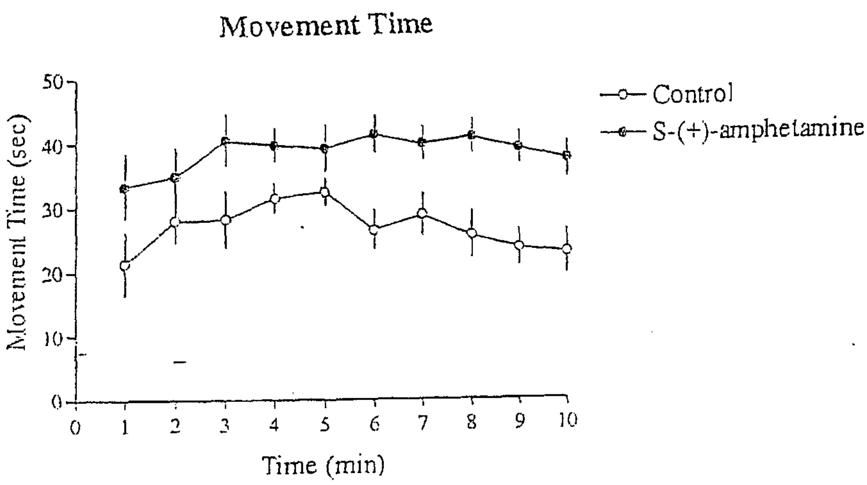
Movement Time
Movement Time (sec)
50
40
30
20
10
0
0
1
2
3
4
5
6
7
8
9
10
Time (min)
Control
S-(+)-amphetamine

FIG. 7C

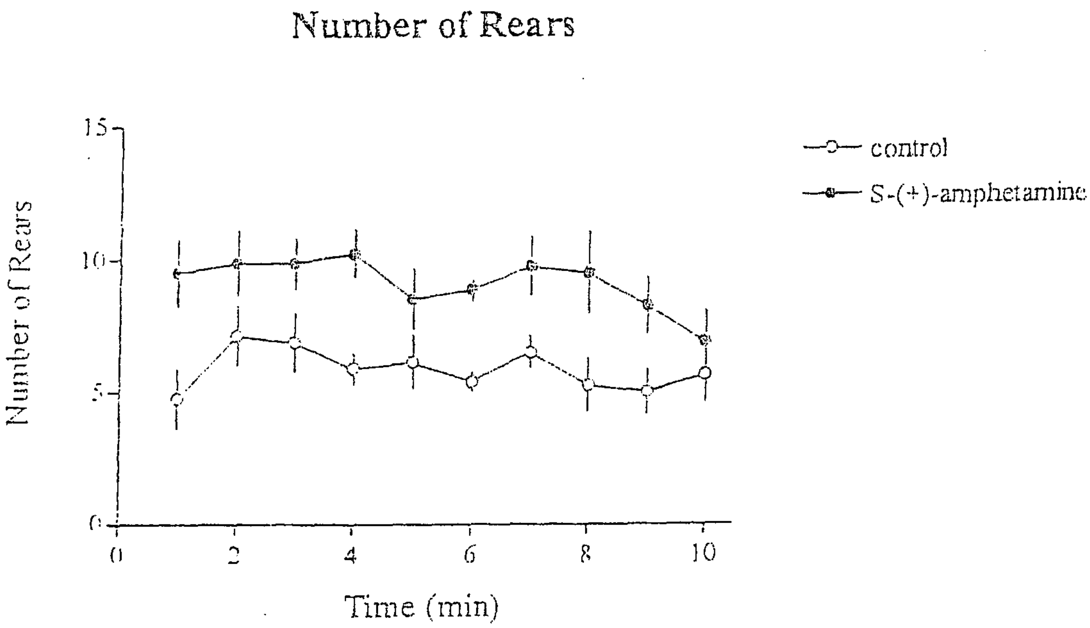
Number of Rears
Number of Rears
15
10
5
0
0
2
4
6
8
10
Time (min)
control
S-(+)-amphetamine

FIG. 7D

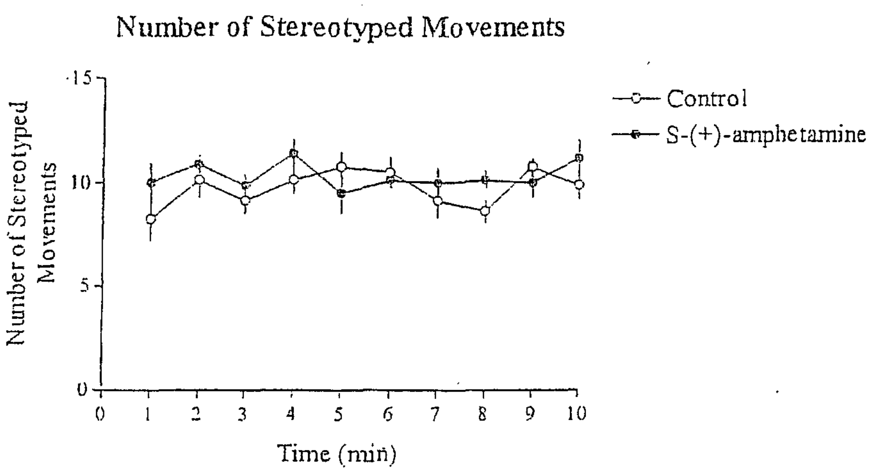
Number of Stereotyped Movements
Number of Stereotyped Movements
15
10
5
0
0 1 2 3 4 5 6 7 8 9 10
Time (min)
Control
S-(+)-amphetamine

FIG. 7E

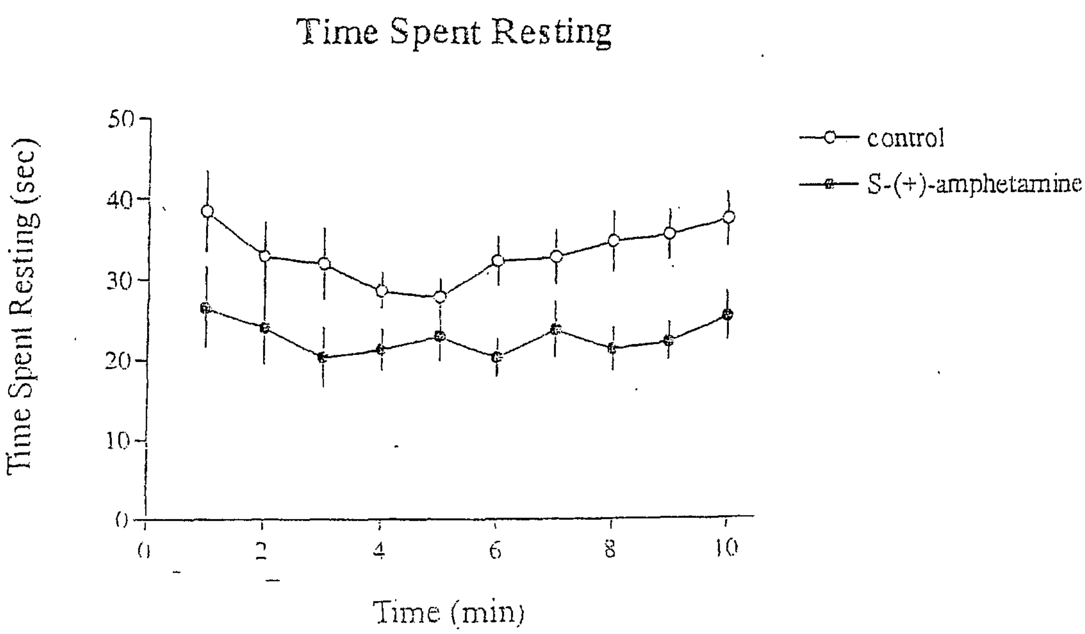
Time Spent Resting
Time Spent Resting (sec)
50
40
30
20
10
0
0
2
4
6
8
10
Time (min)
control
S-(+)-amphetamine

FIG. 7F

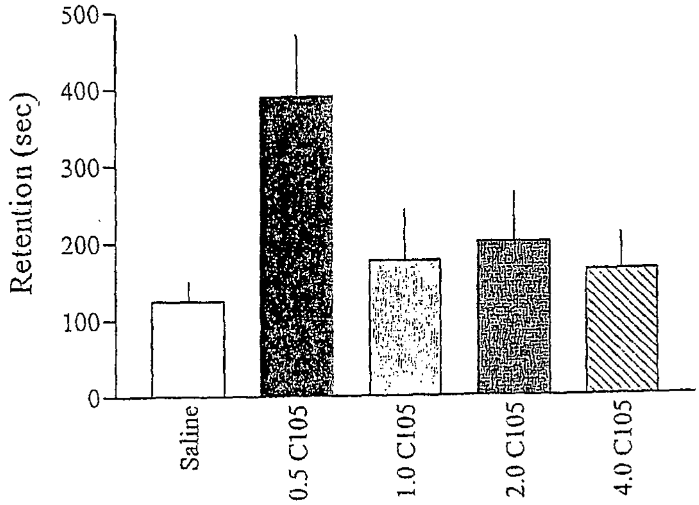
Retention (sec)
500
400
300
200
100
0
Saline
0.5 C105
1.0 C105
2.0 C105
4.0 C105

FIG. 8

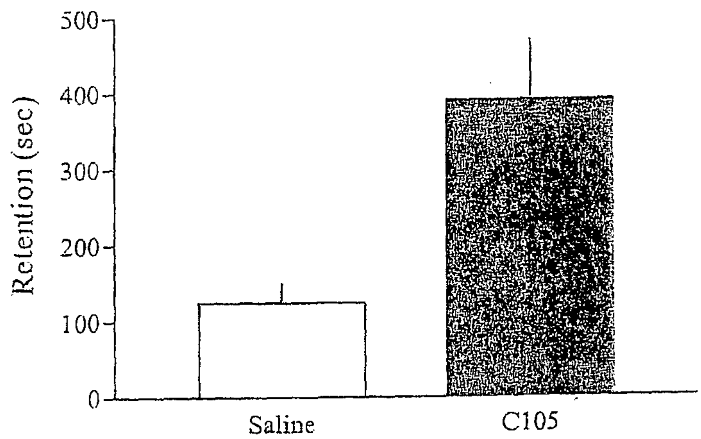
Retention (sec)
500
400
300
200
100
0
Saline
C105

FIG. 9

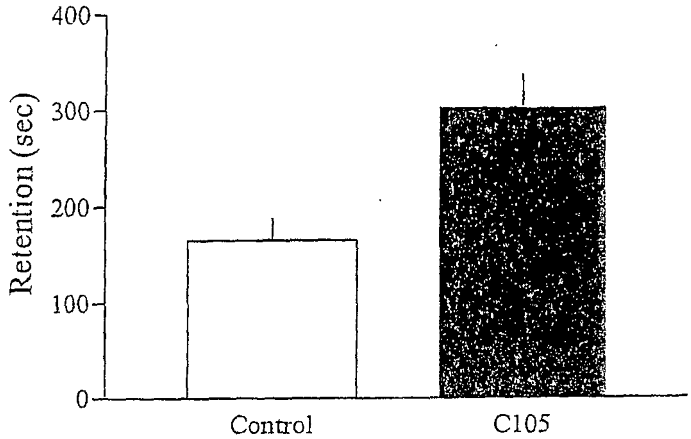


FIG. 10

FIG. 11

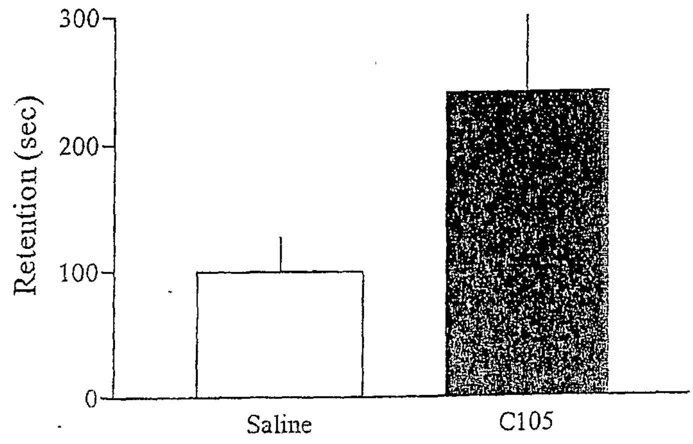
300
200
100
0
Retention (sec)
Saline
C105

FIG. 12

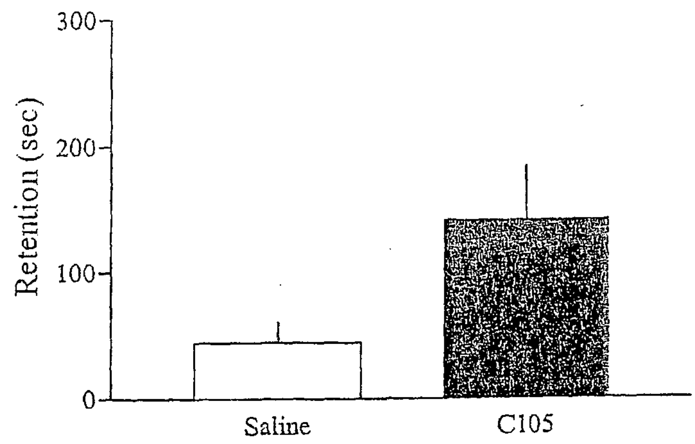
300
200
100
0
Retention (sec)
Saline
C105

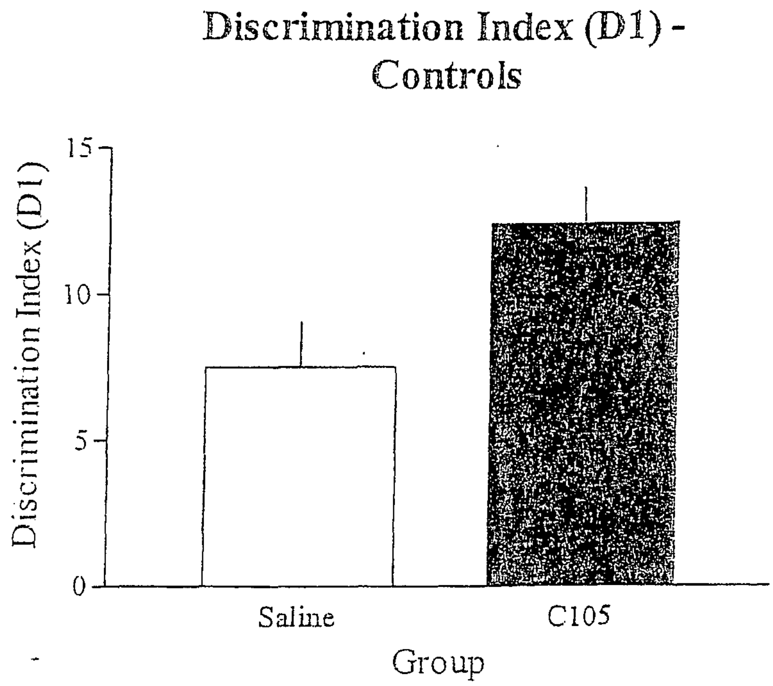
Discrimination Index (D1) -
Controls
15
10
5
0
Discrimination Index (D1)
Saline
C105
Group

FIG. 13A

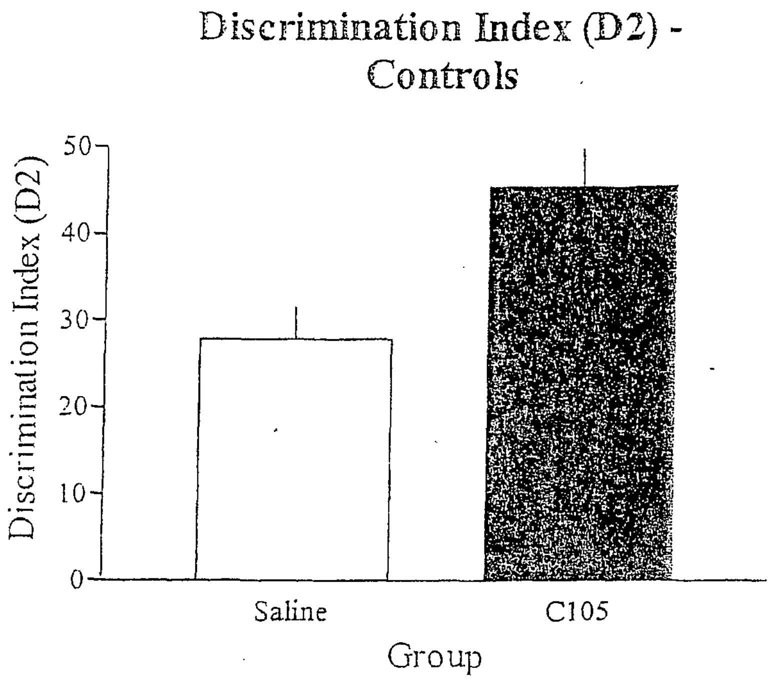
Discrimination Index (D2) -
Controls
Discrimination Index (D2)
50
40
30
20
10
0
Saline
C105
Group

FIG. 13B

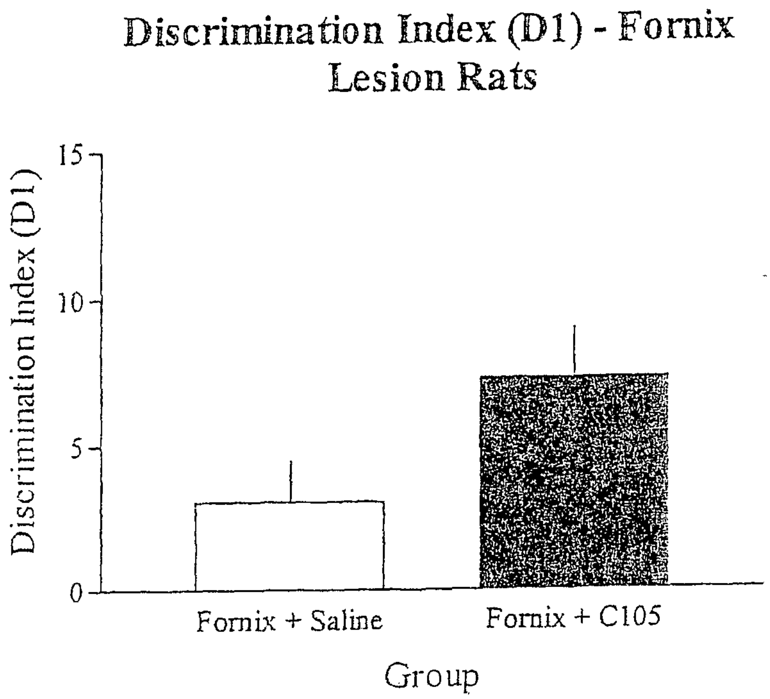
Discrimination Index (D1) - Fornix Lesion Rats
Discrimination Index (D1)
15
10
5
0
Fornix + Saline
Fornix + C105
Group

FIG. 13C

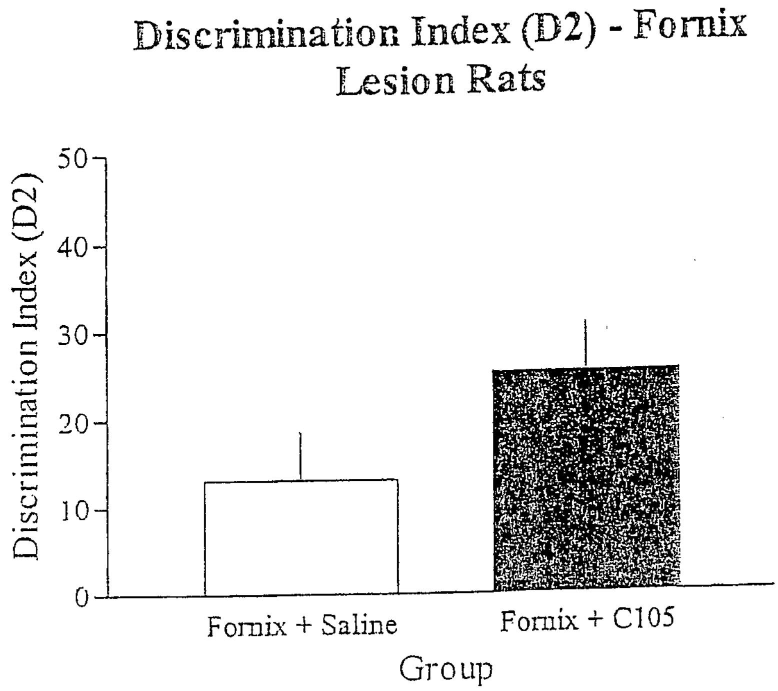
Discrimination Index (D2) - Fornix
Lesion Rats
Discrimination Index (D2)
50
40
30
20
10
0
Fornix + Saline
Fornix + C105
Group

FIG. 13D

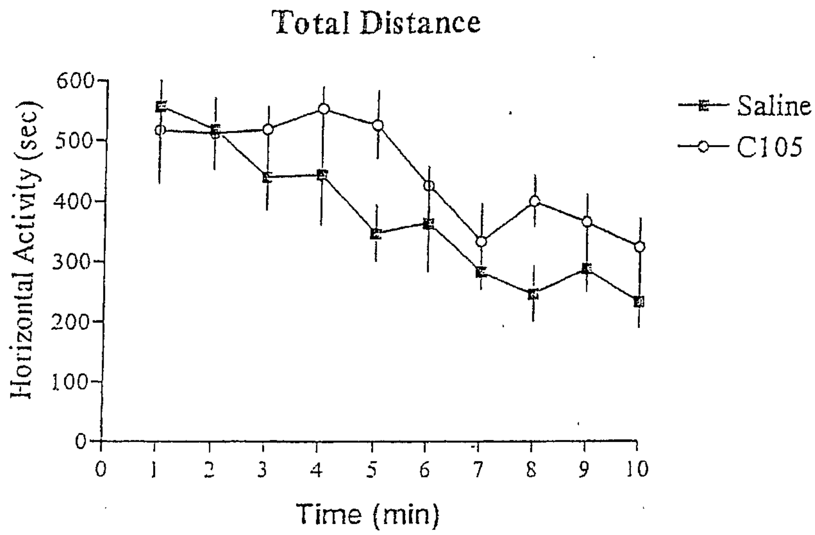
Total Distance
Horizontal Activity (sec)
600
500
400
300
200
100
0
0 1 2 3 4 5 6 7 8 9 10
Time (min)
Saline
C105

FIG. 14A

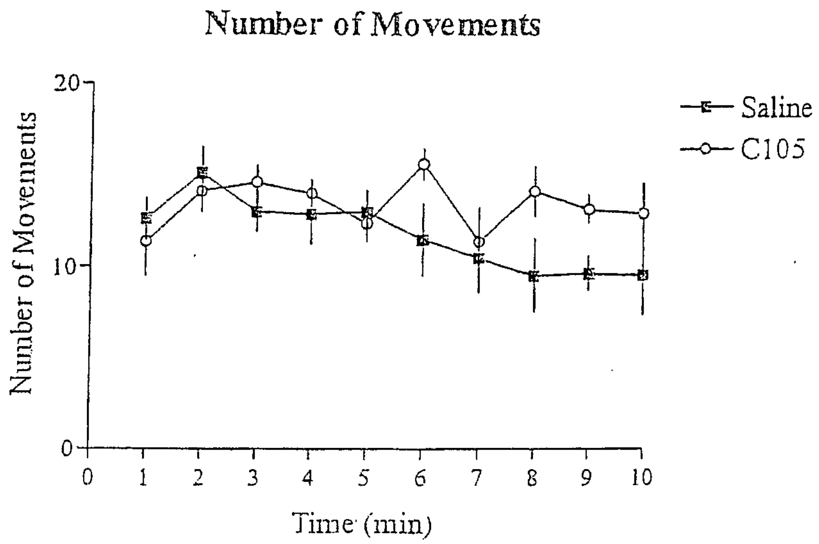
Number of Movements
Number of Movements
20
10
0
0
1
2
3
4
5
6
7
8
9
10
Time (min)
Saline
C105

FIG. 14B

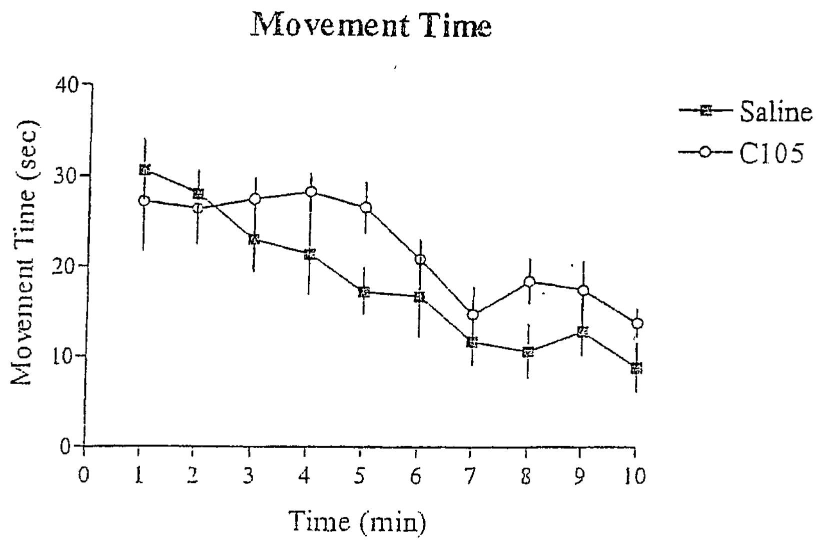
Movement Time
Movement Time (sec)
40
30
20
10
0
0
1
2
3
4
5
6
7
8
9
10
Time (min)
Saline
C105

FIG. 14C

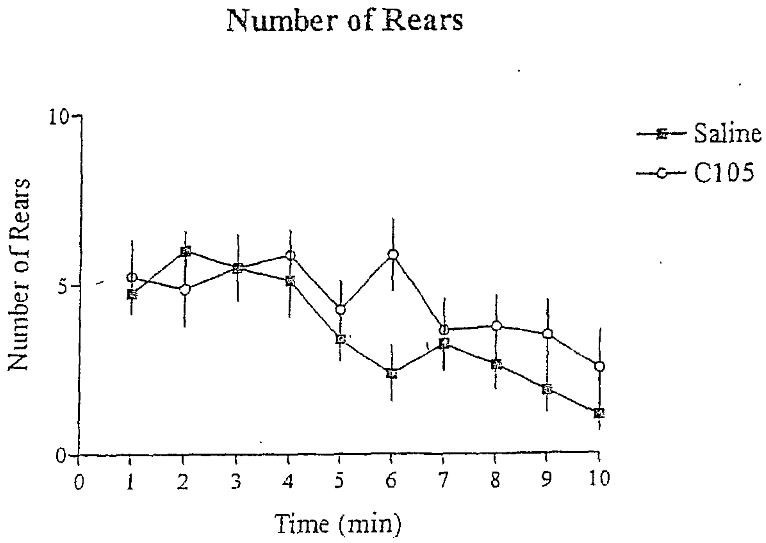
Number of Rears
10
5
0
Number of Rears
Saline
C105
0
1
2
3
4
5
6
7
8
9
10
Time (min)

FIG. 14D

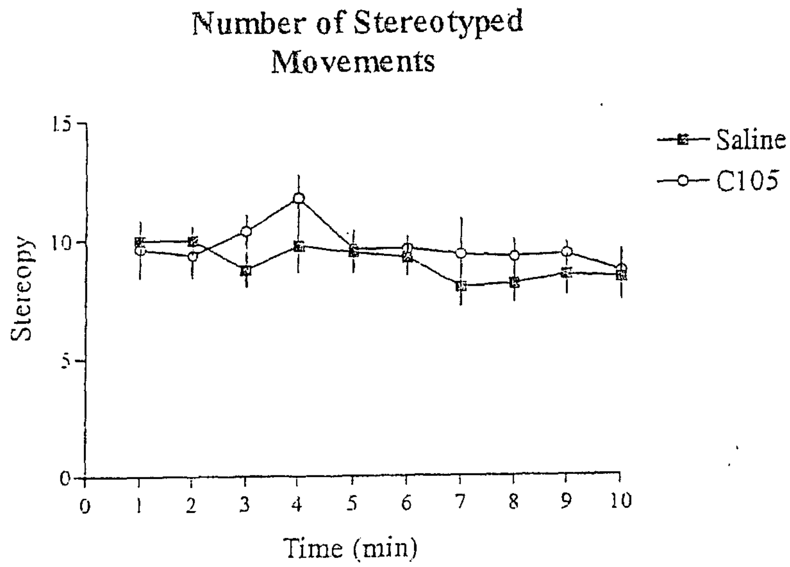
Number of Stereotyped Movements
Saline
C105
Stereopy
15
10
5
0
0
1
2
3
4
5
6
7
8
9
10
Time (min)

FIG. 14E

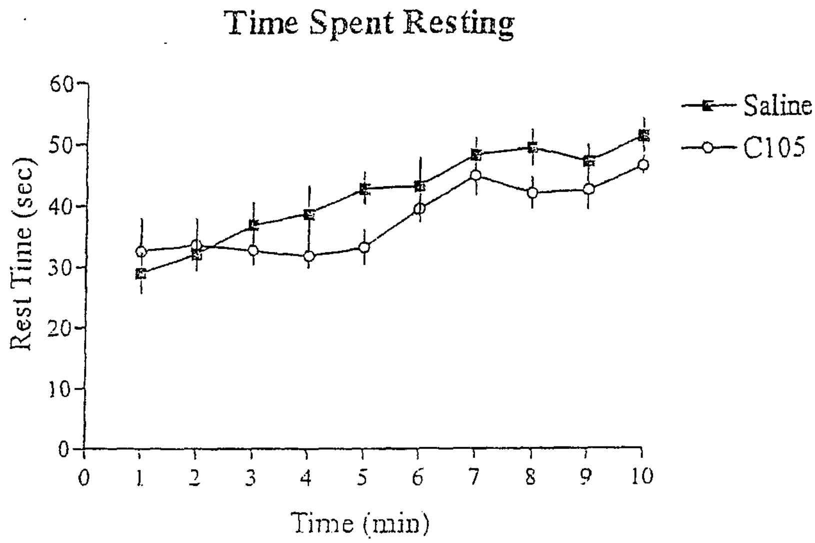
Time Spent Resting
Rest Time (sec)
60
50
40
30
20
10
0
0 1 2 3 4 5 6 7 8 9 10
Time (min)
Saline
C105

FIG. 14F

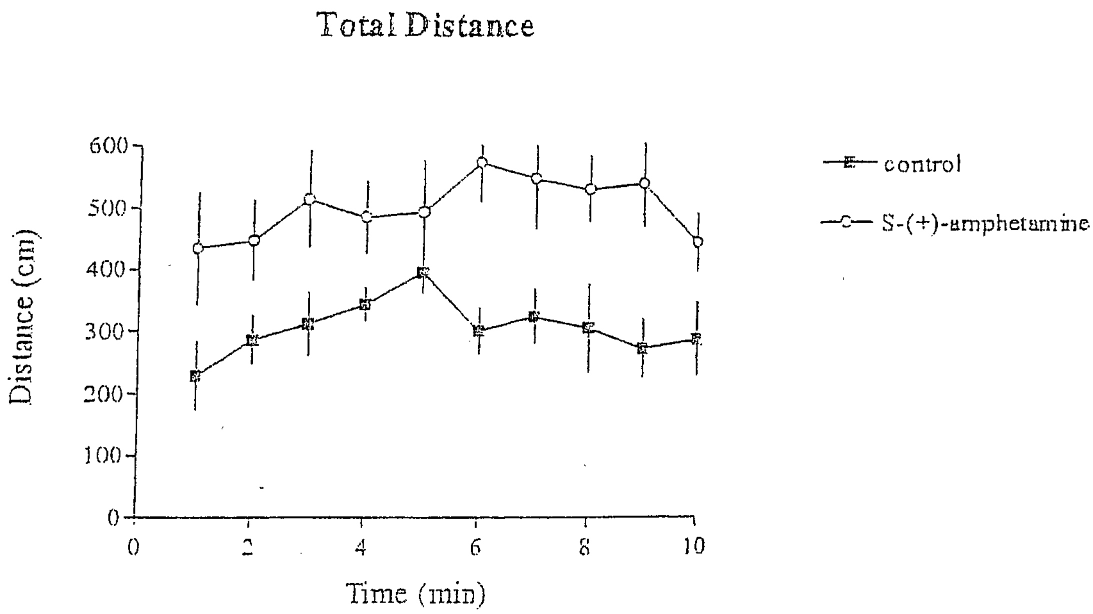
Total Distance
Distance (cm)
600
500
400
300
200
100
0
0
2
4
6
8
10
Time (min)
control
S-(+)-amphetamine

FIG. 15A

Number of Movements

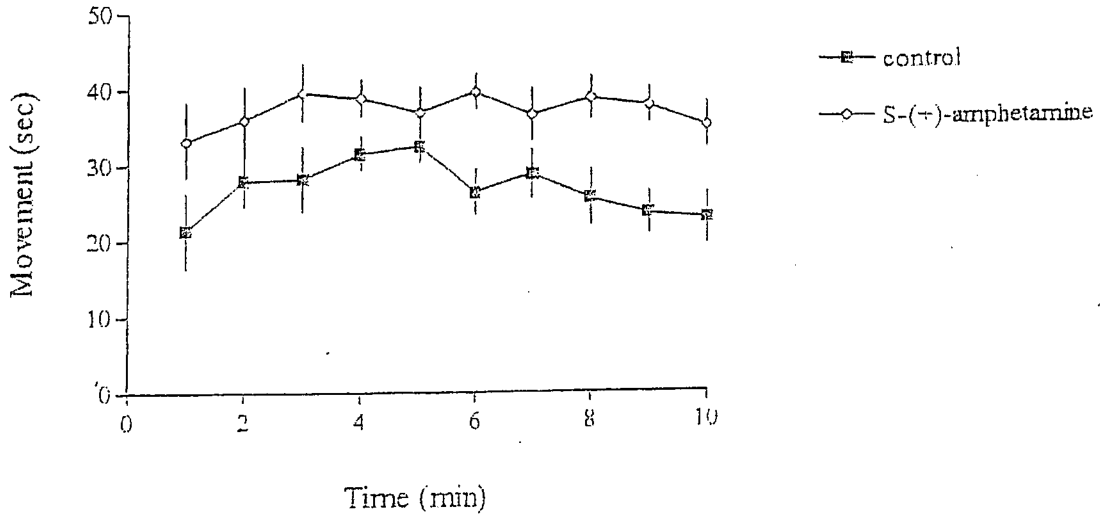


FIG. 15B

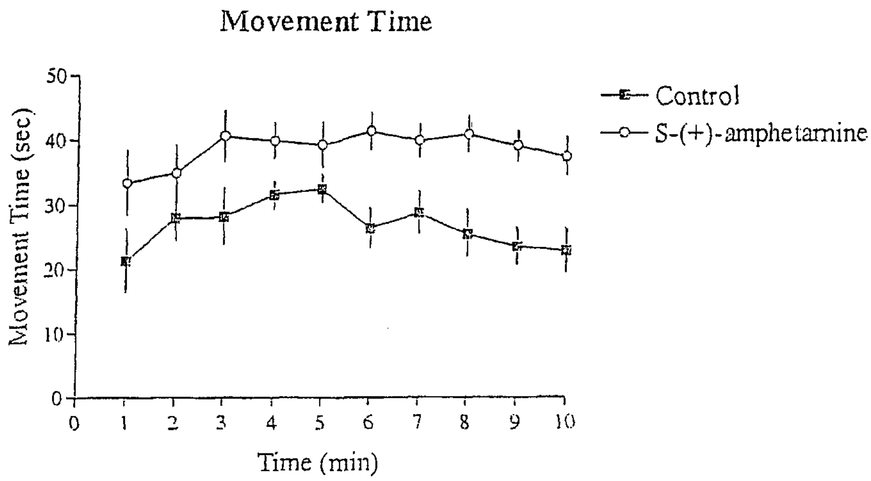
Movement Time
Movement Time (sec)
50
40
30
20
10
0
0 1 2 3 4 5 6 7 8 9 10
Time (min)
Control
S-(+)-amphetamine

FIG. 15C

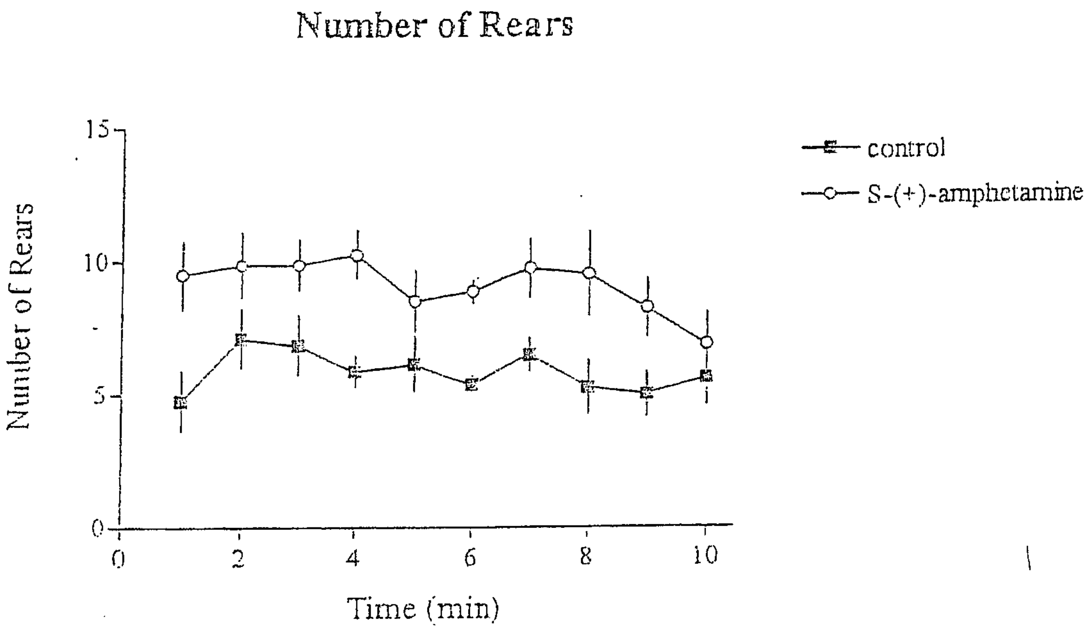
Number of Rears
Number of Rears
15
10
5
0
0
2
4
6
8
10
Time (min)
control
S-(+)-amphetamine

FIG. 15D

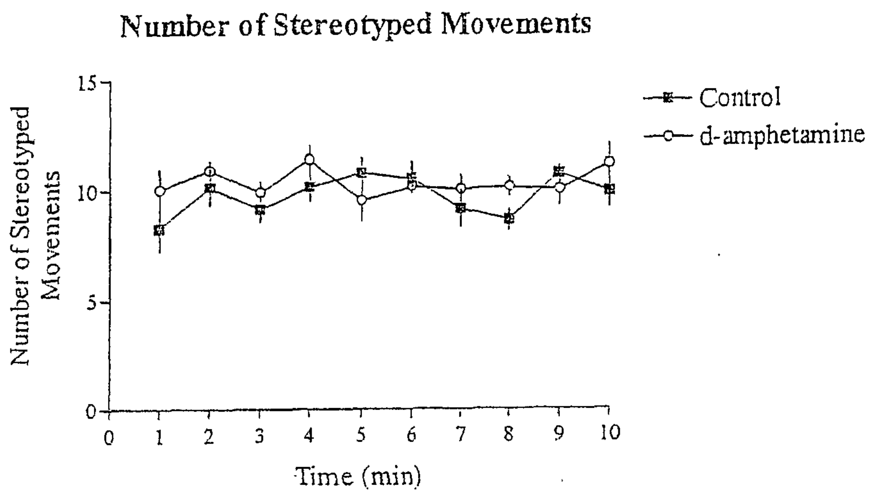
Number of Stereotyped Movements
Number of Stereotyped Movements
15
10
5
0
0 1 2 3 4 5 6 7 8 9 10
Time (min)
Control
d-amphetamine

FIG. 15E

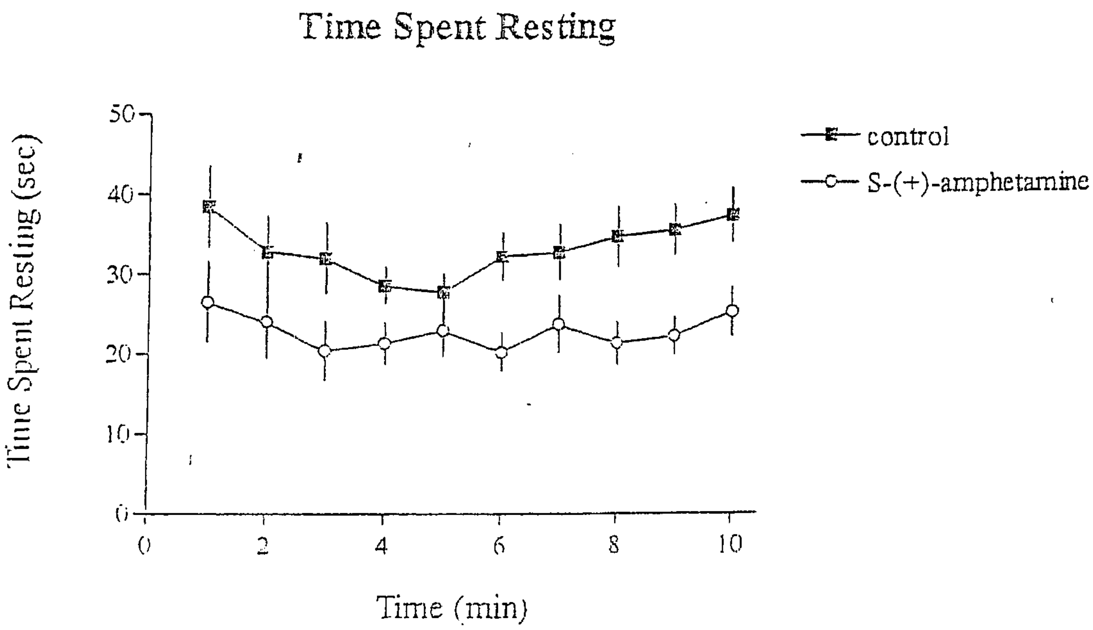
Time Spent Resting
Time Spent Resting (sec)
50
40
30
20
10
0
0
2
4
6
8
10
Time (min)
control
S-(+)-amphetamine

FIG. 15F

FIG. 16

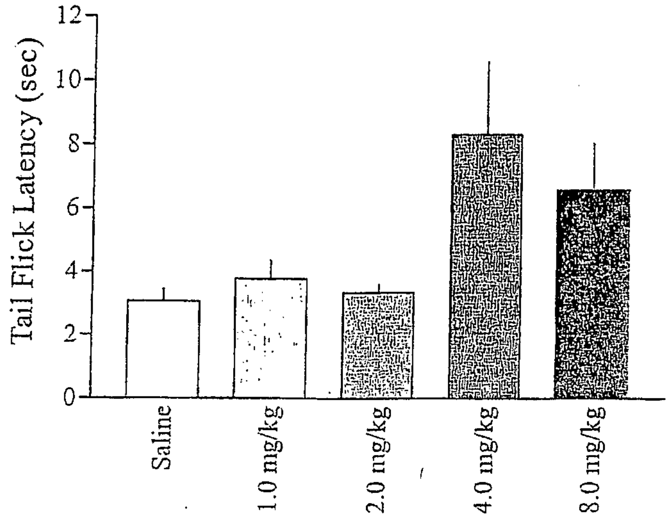
Tail Flick Latency (sec)
12
10
8
6
4
2
0
Saline
1.0 mg/kg
2.0 mg/kg
4.0 mg/kg
8.0 mg/kg

FIG. 17

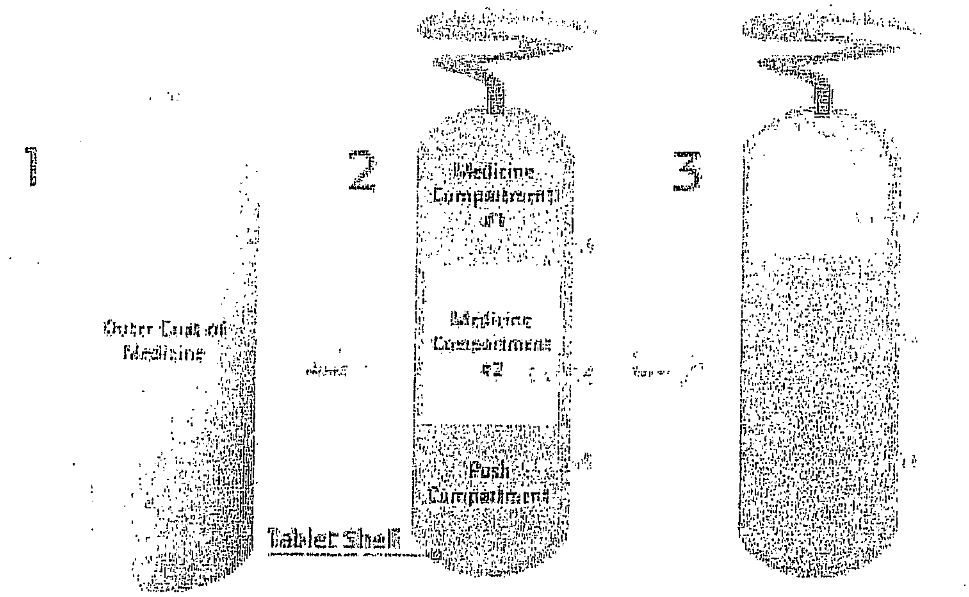
1
2
3
Outer Coat of Medicine
Medicine Compartment #1
Medicine Compartment #2
Push Compartment
Tablet Shell

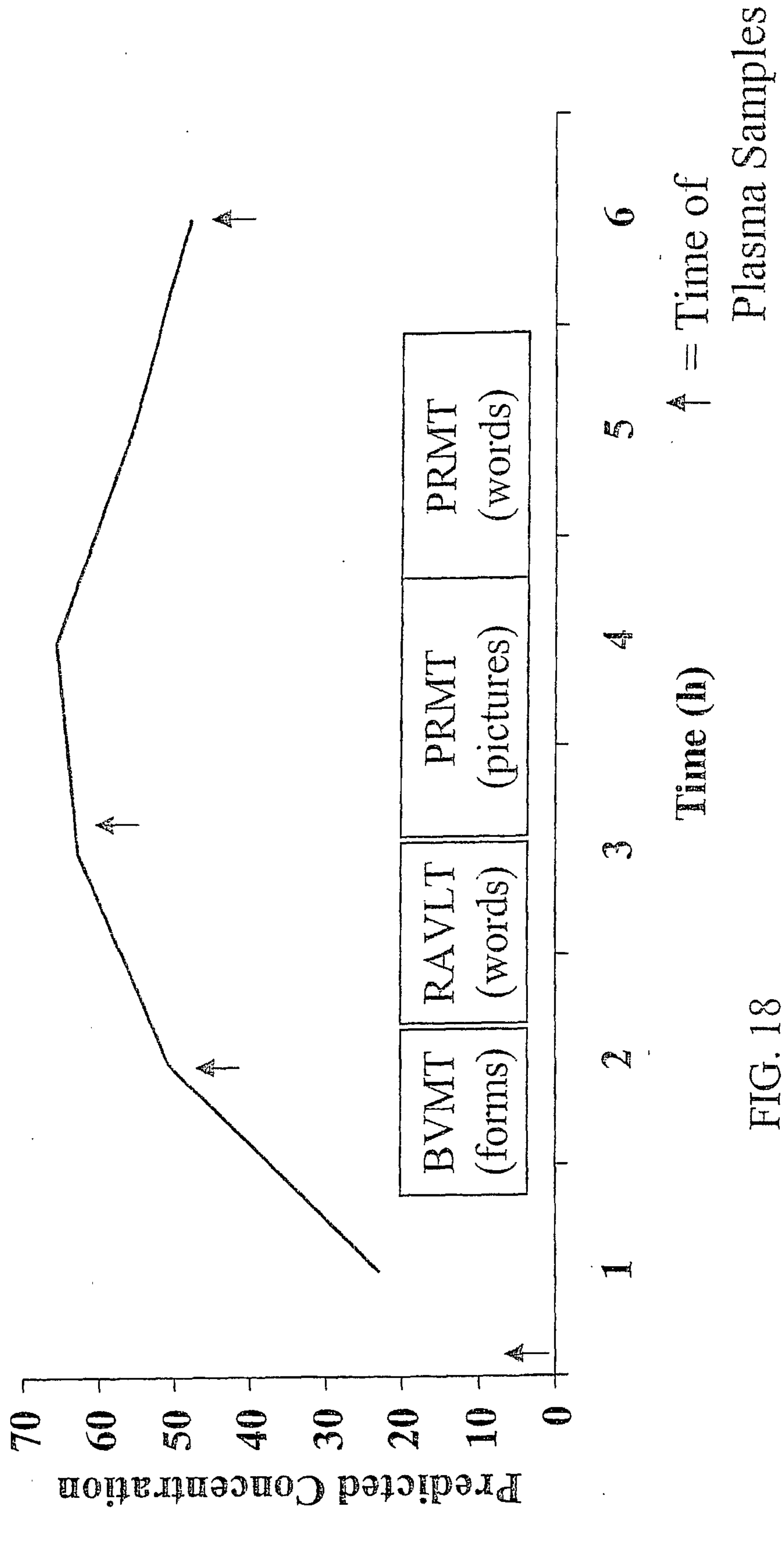

Predicted Concentration
0
10
20
30
40
50
60
70
1
2
3
4
5
6
Time (h)
BVMT (forms)
RAVLT (words)
PRMT (pictures)
PRMT (words)
= Time of Plasma Samples


FIG. 18

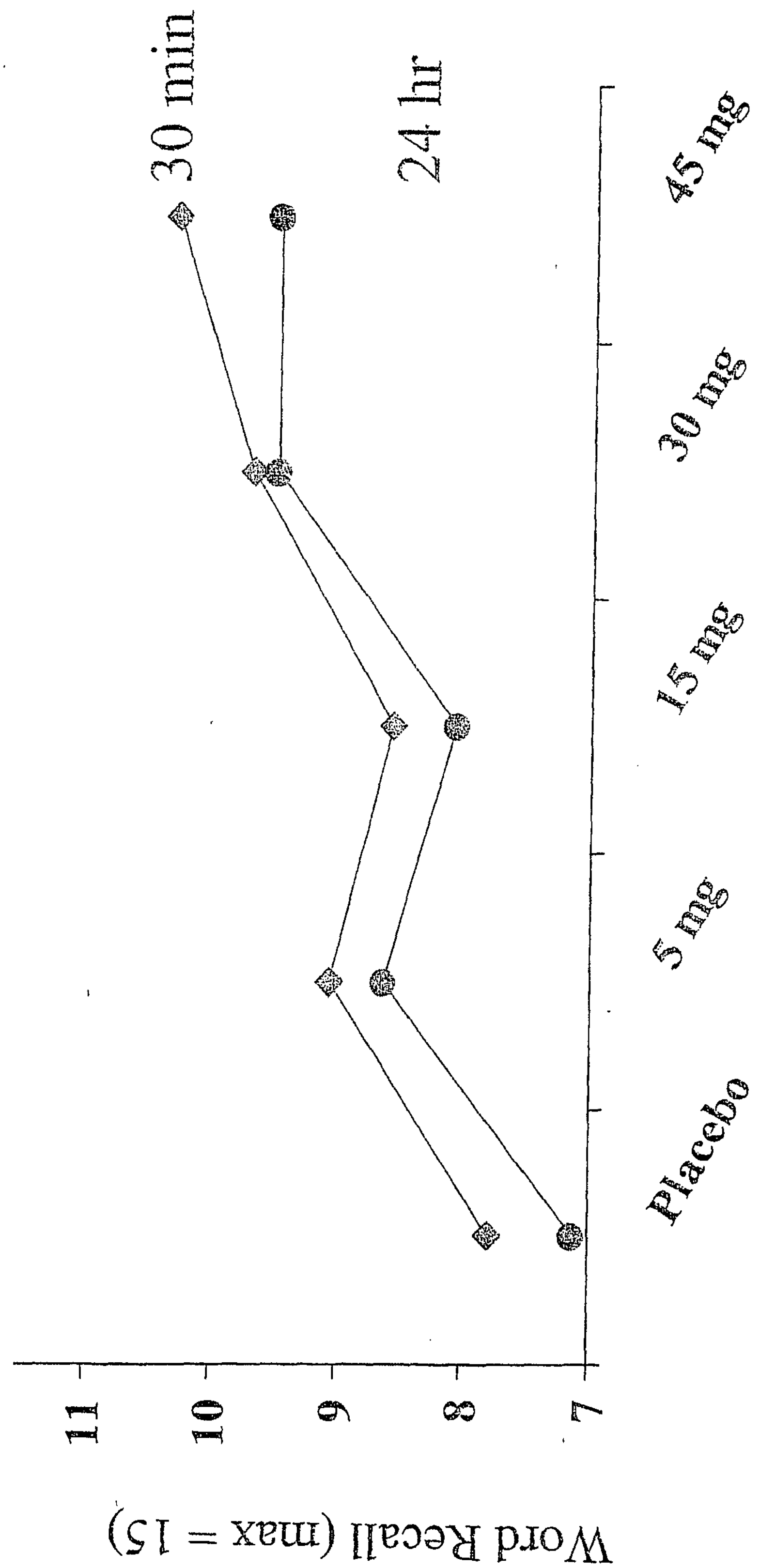
30 min
24 hr
Word Recall (max = 15)
11
10
9
8
7
Placebo
5 mg
15 mg
30 mg
45 mg

FIG. 19

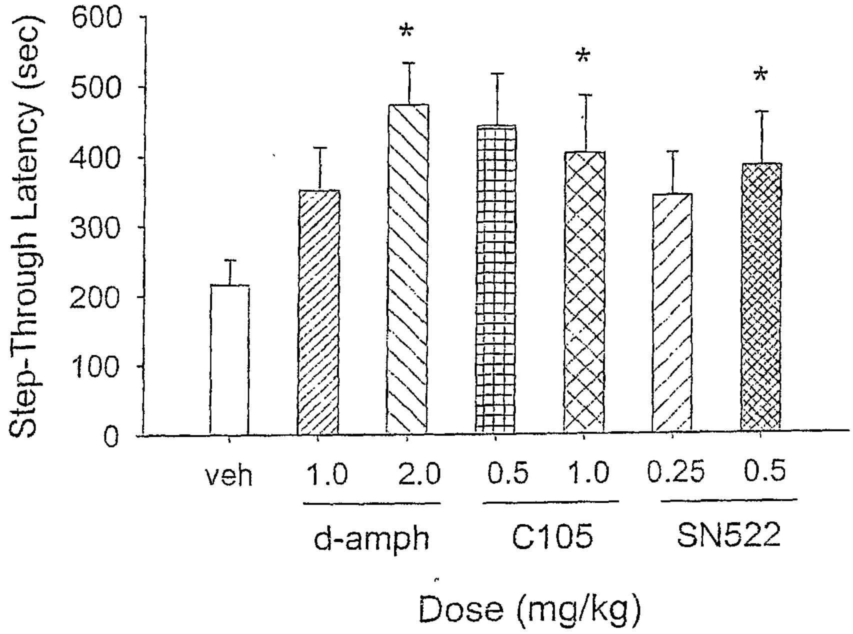
Step-Through Latency (sec)
600
500
400
300
200
100
0
*
*
*
veh
1.0
2.0
0.5
1.0
0.25
0.5
d-amph
C105
SN522
Dose (mg/kg)

FIG. 20

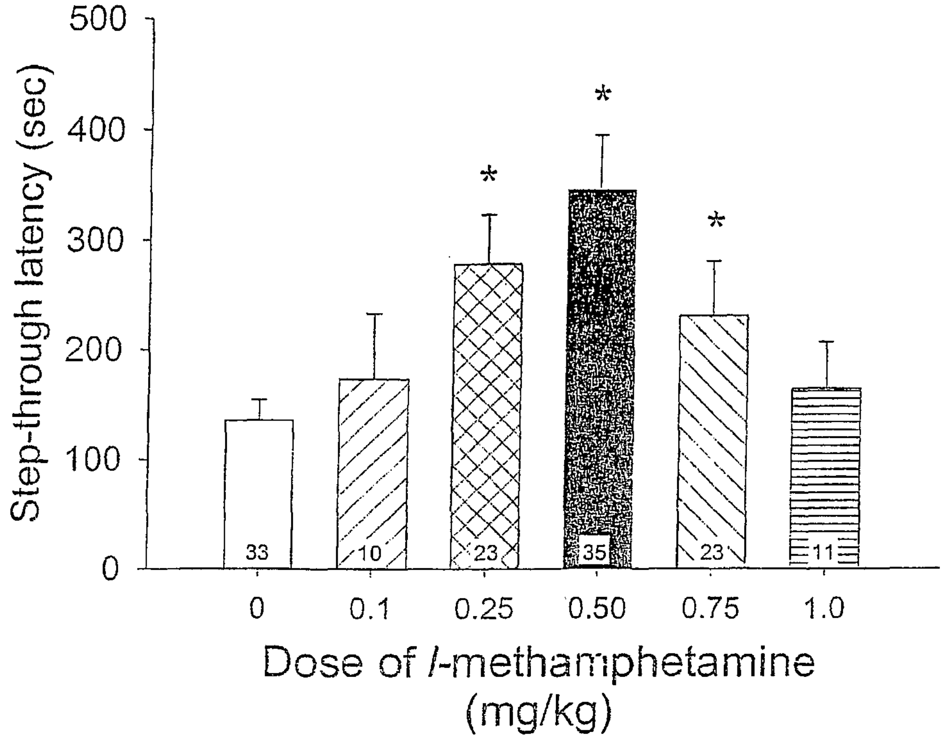
500
400
300
200
100
0
Step-through latency (sec)
*
*
*
33
10
23
35
23
11
0
0.1
0.25
0.50
0.75
1.0
Dose of l-methamphetamine
(mg/kg)

FIG. 21

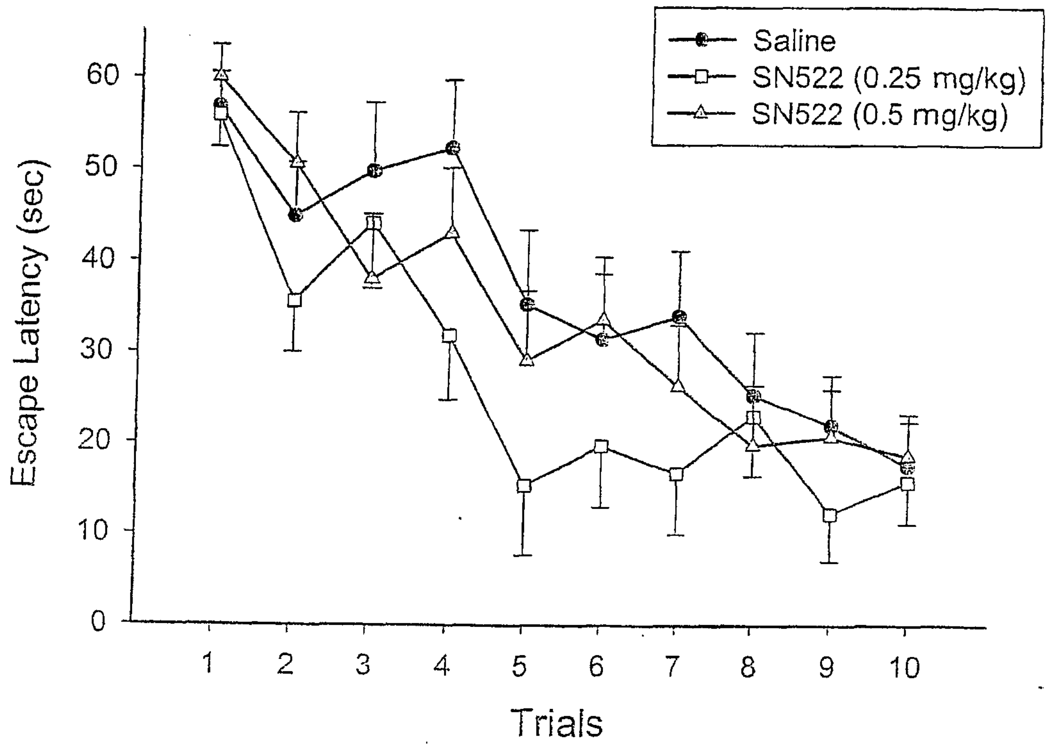
Saline
SN522 (0.25 mg/kg)
SN522 (0.5 mg/kg)
Escape Latency (sec)
0
10
20
30
40
50
60
1
2
3
4
5
6
7
8
9
10
Trials

FIG. 22

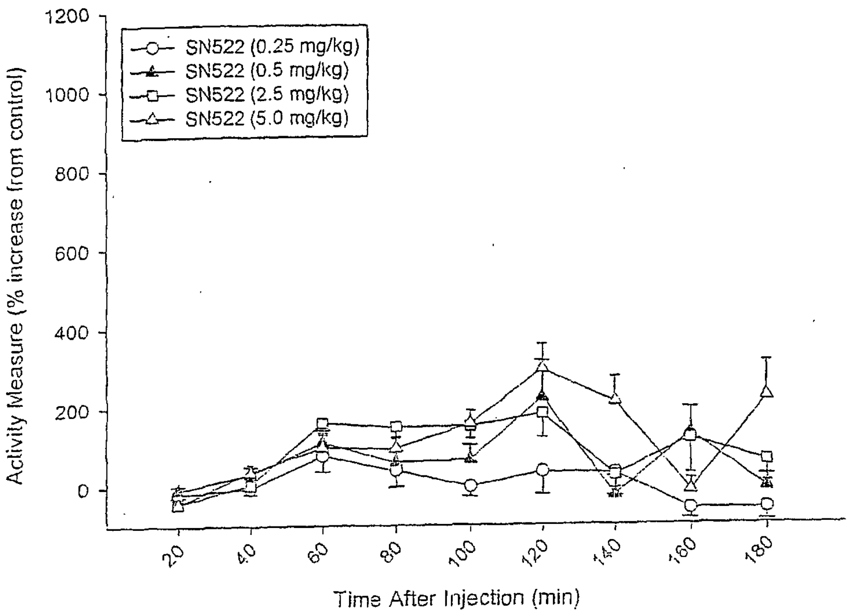

SN522 (0.25 mg/kg)
SN522 (0.5 mg/kg)
SN522 (2.5 mg/kg)
SN522 (5.0 mg/kg)
Activity Measure (% increase from control)
1200
1000
800
600
400
200
0
20
40
60
80
100
120
140
160
180
Time After Injection (min)


FIG. 23

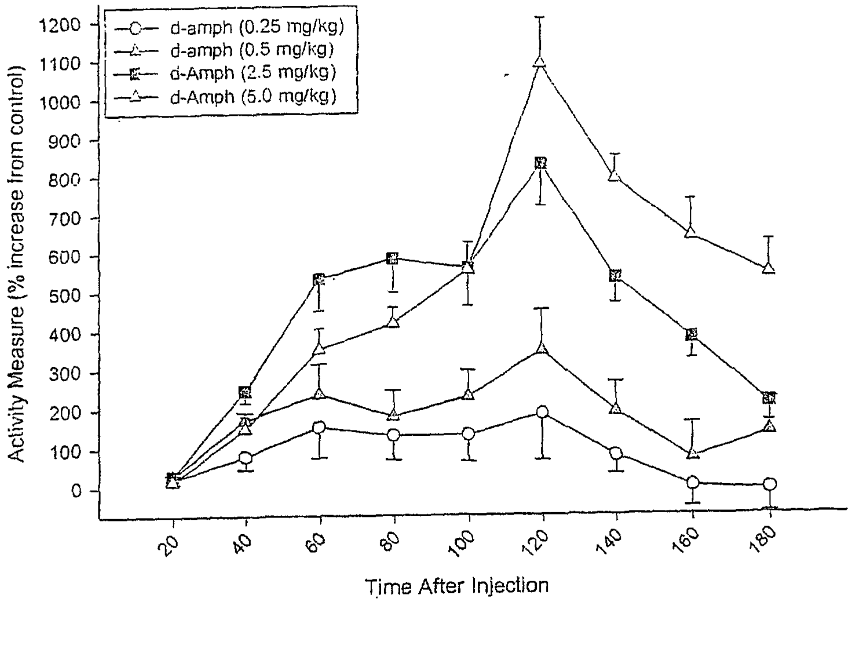

d-amph (0.25 mg/kg)
d-amph (0.5 mg/kg)
d-Amph (2.5 mg/kg)
d-Amph (5.0 mg/kg)
Activity Measure (% increase from control)
1200
1100
1000
900
800
700
600
500
400
300
200
100
0
20
40
60
80
100
120
140
160
180
Time After Injection


FIG. 24

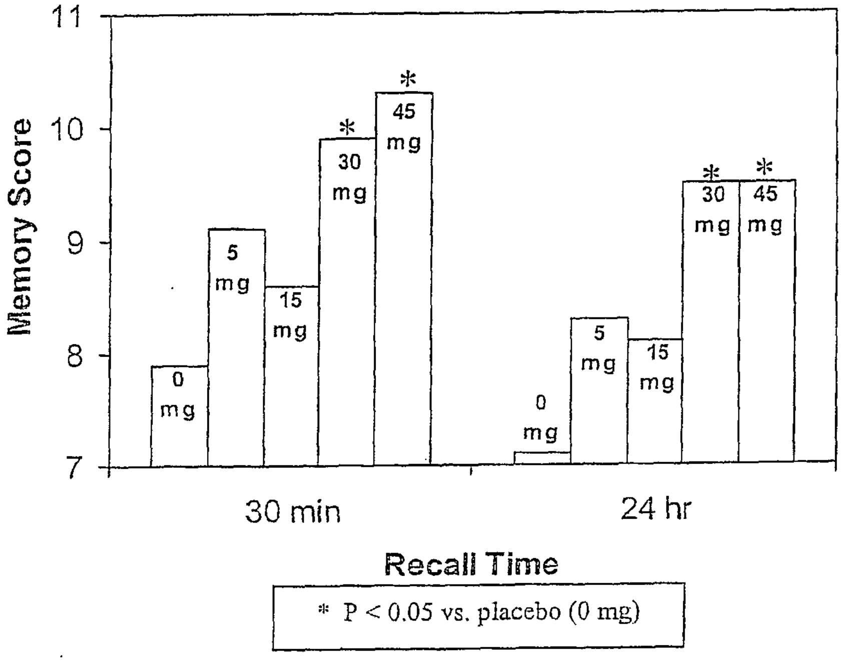
11
10
9
8
7
Memory Score
0 mg
5 mg
15 mg
30 mg
45 mg
*
30 min
24 hr
Recall Time
* P < 0.05 vs. placebo (0 mg)

FIG. 25

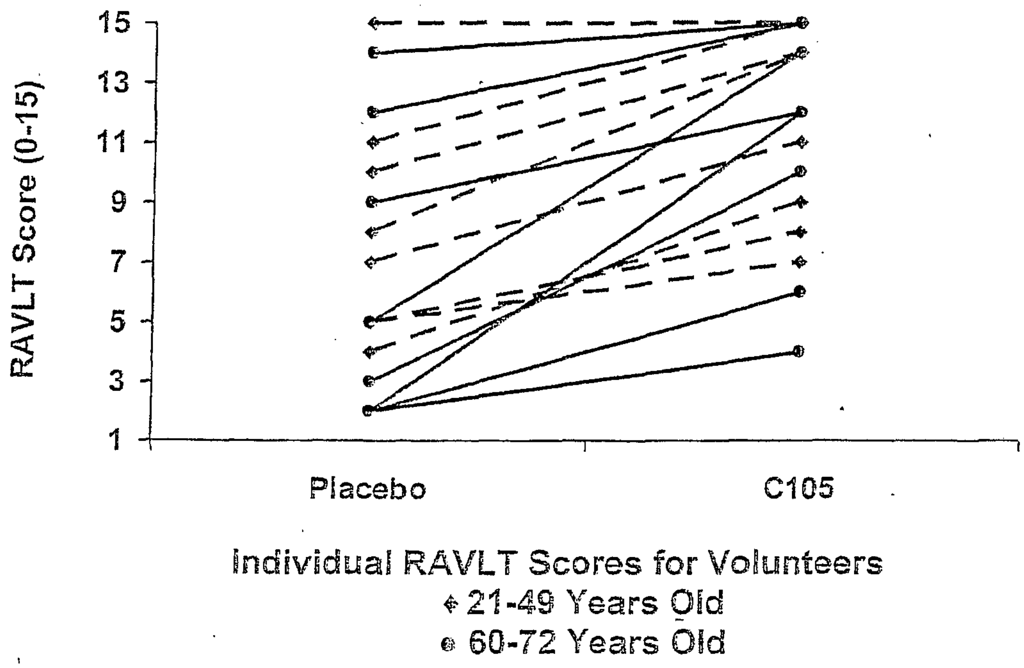
RAVLT Score (0-15)
15
13
11
9
7
5
3
1
Placebo
C105
Individual RAVLT Scores for Volunteers
21-49 Years Old
60-72 Years Old

FIG. 26

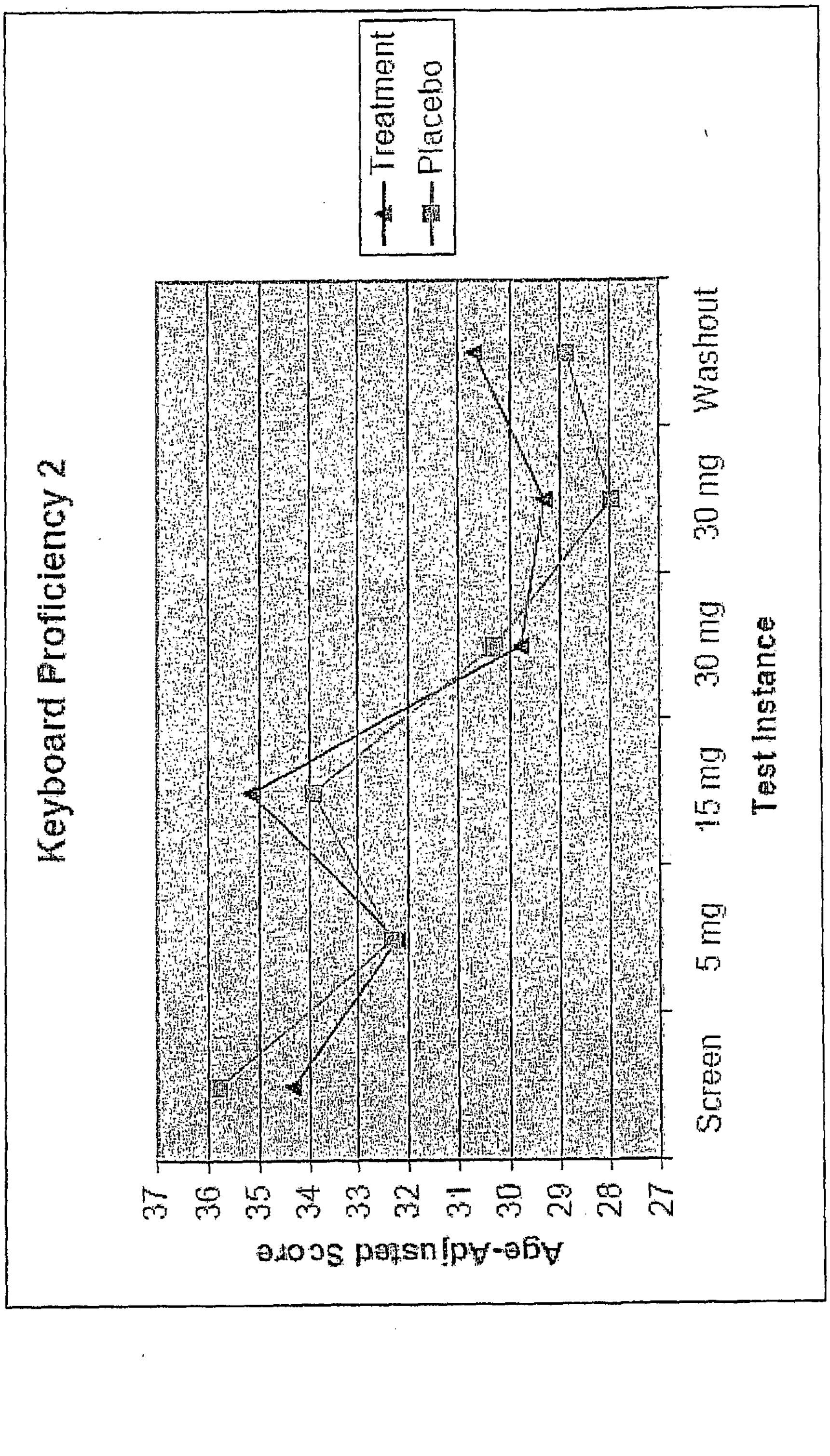
Keyboard Proficiency 2
Age-Adjusted Score
37
36
35
34
33
32
31
30
29
28
27
Screen
5 mg
15 mg
30 mg
30 mg
Washout
Test Instance
Treatment
Placebo

FIG. 27

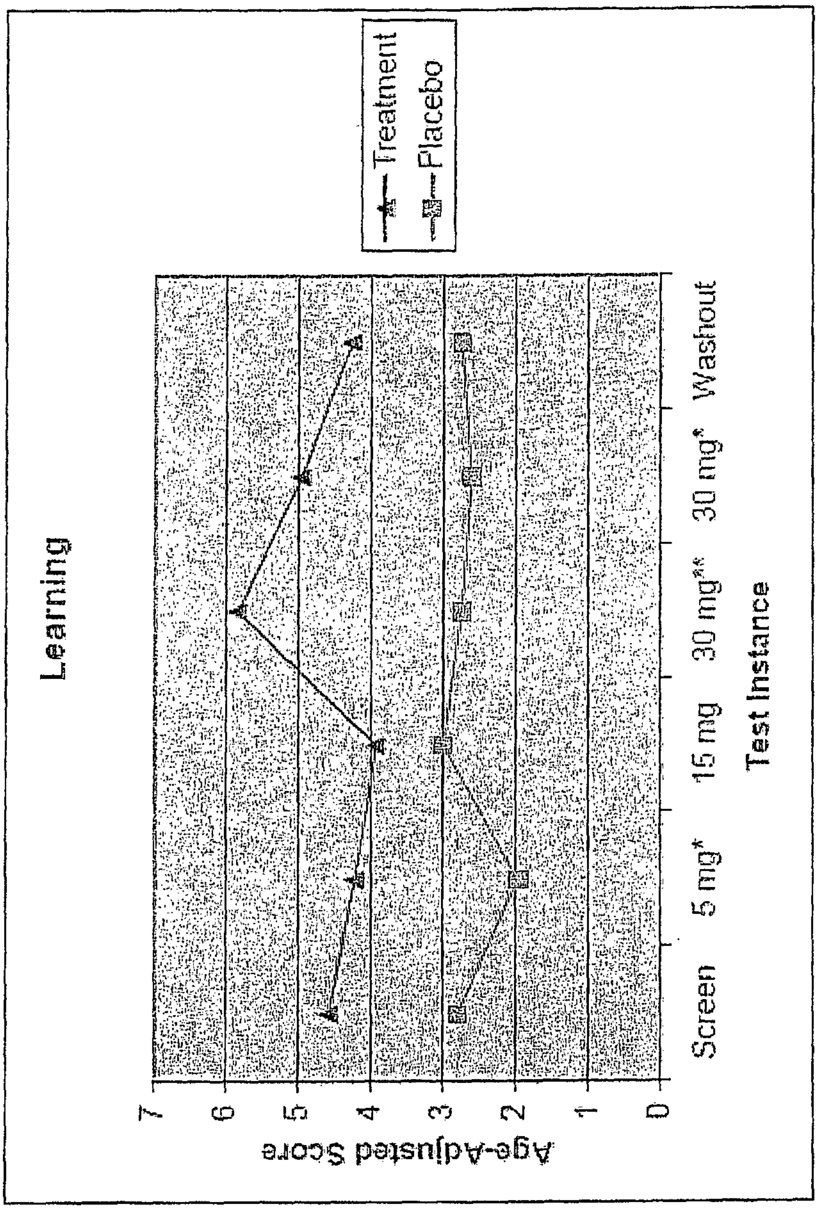
Learning
Treatment
Placebo
Age-Adjusted Score
7
6
5
4
3
2
1
0
Screen
5 mg*
15 mg
30 mg**
30 mg*
Washout
Test Instance

FIG. 28

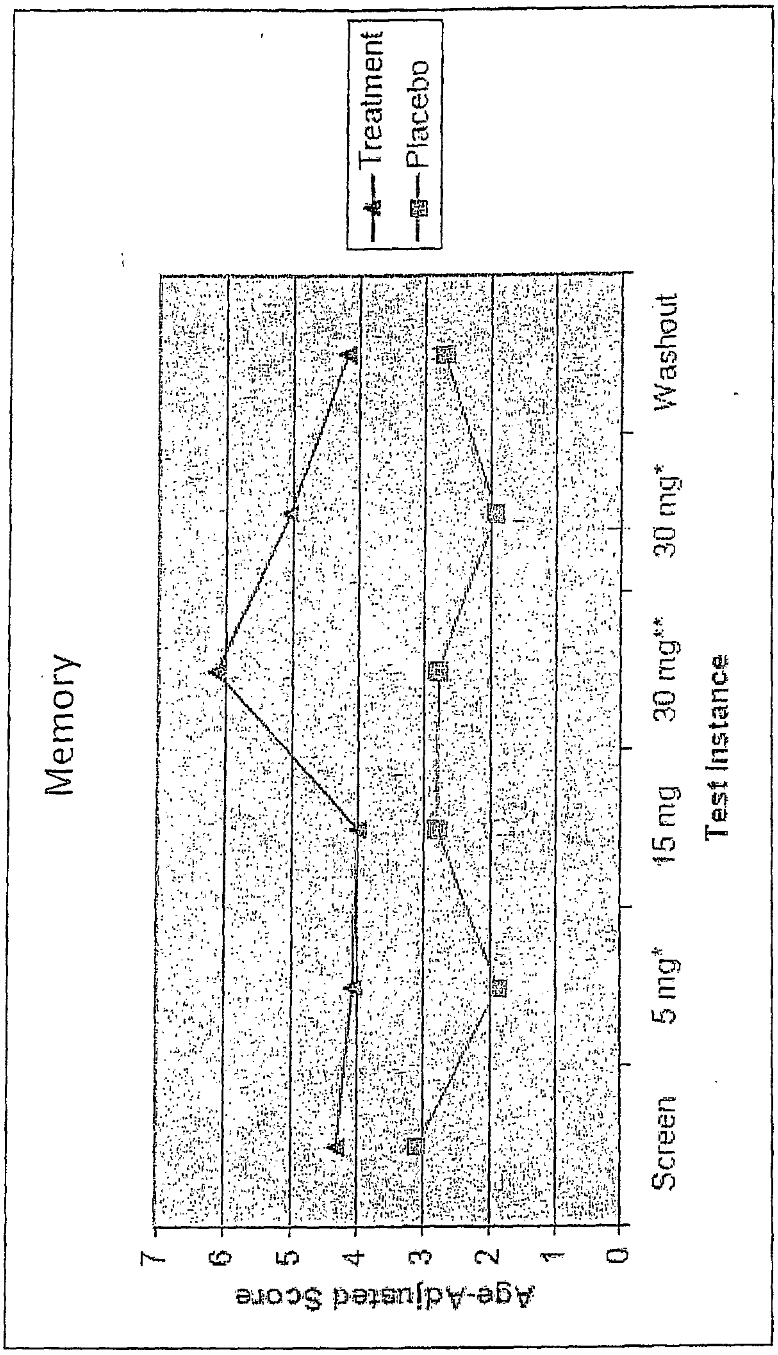
Memory
Treatment
Placebo
Age-Adjusted Score
7
6
5
4
3
2
1
0
Screen
5 mg*
15 mg
30 mg**
30 mg*
Washout
Test Instance

FIG. 29

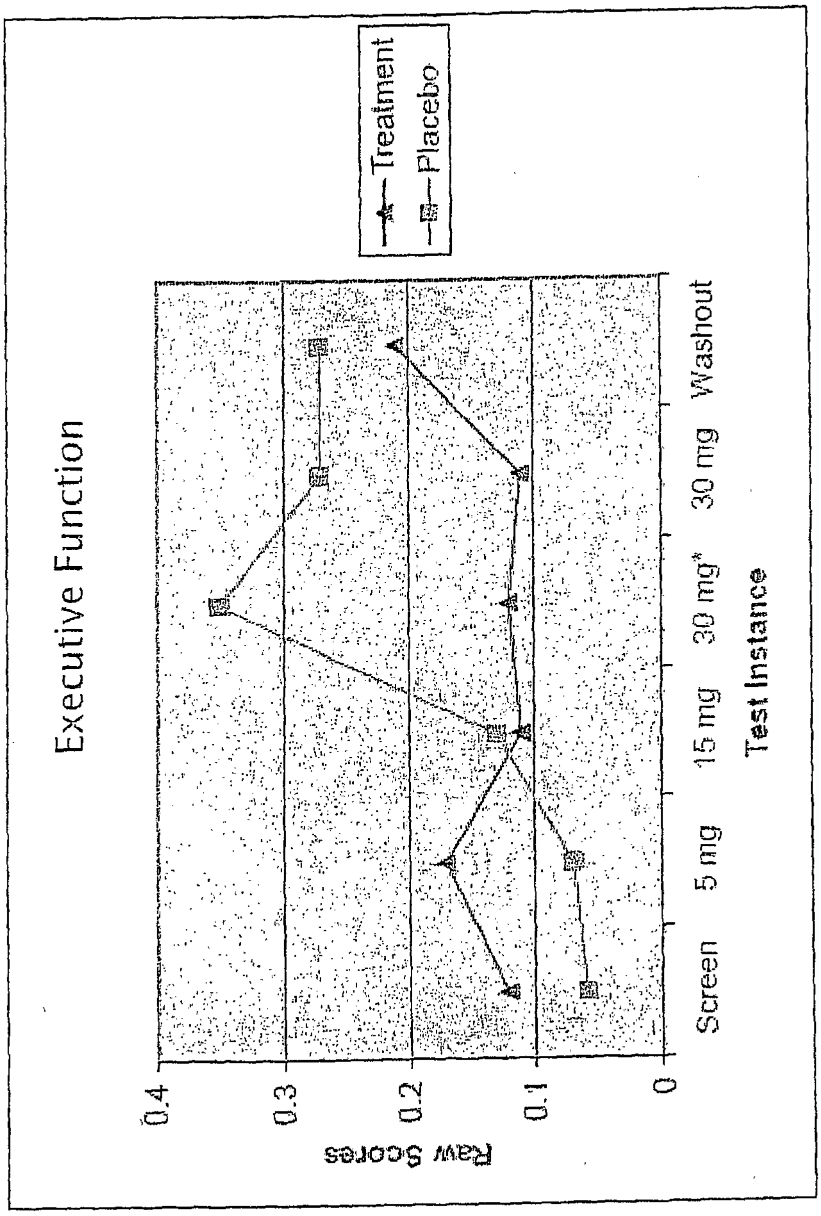
Executive Function
Raw Scores
0.4
0.3
0.2
0.1
0
Screen
5 mg
15 mg
30 mg*
30 mg
Washout
Test Instance
Treatment
Placebo

FIG. 30

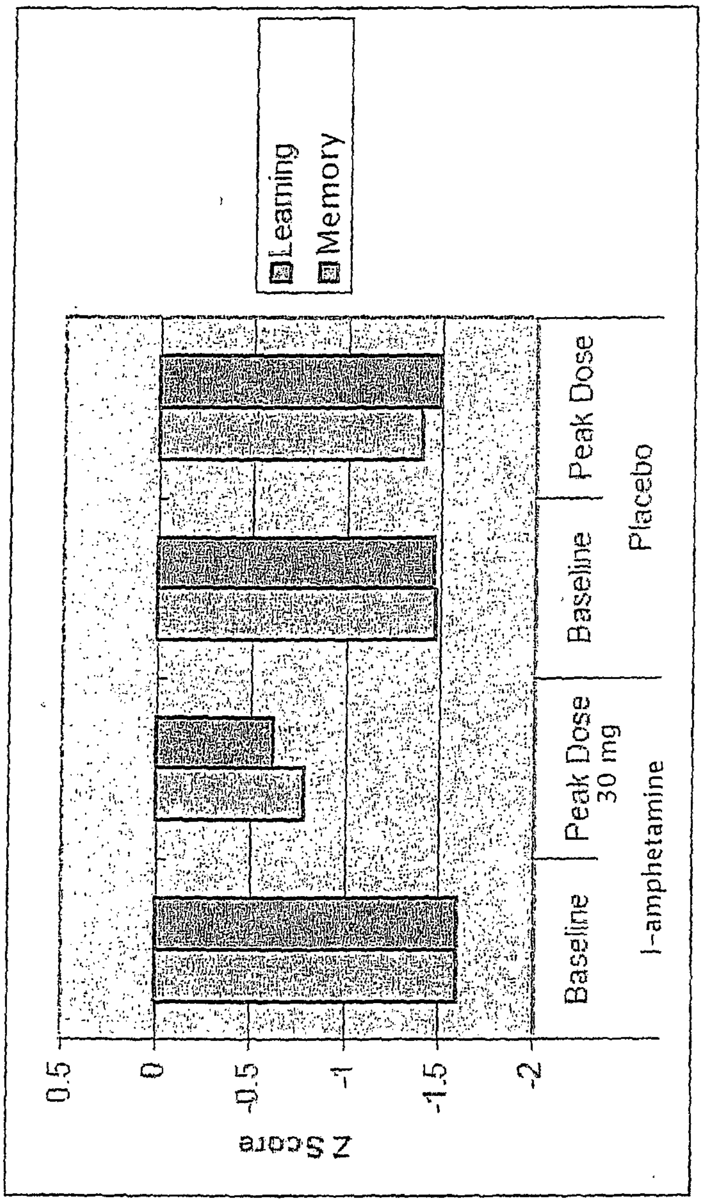
Z Score
0.5
0
-0.5
-1
-1.5
-2
Learning
Memory
Baseline
Peak Dose 30 mg
l-amphetamine
Baseline
Peak Dose
Placebo

FIG. 31

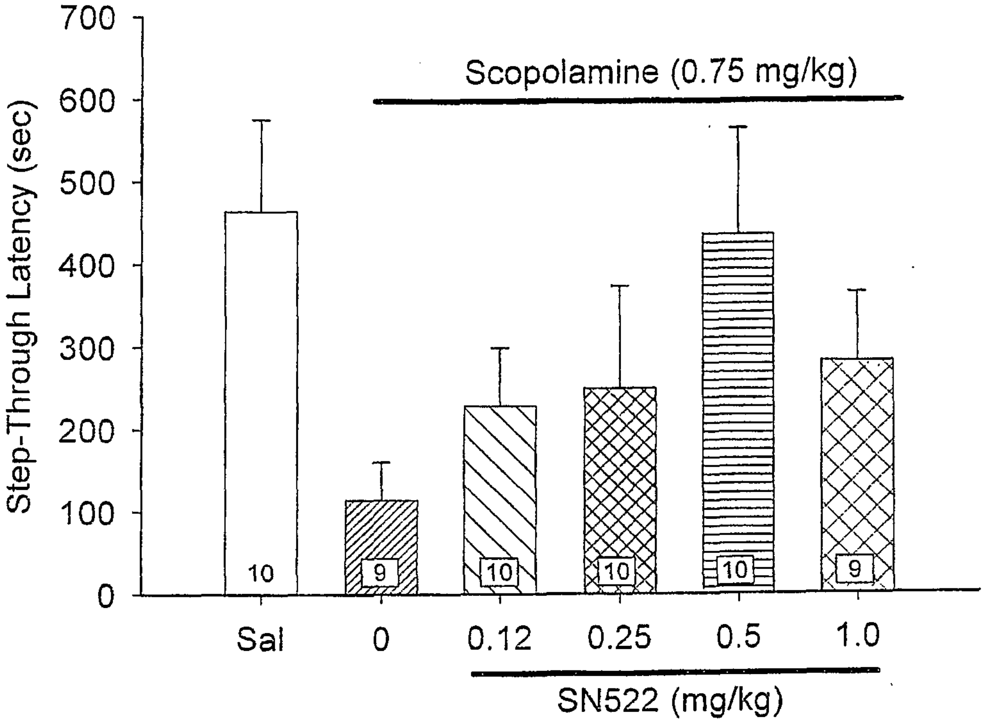
Scopolamine (0.75 mg/kg)
Step-Through Latency (sec)
700
600
500
400
300
200
100
0
10
9
10
10
10
9
Sal
0
0.12
0.25
0.5
1.0
SN522 (mg/kg)

FIG. 32

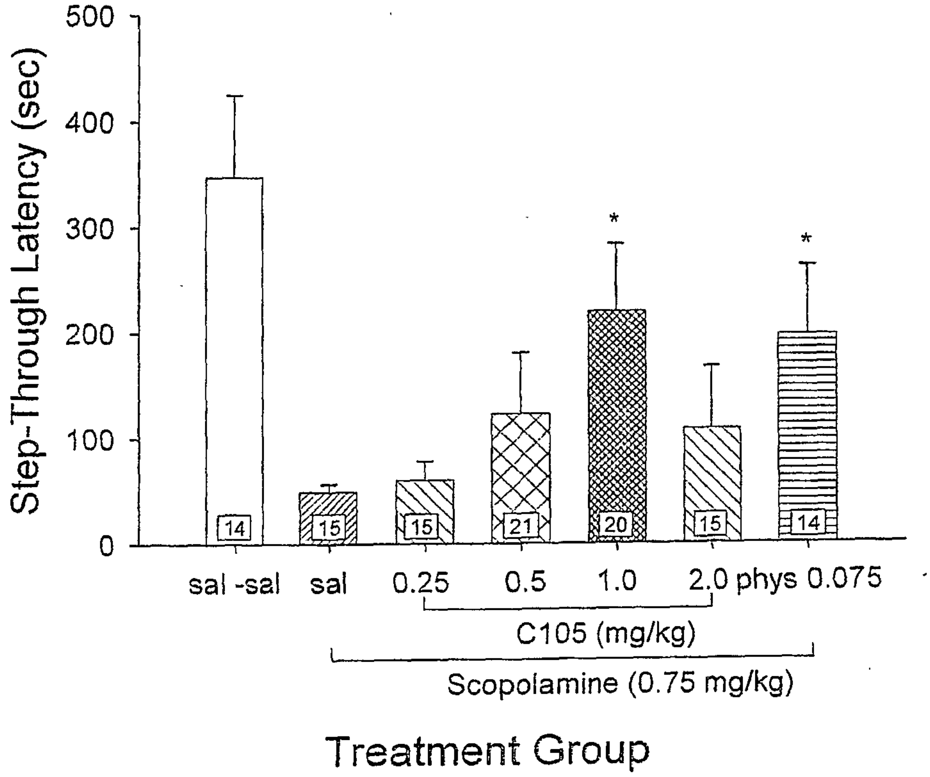
Step-Through Latency (sec)
500
400
300
200
100
0
14
15
15
21
20
15
14
*
*
sal -sal
sal
0.25
0.5
1.0
2.0
phys 0.075
C105 (mg/kg)
Scopolamine (0.75 mg/kg)
Treatment Group

FIG. 33

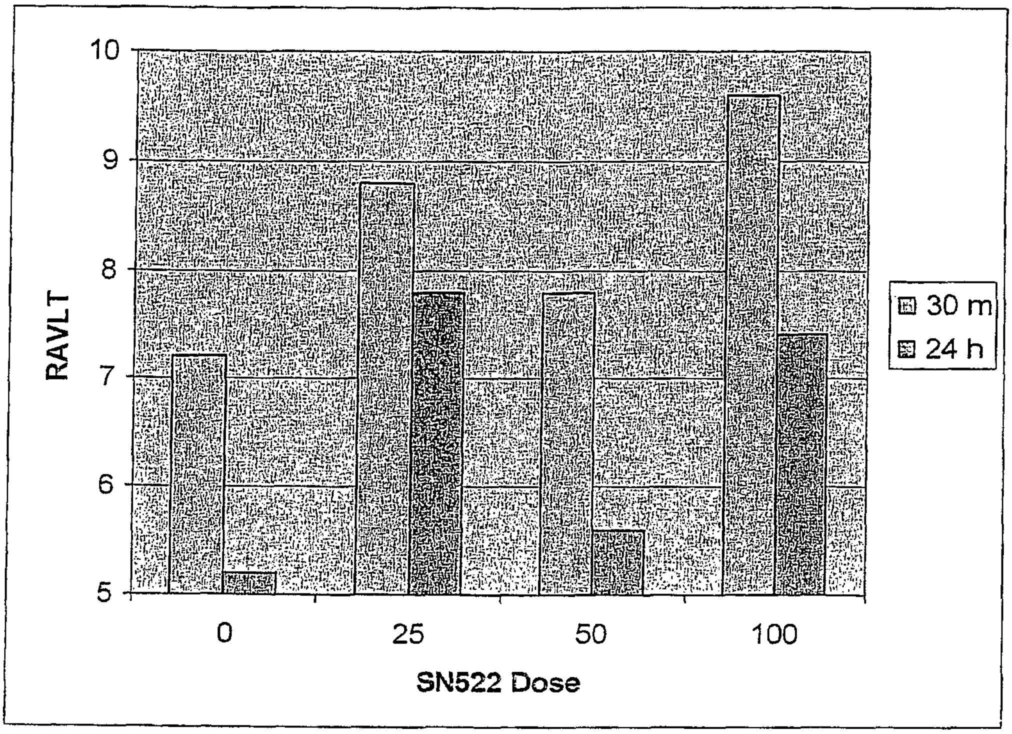


FIG. 34

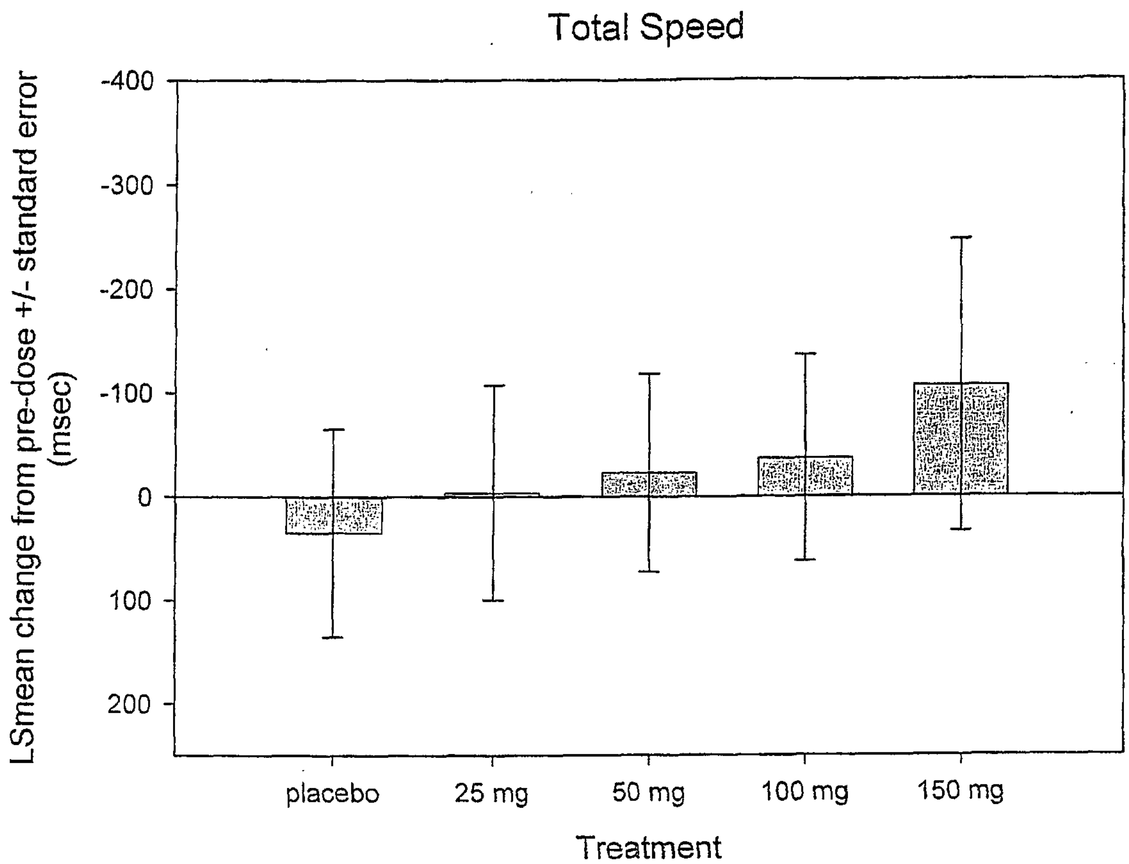
Total Speed
LSmean change from pre-dose +/- standard error (msec)
-400
-300
-200
-100
0
100
200
placebo
25 mg
50 mg
100 mg
150 mg
Treatment

FIG. 35

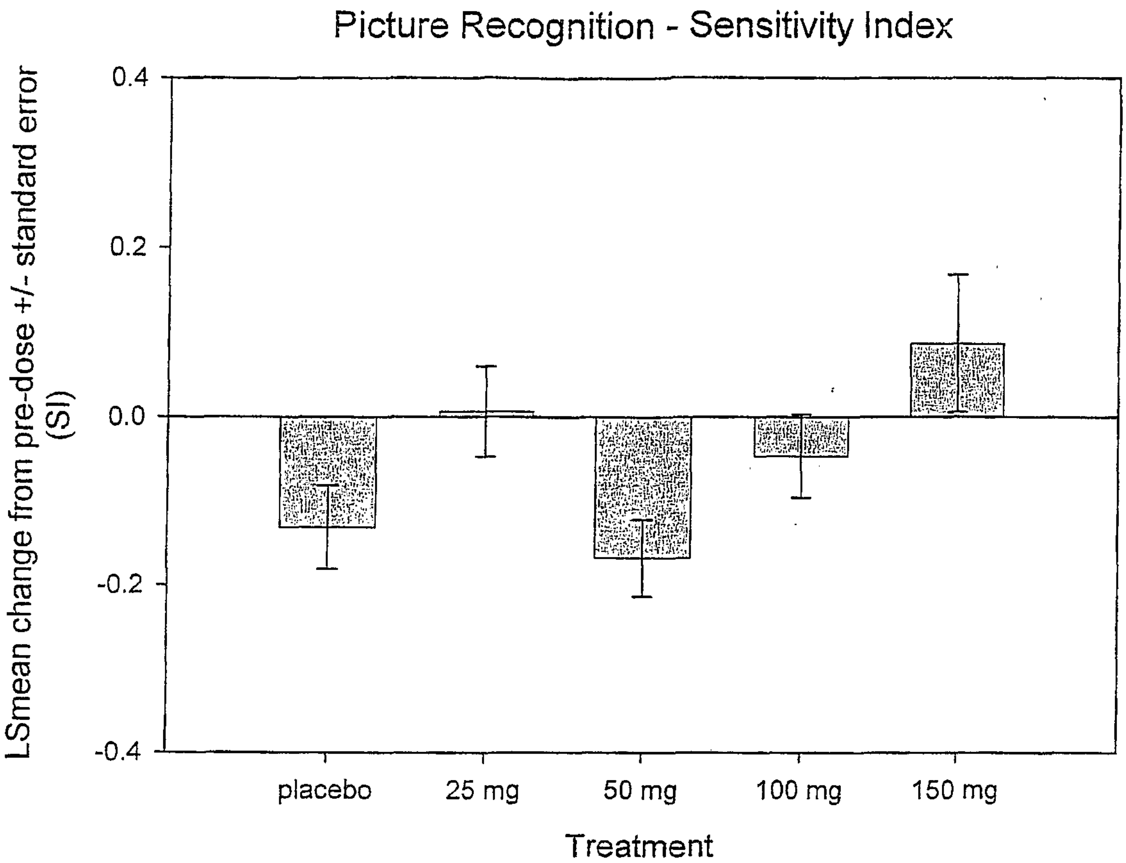
Picture Recognition - Sensitivity Index
LSmean change from pre-dose +/- standard error (SI)
0.4
0.2
0.0
-0.2
-0.4
placebo
25 mg
50 mg
100 mg
150 mg
Treatment

FIG. 36

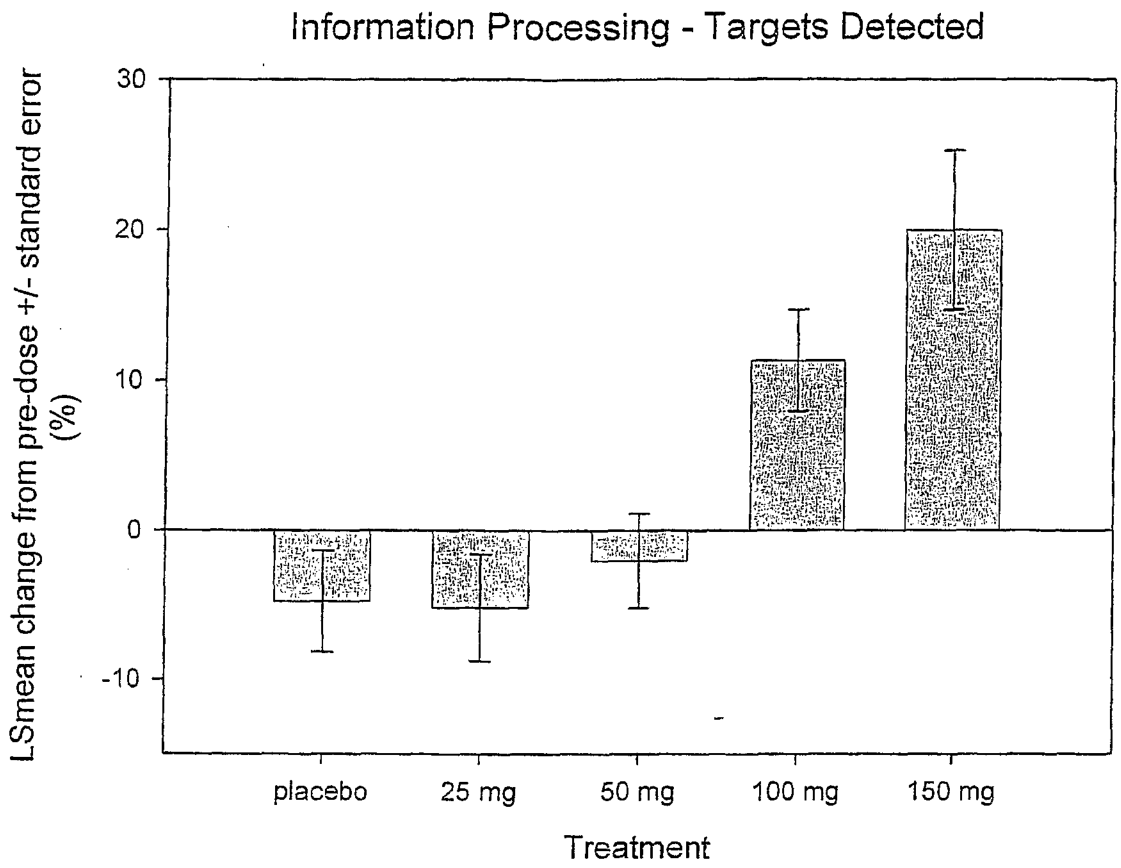
Information Processing - Targets Detected
LSmean change from pre-dose +/- standard error (%)
30
20
10
0
-10
placebo
25 mg
50 mg
100 mg
150 mg
Treatment

FIG. 37

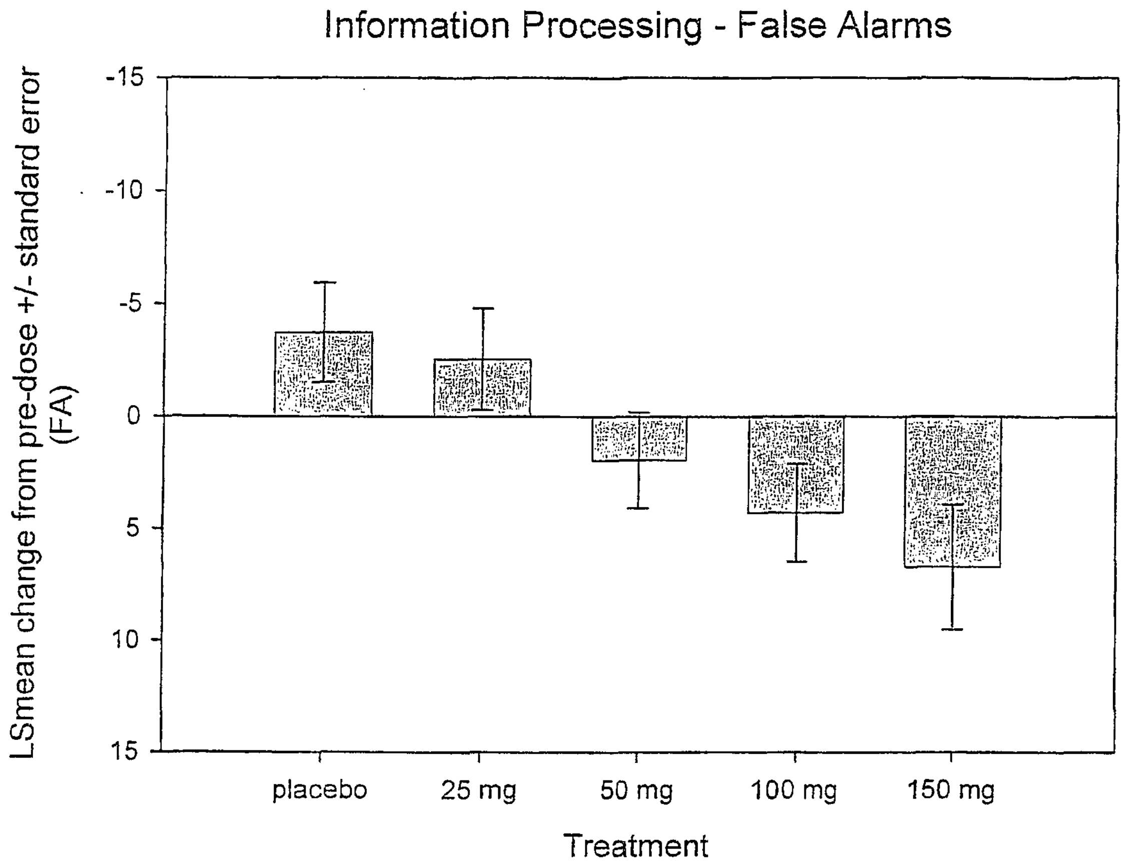
Information Processing - False Alarms
LSmean change from pre-dose +/- standard error (FA)
-15
-10
-5
0
5
10
15
placebo
25 mg
50 mg
100 mg
150 mg
Treatment

FIG. 38

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0139998 A
- US 5075338 A, Knoll
- US 2828343 A, Tindall
- US 3458576 A, Bryan
- GB 2122617 A
- US 3996381 A, Florvall
- WO 9408051 A
- US 5359115 A
- US 5362899 A
- US 5288514 A, Ellman
- US 5736412 A
- US 5712171 A
- WO 9210092 A
- WO 9309668 A
- WO 910708 A
- WO 9320242 A, Lemer
- US 4434153 A
- US 4721613 A
- US 4853229 A
- US 2996431 A
- US 3139383 A
- US 4752470 A
- US 4083949 A
- US 3811444 A
- US 3962414 A
- US 4066747 A
- US 4070347 A
- US 4079038 A
- US 4093709 A
- US 3992518 A
- US 4006218 A
- US 3551154 A
- US 3472931 A
- US 4973468 A
- US 4820720 A
- US 5006342 A
- US 4863970 A
- US 4933184 A
- US 229130 A
- US 4440777 A
- WO 9902141 A
- US 20030073885 A1
- WO 0154650 A
- WO 0172217 A
- WO 0154559 A


### Non-patent literature cited in the description


- **YOUNGJOHN J.R. et al.** *Archives of Clinical Neuropsychology,* 1991, vol. 6, 287-300
- **WECHSLER, D.** Wechsler Memory Scale-Revised Manual, NY. The Psychological Corp, 1987
- The Californian Verbal Learning Test. **DELIS, D.C. et al.** Manual, San Antonio, TX. The Psychological Corporation, 2000
- **BUSCHKE, H. et al.** *Neurology,* 1974, vol. 24, 1019-1025
- **THORNTON, A.E. et al.** *Neuropsychology,* 1997, vol. 11, 357-366
- **BROWN, J.** *Quarterly J. of Exp. Psychology,* 1958, vol. 10, 12-21
- **GRONWALL, D.M.A.** *Perceptual and Motor Skills,* 1977, vol. 44, 367-373
- **DELUCA, J. et al.** *J. Clinical and Exp. Neuropsychology,* 2004
- **J. MARCH.** Advanced Organic Chemistry: Reactions, Mechanisms, And Structure. John Wiley and Sons, 1992
- **A. J. GORDON ; R. A. FORD.** The Chemist's Companion: A Handbook Of Practical Data, Techniques, And References. Wiley, 1972
- **W.A. HERRMANN ; BRAUER.** Synthetic Methods Of Organometallic And Inorganic Chemistry. Georg Thieme Verlag, 1996
- **D. TODD.** Experimental Organic Chemistry. Prentice-Hall, 1979
- **L. M. HARWOOD.** Experimental Organic Chemistry: Standard And Microscale. Blackwell Science, 1999
- **I. H. IVERSEN ; K. A. LATTAL.** Experimental Analysis Of Behavior. Elsevier, 1991
- **R. SOMMER ; B. SOMMER.** A Practical Guide To Behavioral Research: Tools And Techniques. Oxford University Press, 2002
- **A. L. HARVEY.** Advances In Drug Discovery Techniques. Chichester, 1998
- **T. P. HADJIIOANNOU.** Quantitative Calculations In Pharmaceutical Practice And Research. VCH, 1993

- **E. R. GARRETT ; J. L. HIRTZ.** Drug Fate And Metabolism: Methods And Techniques. M. Dekker, 1977
- **M. K. TICHY.** Behavioral Science Techniques: An Annotated Bibliography For Health Professionals. Praeger, 1975
- **GELOWITZ D et al.** *Pharacology Biochemistry and Behavior,* 1994, vol. 47, 41-45
- **YASAR S et al.** Are metabolites of L-Depeny1 (selegiline) useful or harmful? Indications from preclinical research. *J Neural Transm,* 1996, vol. 48, 61-73
- **KINCHLA, R.A. et al.** *Annu. Rev. Psychol.,* 1992, vol. 43, 711-742
- **ELLIOTT, R.** *Br. Med. Bull.,* 2003, vol. 65, 49-59
- The Californian Verbal Learning Test. **DELIS, D.C. et al.** Adult Version, Manual, San Antonio, TX. The Psychological Corporation, 2000
- **PERRY, R.J. et al.** *Neuropsychologia,* 2000, vol. 38, 252-271
- **BEAR, M.F. et al.** Neuroscience: Exploring The Brain. Williams & Wilkins, 1996, 517-545
- **MCGAUGH, J.L.** *Science,* 2000, vol. 287, 248-251
- Neuroscience: Exploring The Brain. **BEAR, M.F. et al.** Neuroscience: Exploring The Brain. Williams & Wilkins, 1996, 517-545
- **BECKER, J.T. et al.** *Brain and Cognition,* 1999, vol. 41, 1-8
- **BEAR, M.F. et al.** Neuroscience: Exploring The Brain. William & Wilkins, 1996, 517-545
- **TULVING, E et al.** *Science,* 1990, vol. 247, 301-306
- **SQUIRE, L.R. et al.** *Proc. Natl. Acad. Sci.,* 1996, vol. 93, 13515-13522
- **CARLSON, N.R.** Physiology of Behavior. Allyn and Bacon, 1986
- **MEHLER, J. et al.** Bradford Books. 1995
- **CROCE et al.** *Gazz. Chim. Ital.,* 1996, vol. 126, 107-109
- **MASTAGLI.** *Bull. Soc. Chim. Fr.,* 1950, 1045-1047
- **SMITH et al.** *J. Med. Chem.,* 1988, vol. 31, 1558-1566
- **BOBRANSKII et al.** *J. Applied Chem.,* 1941, vol. 14, 410-414
- **MAGIDSON.** *J. Gen. Chem.,* 1941, vol. 11, 339-343
- **J. JACQUES ; A. COLLET ; S. WILEN.** Enantiomers, Racemates and Resolutions. Wiley, 1981
- *J. Med. Chem,* 1988, vol. 31, 1558-1570
- **BLONDELLE et al.** *Trends Anal. Chem.,* 1995, vol. 14, 83
- **CHEN et al.** *JACS,* 1994, vol. 116, 2661
- **KERR et al.** *JACS,* 1993, vol. 115, 252
- **GAGE et al.** *Brain Res.,* 1983, vol. 268, 27
- **GAGE et al.** *Neuroscience,* 1986, vol. 19, 241
- **KORDOWER et al.** *J. Comp. Neurol.,* 1990, vol. 298, 443
- **RUSH.** *Behav. Neural. Biol.,* 1988, vol. 50, 255
- Remington's Pharmaceutical Sciences. Mack Publishing Company, 1985
- **BRILL.** Pharmacology in Medicine. McGraw-Hill, 1965, 227
- The Pharmacological Bases of Therapeutics. Pergamon Press, 1990, 72
- **LIU et al.** *Inter. J. of Pharm.,* 1994, vol. 112, 105-116
- **LIU et al.** *Inter. J. of Pharm.,* 1994, vol. 112, 117-124
- **REMINGTON.** Pharm. Sci. 1970, 1626-1628
- **FINCHER et al.** *J. Pharm. Sci.,* 1968, vol. 57, 1825-1835
- **ROSOFF.** Controlled Release of Drugs. 1989, 53-95
- **COLEMAN et al.** Polymers. 1990, vol. 31, 1187-1230
- **ROERDINK et al.** Drug Carrier Systems. 1989, vol. 9, 57-109
- **LEONG et al.** *Adv. Drug Delivery Rev.,* 1987, vol. 1, 199-233
- **ROFF et al.** Handbook of Common Polymers. CRC Press, 1971
- **HOLFORD et al.** *Pharmac.,* 1982, vol. 16, 143-166
- **BERGE et al.** Pharmaceutical Salts. *J. Pharm. Sci.,* 1977, vol. 66, 1-19
- **BAMMER, G.** *Neurosci & Biobev. Rev.,* 1982, vol. 6, 247-296
- **ENNACEUR, A. et al.** *Psychopharmacol.,* 1992, vol. 109, 321-330
- **ENNACEUR, A. et al.** *Behav. Brain Res.,* 1989, vol. 33, 197-207
- **MORRIS, R.** *J. Neurosci Methods,* 1984, vol. 11, 47-60
- **D'AMOUR, F.E. et al.** *J. Pharmacol. Exp. Ther.,* 1941, vol. 72, 174-179
- **REY, A.** The Rey Auditory Visual Learning Test. *RAVLT,* 1941
- L'examen psychologique dans les cas d'encéphalopathie traumatique . Archives de Psychologie. L'examen psychologique dans les cas d'encéphalopathie traumatique. Archives de Psychologie. 1995, vol. 28, 21
- **LEZAK, M.D.** Neuropsychological Assessment. Oxford University Press
- **ERLANGER, D.M. et al.** *J. Head Trauma Rehabil.,* 2002, vol. 17, 458-476